(19) Europäisches Patentamt
European Patent Office
Office européen des brevets

(11) **EP 2 436 687 A1**

(12) **EUROPEAN PATENT APPLICATION**

(43) Date of publication:
**04.04.2012 Bulletin 2012/14**

(21) Application number: **11184538.4**

(22) Date of filing: **01.03.2006**

(51) Int Cl.:
***C07H 17/00*** *(2006.01)* ***C12Q 1/00*** *(2006.01)*
***C07D 513/04*** *(2006.01)* ***C07D 471/04*** *(2006.01)*
***A61K 31/427*** *(2006.01)* ***A61K 31/35*** *(2006.01)*
***C07D 309/14*** *(2006.01)* ***C07D 277/62*** *(2006.01)*
***C07D 211/56*** *(2006.01)* ***A61K 31/445*** *(2006.01)*

(84) Designated Contracting States:
**AT BE BG CH CY CZ DE DK EE ES FI FR GB GR HU IE IS IT LI LT LU LV MC NL PL PT RO SE SI SK TR**

(30) Priority: **01.03.2005 US 656878 P**

(62) Document number(s) of the earlier application(s) in accordance with Art. 76 EPC:
**06705254.8 / 1 869 060**

(71) Applicant: **Simon Fraser University**
**Burnaby, British Columbia V5A 1S6 (CA)**

(72) Inventors:
- **Vocadlo, David**
  **New Westminster, British Columbia V3M 1P6 (CA)**
- **Whitworth, Garrett**
  **Surrey, British Columbia V3S 8L9 (CA)**
- **Macauley, Matthew**
  **Port Alberni, British Columbia V9Y 7L4 (CA)**
- **Stubbs, Keith**
  **Greenwood, Western Australia 6024 (AU)**

(74) Representative: **Grund, Martin**
**Grund**
**Intellectual Property Group**
**Postfach 44 05 16**
**80754 München (DE)**

Remarks:
This application was filed on 10-10-2011 as a divisional application to the application mentioned under INID code 62.

(54) **Selective glycosidase inhibitors, methods of making inhibitors, and uses thereof**

(57) The invention comprises compounds for selectively inhibiting glycosidases, prodrugs of the compounds, and pharmaceutical compositions containing the compounds or prodrugs of the compounds. The invention also comprises animal models and methods of making the animal models for studying diseases and disorders related to deficiency or overexpression of O-GlcNAcase, accumulation or deficiency of O-GlcNAc, and treatment of such diseases and disorders. The invention also comprises methods of treating such diseases and disorders. The invention also comprises methods of making the compounds, and methods of making selective glycosidase inhibitors.

**Figure 1**

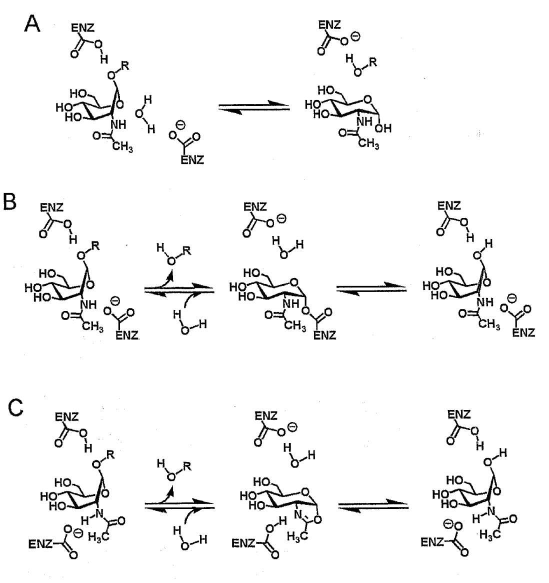

EP 2 436 687 A1

## Description

Related Application

This application claims the benefit of the filing date of United States provisional patent application No. 60/656,878 filed 1 March 2005 which is hereby incorporated by reference.

Technical Field

This application relates to compounds which selectively inhibit glycosidases, methods of making the inhibitors, and uses thereof.

Background

A wide range of cellular proteins, both nuclear and cytoplasmic, are post-translationally modified by the addition of the monosaccharide 2-acetamido-2-deoxy-β-D-glucopyranoside (β-N-acetylglucosamine) which is attached via an O-glycosidic linkage[1]. This modification is generally referred to as O-linked N-acetylglucosamine or O-GlcNAc.

*O*-GlcNAc-modified proteins have a wide range of vital cellular functions including, for example, transcription[2-5], proteasomal degradation[6], and cellular signaling[7]. O-GlcNAc is also found on many structural proteins[8-10]. For example, it has been found on a number of cytoskeletal proteins, including neurofilament proteins[11,12], synapsins[13,14], synapsin-specific clathrin assembly protein AP-3[15], and ankyrinG[16]. O-GlcNAc modification has been found to be abundant in the brain [17, 18]. It has also been found on proteins clearly implicated in the etiology of several diseases including type II diabetes, Alzheimer's disease (AD), and cancer.

For example, it is well established that AD and a number of related tauopathies including Downs' syndrome, Pick's disease, Niemann-Pick Type C disease, and amyotrophic lateral sclerosis (ALS) are characterized, in part, by the development of neurofibrillary tangles (NFTs). These NFTs are aggregates of paired helical filaments (PHFs) and are composed of an abnormal form of the important protein "tau". Normal tau stabilizes a key cellular network of microtubules that is essential for distributing proteins and nutrients within neurons. In AD patients, however, tau becomes hyperphosphorylated, disrupting its normal functions, forming PHFs and ultimately aggregating to form detrimental NFTs. A clear parallel between NFT levels in the brains of AD patients and the severity of dementia strongly supports a key role for tau dysfunction in AD[19,20]. The precise causes of this hyperphosphorylation of tau remain elusive. Accordingly, considerable effort has been dedicated toward a) elucidating the molecular physiological basis of tau hyperphosphorylation, [6] ; and b) identifying strategies that could limit tau hyperphosphorylation in the hope that these might halt, or even reverse, the progression of Alzheimer's disease. [7, 8]. Thus far, several lines of evidence suggest that up-regulation of a number of kinases may be involved [9, 10, 21] in hyperphosphorylation of tau, although very recently, an alternative basis for this hyperphosphorylation has been advanced. [21]. In particular, it has recently emerged that phosphate levels of tau are regulated by the levels of O-GlcNAc on tau. Hyperphosphorylated tau in human AD brains has markedly lower levels of O-GlcNAc than are found in healthy human brains[2, 3]. These results suggest that a malfunction in the mechanisms regulating tau O-GlcNAc levels may be vitally important in the formation of NFTs and associated neurodegeneration.

Humans have three genes encoding enzymes that cleave terminal β-N-acetylglucosamine residues from glycoconjugates. The first of these encodes the enzyme O-glycoprotein 2-acetamido-2-deoxy-β-D-glucopyranosidase, (O-GlcNAcase). O-GlcNAcase is a member of family 84 of glycoside hydrolases that includes enzymes from organisms as diverse as prokaryotic pathogens to humans (for the family classification of glycoside hydrolases see Coutinho, P.M. & Henrissat, B. (1999) Carbohydrate-Active Enzymes server at URL: http://afmb.cnrs-mrs.fr/CAZY/ ([19,20]). O-GlcNAcase acts to hydrolyse O-GlcNAc off of serine and threonine residues of post-translationally modified proteins [1, 22, 23]. Consistent with the presence of O-GlcNAc on many intracellular proteins, the enzyme O-GlcNAcase appears to have a role in the etiology of several diseases including type II diabetes [7, 21], AD[9, 17, 24], and cancer[18]. Although O-GlcNAcase was likely isolated earlier on [11, 12], about 20 years elapsed before its biochemical role in acting to cleave O-GlcNAc from serine and threonine residues of proteins was understood [13]. More recently O-GlcNAcase has been cloned [15], partially characterized [16], and suggested to have additional activity as a histone acetyltransferase [14]. However, little was known about the catalytic mechanism of this enzyme.

The other two genes, HEXA and HEXB, encode enzymes catalyzing the hydrolytic cleavage of terminal β-N-acetylglucosamine residues from glycoconjugates. The gene products of HEXA and HEXB predominantly yield two dimeric isozymes, hexosaminidase A and hexosaminidase B, respectively. Hexosaminidase A (αβ), a heterodimeric isozyme, is composed of an α- and a β-subunit. Hexosaminidase B (ββ), a homodimeric isozyme, is composed of two β-subunits. The two subunits, α- and β-, bear a high level of sequence identity. Both of these enzymes are classified as members of family 20 of glycoside hydrolases and are normally localized within lysosomes. The proper functioning of

these lysosomal β-hexosaminidases is critical for human development, a fact that is underscored by the tragic genetic illnesses, Tay-Sach's and Sandhoff diseases which stem from a dysfunction in, respectively, hexosaminidase A and hexosaminidase B[25]. These enzymatic deficiencies cause an accumulation of glycolipids and glycoconjugates in the lysosomes resulting in neurological impairment and deformation. The deleterious effects of accumulation of gangliosides at the organismal level are still being uncovered[26].

As a result of the biological importance of these β-*N*-acetyl-glucosaminidases, small molecule inhibitors of glycosidases [27-30] have received a great deal of attention [31], both as tools for elucidating the role of these enzymes in biological processes and in developing potential therapeutic applications. The control of glycosidase function using small molecules offers several advantages over genetic knockout studies including the ability to rapidly vary doses or to entirely withdraw treatment.

However, a major challenge in developing inhibitors for blocking the function of mammalian glycosidases, including O-GlcNAcase, is the large number of functionally related enzymes present in tissues of higher eukaryotes. Accordingly, the use of non-selective inhibitors in studying the cellular and organismal physiological role of one particular enzyme is complicated because complex phenotypes arise from the concomitant inhibition of such functionally related enzymes. In the case of β-N-acetylglucosaminidases, existing compounds that act to block O-GlcNAcase function are non-specific and act potently to inhibit the lysosomal β-hexosaminidases. Heretofore no potent inhibitor was known that is selective for nucleocytoplasmic O-GlcNAcase over the lysosomal β-hexosaminidases.

A few of the better characterized inhibitors of β-*N*-acetyl-glucosaminidases which have been used in studies of O-GlcNAc post-translational modification within both cells and tissues are streptozotocin (STZ), 2'-methyl-α-D-glucopyrano-[2,1-*d*]-Δ2'-thiazoline (NAG-thiazoline) and *O*-(2-acetamido-2-deoxy-D-glucopyranosylidene)amino *N*-phenylcarbamate (PUGNAc) [7, 32-35].

STZ has long been used as a diabetogenic compound because it has a particularly detrimental effect on β-islet cells [36]. STZ exerts its cytotoxic effects through both the alkylation of cellular DNA [36, 37] as well as the generation of radical species including nitric oxide [38]. The resulting DNA strand breakage promotes the activation of poly(ADP-ribose) polymerase (PARP) [39] with the net effect of depleting cellular NAD+ levels and, ultimately, leading to cell death [40, 41]. Other investigators have proposed instead that STZ toxicity is a consequence of the irreversible inhibition of O-GlcNAcase, which is highly expressed within β-islet cells [32,42]. This hypothesis has, however, been brought into question by two independent research groups [43,44]. Because cellular O-GlcNAc levels on proteins increase in response to many forms of cellular stress [45] it seems possible that STZ results in increased O-GlcNAc-modification levels on proteins by inducing cellular stress rather than through any specific and direct action on O-GlcNAcase. Indeed, Hanover and coworkers have shown that STZ functions as a poor and somewhat selective inhibitor of O-GlcNAcase [46] and although it has been proposed by others that STZ acts to irreversibly inhibit O-GlcNAcase [47], there has been no clear demonstration of this mode of action.

NAG-thiazoline has been found to be a potent inhibitor of family 20 hexosaminidases, [30, 48] and more recently, the family 84 *O*-GlcNAcases[49]. Despite its potency, a downside to using NAG-thiazoline in a complex biological context is that it lacks selectivity and therefore perturbs multiple cellular processes.

PUGNAc is another compound that suffers from the same problem of lack of selectivity, yet has enjoyed use as an inhibitor of both human *O*-GlcNAcase[13,50] and the family 20 human β-hexosaminidases [51]. This molecule, developed by Vasella and coworkers, was found to be a potent competitive inhibitor of the β-*N*-acetyl-glucosaminidases from *Canavalia ensiformis*, *Mucor rouxii*, and the β-hexosaminidase from bovine kidney [28].

The need has therefore arisen for improved selective inhibitors of glycosidases.

## Summary of Invention

The embodiments of the inventions relate to compounds which selectively inhibit glycosidases. The invention also relates to methods of making such compounds and uses thereof.

In one embodiment of the invention, the compounds have the general chemical formula (I):

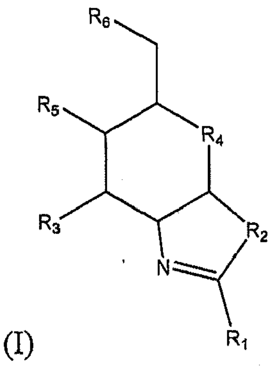

(I)

wherein $R_3$, $R_5$, $R_6$, are selected from the group consisting of branched alkyl chains, unbranched alkyl chains, cycloalkyl groups, aromatic groups, alcohols, ethers, amines, substituted or unsubstituted carbamates, substituted or unsubstituted ureas, esters, amides, aldehydes, carboxylic acids, and heteroatom containing derivatives thereof, wherein said esters and amides may comprise an acyl group selected from the group consisting of branched alkyl chains, unbranched alkyl chains, cycloalhyl groups, aromatic groups, and heteroatom derivatives thereof; $R_2$ and $R_4$ are $CH_2$, $CHR_1$, NH, $NR_1$, or any heteroatom, and $R_1$ is selected from the group consisting of H, ethers, amines, branched alkyl chains, unbranched alkyl chains, cycloalkyl groups, aromatic groups, and heteroatom derivatives thereof. The invention includes pharmaceutically acceptable salts of the above compounds. In some embodiments of the invention, $R_2$ is S, and $R_1$ is selected from the group consisting of $CH_2CH_3$, $(CH_2)_2CH_3$, $(CH_2)_3CH_3$, $(CH_2)_4CH_3$, $CH(CH_3)_2$ and $CH_2CH(CH_3)_2$, and $R_4$ is O. The invention also relates to prodrugs of the compounds, pharmaceutical compositions containing the compounds and a pharmaceutically acceptable carrier, and pharmaceutical compositions containing prodrugs of the compounds and a pharmaceutically acceptable carrier.

In another embodiment of the invention, the compounds have the general chemical formula (II):

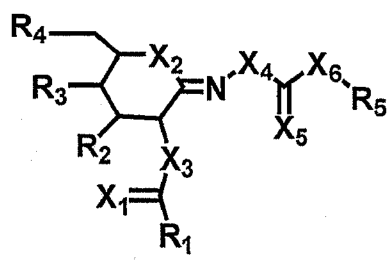

(II)

wherein $X_1$-$X_6$ are O, NH, $NR_1$, $CHR_1$, $CH_2$ or any heteroatom, $R_1$ to $R_5$ are selected from the group consisting of branched alkyl chains, unbranched alkyl chains, cycloalkyl groups, aromatic groups, alcohols, ethers, amines, substituted or unsubstituted carbamates, substituted or unsubstituted ureas, esters, amides, aldehydes, carboxylic acids, and heteroatom containing derivatives thereof, wherein said esters and amides may comprise an acyl group selected from the group consisting of branched alkyl chains, unbranched alkyl chains, cycloalkyl groups, aromatic groups, and heteroatom derivatives thereof, and pharmaceutically acceptable salts thereof.

In some embodiments the compounds selectively inhibit the activity of specific glycosidases over other glycosidases. In one embodiment of the invention, the glycosidases comprise glycoside hydrolases. In another embodiment of the invention, the glycoside hydrolases are family 84 glycoside hydrolases. In a specific embodiment of the invention, the glycosidase is O-GlcNAcase. The compounds selectively inhibit the activity O-GlcNAcase over β-hexosaminidase. In particular, the compounds selectively inhibit the cleavage of 2-acetamido-2-deoxy-β-D-glucopyranoside (O-GlcNAc) from proteins in this particular embodiment.

The compounds are useful in the development of animal models for studying diseases or disorders related to deficiencies in O-GlcNAcase, over-expression of O-GlcNAcase, accumulation of O-GlcNAc, depletion of O-GlcNAc, and for studying treatment of diseases and disorders related to deficiency or over-expression of O-GlcNAcase, or accumulation or depletion of O-GlcNAc. Such diseases and disorders include diabetes, neurodegenerative diseases, including Alzheimer's disease, and cancer. The compounds are also useful in the treatment of diseases and disorders responsive to glycosidase inhibition therapy. The compounds are also useful in preparing cells and tissues for stress associated with tissue damage or stress, stimulating cells, and promoting differentiation of cells. These compounds may also be useful in inhibiting specific glycosidases, for example microbial toxins that are members of family 84 glycoside hydrolases, and thereby find use as antimicrobials.

The invention also relates to methods of making the compounds. In one embodiment, the method may comprise the steps of:

a) Acylating the hydrochloride salt of 2-amino-2-deoxy-1,3,4,6-tetra-O-acetyl-β-D-glucopyranose with a range of acylating agents to yield a series of 1,3,4,6-tetra-O-acetyl-2-deoxy-2-N-acyl-β-D-glucopyranose derivatives;

b) Converting the amides of the series of 1,3,4,6-tetra-O-acetyl-2-deoxy-2-N-acyl-β-D-glucopyranose derivatives to corresponding thioamides and cyclizing the thioamides to yield a series of 3,4,6-tri-O-acetyl-1,2-dideoxy-2'-alkyl-α-D-glucopyranoso-[2,1-d]-Δ2'-thiazolines; and

c) Deacylating the thiazoline compounds to yield a series of 1,2-dideoxy-2'-alkyl-α-D-glucopyranoso-[2,1-d]-Δ2'-thiazolines.

The invention also relates to methods of making selective glycosidase inhibitors comprising:

a) Selecting an inhibitor of two or more glycosidases or a class of glycosidases;

b) Modifying one or more side chains of the inhibitor by enlarging or reducing the side chain; and

c) Testing the modified inhibitor for selective inhibition of one or more of the glycosidases.

In some embodiments of the invention, the method of making selective glycoside inhibitors comprises the steps of:

a) Selecting an inhibitor of β-*N*-acetyl-glucosaminidases or β-hexosaminidases having a general formula selected from:

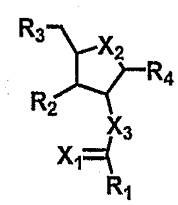 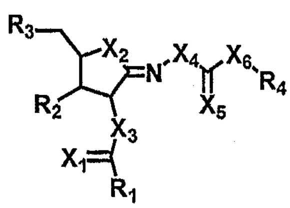 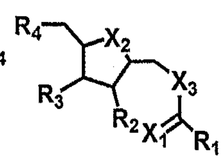

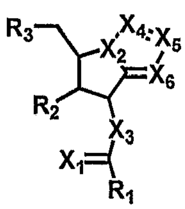 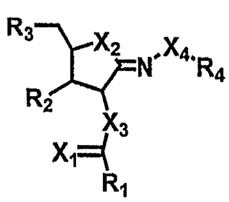 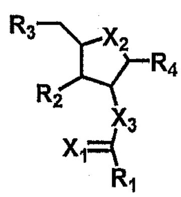

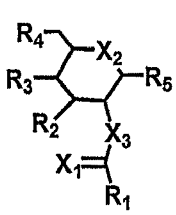 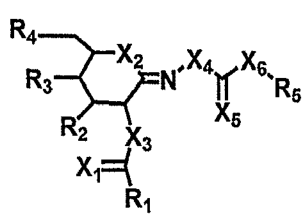 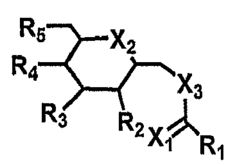

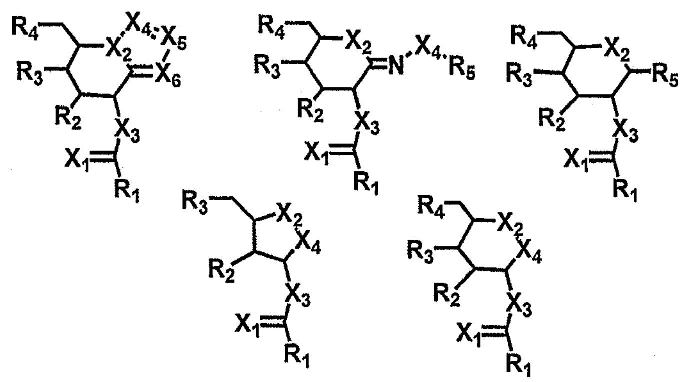

wherein $X_1$-$X_6$ are O, NH, $NR_1$, $CHR_1$, $CH_2$ or any heteroatom, $R_1$ to $R_5$ are selected from the group consisting of branched alkyl chains, unbranched alkyl chains, cycloalkyl groups, aromatic groups, alcohols, ethers, amines, substituted or unsubstituted carbamates, substituted or unsubstituted ureas, esters, amides, aldehydes, carboxylic acids, and heteroatom containing derivatives thereof, wherein said esters and amides may comprise an acyl group selected from the group consisting of branched alkyl chains, unbranched alkyl chains, cycloalkyl groups, aromatic groups, and heteroatom derivatives thereof, and pharmaceutically acceptable salts thereof;

b) Modifying the $R_1$ side chain of the inhibitor by enlarging or reducing the bulk of the side chain; and

c) Testing the modified inhibitors for selective inhibition of one or more β-N-acetyl-glucosaminidases or β-hexosaminidases.

In some embodiments of the invention, the $R_1$ side chain of the inhibitor can be enlarged by inserting a branched alkyl chain, an unbranched alkyl chain, a cycloalkyl group, an aromatic group, or heteroatom derivatives thereof, into the side chain. In some specific embodiments, the $R_1$ chain is enlarged by inserting groups selected from the group consisting of $CH_2CH_3$, $(CH_2)_2CH_3$, $(CH_2)_3CH_3$, $(CH_2)_4CH_3$, $CH(CH_3)_2$ and $CH_2CH(CH_3)_2$,

Brief Description of Drawings

In drawings which are intended to illustrate embodiments of the invention:

Figure 1 is an illustration of three possible catalytic mechanisms for O-GlcNAcase. Pathway A; the single step inverting mechanism; pathway B; the double displacement retaining mechanism involving formation and breakdown of a covalent glycosyl enzyme intermediate; pathway C; the double displacement retaining mechanism involving formation and breakdown of a bicyclic oxazoline intermediate.

Figure 2 illustrates the activity of O-GlcNAcase and β-hexosaminidase in the presence ofN-fluoroacetyl derivatives of MU-GlcNAc. (A) Initial velocity of the human β-hexosaminidase catalyzed hydrolysis of N-fluoroacetyl derivatives of MU-GlcNAc; (♦) MU-GlcNAc (5), (▼) MU-GlcNAc-F (5a), (■) MU-GlcNAc-F2 (5b), (●) MU-GlcNAcF3 (5c). Inset: detail of the region of the plot at the intersection of the axes. (B) Initial velocity of the human O-GlcNAcase catalyzed hydrolysis ofN-fluoroacetyl derivatives of MU-GlcNAc; (♦) MU-GlcNAc (5), (▼) MU-GlcNAc-F (5a), (■) MU-GlcNAc-F2 (5b), (●) MU-GlcNAcF3 (5c). Inset: detail of the region of the plot at the intersection of the axes. (C) Linear free energy analysis plotting the Taft parameter (σ*) of the N-fluoroacetyl substituent of MU-GlcNAc substrate analogues against the log $Vmax[E]_o / K_M$ values measured for each substrate as shown in panels A and B with (●) O-GlcNAcase and (□) β-Hexosaminidase.

Figure 3 illustrates a competitive inhibition pattern of human O-GlcNAcase catalyzed hydrolysis of pNP-GlcNAc in the presence of NAG-Thiazoline (**9a**). The concentrations of **9a** (mM) used were 0.00 (♦), 0.033 (△), 0.100 (●), 0.300 (X), 0.900 (■), and 3.04 (○). Inset: graphical analysis of $K_I$ from plotting $K_M$ apparent against NAG-Thiazoline (**9a**) concentration.

Figure 4 is a graph illustrating the selectivity of inhibition of O-GlcNAcase over β-hexosaminidase by a panel of thiazoline inhibitors. Bar graph of the $K_I$ values of the thiazoline inhibitors panel (**9a-f**) measured for the inhibition of O-GlcNAcase ( ) and β -hexosaminidase ( )catalyzed hydrolysis of MU-GlcNAc.

Figure 5 is a Western blot of proteins from COS-7 cells cultured for 40 hours in the presence or absence of 50

μM of different thiazoline inhibitors. Incubation of COS-7 cells with thiazoline inhibitors causes an increase in cellular levels of O-GlcNAc-modified proteins. Lane 1, thiazoline **9a**; lane 2, thiazoline **9c**; lane 3, thiazoline **9g**; lane 4, no inhibitor. (A) Western blot analysis of cellular levels of O-GlcNAc-modified proteins using anti-O-GlcNAc MAb CTD 110.6 followed by an anti-mouse IgG-HRP conjugate. (B) Western blot of samples loaded in (A) treated with anti-β-actin mAb Clone AC-40 followed by an anti-mouse IgG-HRP conjugate reveals equivalent β-actin levels in each sample.

Figure 6 is a Western blot of proteins from COS-7 cells cultured over 40 hours in the presence or absence of 50 μM thiazoline inhibitor (**9c**). Incubation of COS-7 cells with thiazoline inhibitor (**9c**) results in a time dependent increase in cellular levels of O-GlcNAc-modified proteins. (A) Western blot analysis of cellular levels of O-GlcNAc-modified proteins as a function of time after exposure to inhibitor (**9c**) using anti-O-GlcNAc MAb CTD 110.6 followed by an anti-mouse IgG-HRP conjugate. (B) Western blot of samples loaded in (A) treated with anti-β-actin mAb Clone AC-40 followed by an anti-mouse IgG-HRP conjugate reveals equivalent β-actin levels in each sample. (C) Western blot analysis of cellular levels of O-GlcNAc-modified proteins as a function of time in the absence of inhibitor indicate that levels of O-GlcNAc-modified proteins do not significantly change as a function of confluence. At 0 hours plates were ≈ 25% confluent and after 40 hours plates were ≈ 90% confluent. (D) Western blot of samples loaded in (C) treated as for panel (B) reveals equivalent β-actin levels in both samples.

Figure 7 illustrates the decomposition of Streptozotocin (STZ) in $D_2O$. STZ dissolved in $D_2O$ was monitored over a period of 24 hours. The resonance marked with an asterisk (*) is from H-1 of the α-anomer of STZ. Spectra were collected at room temperature from a sample of STZ (≈ 10 mM) freshly dissolved in $D_2O$. NMR spectra were collected A) 5 minutes, B) 20 minutes, C) 40 minutes, D) 2 hours, E) 4 hours, and F) 24 hours after dissolving STZ.

Figure 8 is a graph which illustrates that STZ does not show a time dependent inactivation of *O*-GlcNAcase or β-hexosaminidase. A) O-GlcNAcase (0.116 mg/mL) was incubated with 10 mM STZ in the presence of 50 mM $NaH_2PO_4$, 100 mM NaCl, 1% BSA, 5 mM β-mercaptoethanol, pH 6.5. At several time intervals the enzyme activities of the inactivation mixture and control were assayed. These values are not uniform because some STZ is transferred with the aliquot of enzyme (3 μL) into the assay mixture (25 μL total) containing Mu-GlcNAc (5.7 mM) to yield a final concentration of 1.2 mM STZ in the assay mixture. Indeed, the expected ratio of reaction velocities of the assays for the control and experiment (control / $V_{STZ}$) calculated assuming STZ acts a competitive inhibitor of *O*-GlcNAcase ($K_I$ =1.5 mM) is 1.17, entirely consistent with the values measured here over 6 hours. B) β-hexosaminidase (0.036 mg/mL) was incubated with 10 mM STZ in the presence of 50 mM citrate, 100 mM NaCl, 0.1 % BSA, pH 4.25. At several time intervals the enzyme activities of the inactivation mixture and control were assayed as described in the methods and materials. These values are not exactly uniform because some STZ is transferred with the aliquot of enzyme (3 μL) into the assay mixture (25 μL total) containing Mu-GlcNAc (5.7 mM) to yield a final concentration of 1.2 mM STZ in the assay mixture. Indeed, the expected ratio of reaction velocities of the assays for the control and experiment ($V_{Control}$ / $V_{STZ}$) calculated assuming STZ acts a competitive inhibitor of β-hexosaminidase ($K_I$ = 47 mM) is 1.01, consistent with the values measured here over 6 hours.

Figure 9 shows Western blots of proteins from brain, muscle and liver tissue of rats injected with varying doses of inhibitor **9c.** Four rats were treated by tail vein injection of different doses of inhibitor 9c or a buffer control. Two sequential injections were performed approximately 18 hours apart and the rats were then sacrificed 24 hours after the first injection. Western blot analyses using an α-*O*-GlcNAc antibody (CTD110.6) clearly indicate that the inhibitor **9c** easily gains access into a variety of tissues including brain (A), muscle (B) and liver (C) and doses as low as 50 mg $kg^{-1}$ are almost enough to elicit a maximal increase in O-GlcNAc levels.

Figure 10 illustrates the results of an intravenous glucose tolerance test conducted on rats. (**A**) Eight rats, four of which were exposed to a 50 mg $kg^{-1}$ tail vein injection of inhibitor 9c, were tested for their ability to clear glucose. An intravenous glucose tolerance test (IVGTT) was performed with a 1 g $kg^{-1}$ challenge of glucose that was also delivered via the tail vein. Error bars indicate variance between the four rats used under each condition. (**B**) Western blot analyses using an α-O-GlcNAc antibody (CTD110.6) indicates that the four rats treated with the inhibitor in the IVGTT did have increased levels of *O*-GlcNAc modified proteins.

Figure 11 shows Western blots of proteins from brain and muscle tissue of rats injected with inhibitor **9c** at various time periods. Several rats were treated by tail vein injection with 50 mg $kg^{-1}$ of inhibitor 9c and sacrificed at specific times after the injection to observe how O-GlcNAc levels vary over time. Western blot analyses using an α-*O*-GlcNAc antibody (CTD110.6) was used to monitor O-GlcNAc levels. Brain (A) and muscle (B) tissue show that the inhibitor is able to gain access to tissues rapidly where it acts quickly to elevate O-GlcNAc levels (< 3 hours). Within approximately 24-32 hours, inhibitor **9c** is cleared from the tissues and O-GlcNAc levels return to normal. Time 0 and 32(-) represent untreated control animals at time 0 hours and 32 hours.

Figure 12 shows Western blots of proteins from brain, muscle, pancreas, fat and spleen tissue of rats fed two different forms and doses of inhibitor **9c.** Rats were fed food containing varying amounts of the deprotected (polar) or protected (nonpolar) inhibitor 9c or no inhibitor at all for three days. Levels of O-GlcNAc in tissues were evaluated by Western blot analyses using an α-*O*-GlcNAc antibody (CTD110.6). A dose of 100 mg $kg^{-1}$ $day^{-1}$ is enough to inhibit *O*-GlcNAcase and cause large increases in *O*-GlcNAc modified proteins, as judge by western blot analyses using an

α-*O*-GlcNAc antibody in brain (A), muscle (B), pancreas (C), fat (D) and spleen (E) tissue. A dose of 1000 mg $kg^{-1}$ $day^{-1}$ causes slightly greater increases in *O*-GlcNAc modified proteins. Control blots with an α-actin anitibody or SDS-PAGE shows that sample loading was equal.

## Detailed Description of the Invention

Throughout the following description, specific details are set forth in order to provide a more thorough understanding of the invention. However, the invention may be practiced without these particulars. In other instances, well known elements have not been shown or described in detail to avoid unnecessarily obscuring the invention. Accordingly, the specification and drawings are to be regarded in an illustrative, rather than a restrictive, sense.

The invention comprises compounds which selectively inhibit glycosidases. In one embodiment of the invention, the compounds have the general chemical formula (I):

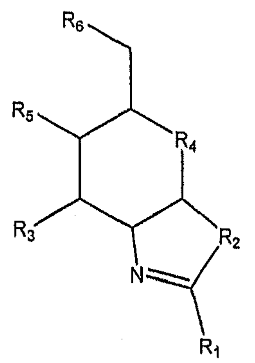

(I)

wherein $R_3$, $R_5$, $R_6$, are selected from the group consisting of branched alkyl chains, unbranched alkyl chains, cycloalkyl groups, aromatic groups, alcohols, ethers, amines, substituted or unsubstituted carbamates, substituted or unsubstituted ureas, esters, amides, aldehydes, carboxylic acids, and heteroatom containing derivatives thereof, wherein said esters and amides may comprise an acyl group selected from the group consisting of branched alkyl chains, unbranched alkyl chains, cycloalkyl groups, aromatic groups, and heteroatom derivatives thereof; $R_2$ and $R_4$ are $CH_2$, $CHR_1$, NH, $NR_1$, or any heteroatom, and $R_1$ is selected from the group consisting of H, ethers, amines, branched alkyl chains, unbranched alkyl chains, cycloalkyl groups, aromatic groups, and heteroatom derivatives thereof. The invention includes pharmaceutically acceptable salts of the above compounds. In some embodiments, $R_2$ is S, $R_1$ is selected from the group consisting of $CH_2CH_3$, $(CH_2)_2CH_3$, $(CH_2)_3CH_3$, $(CH_2)_4CH_3$, $CH(CH_3)_2$ and $CH_2CH(CH_3)_2$ and $R_4$ is O. In some specific embodiments of the invention, the compounds comprise *1,2-dideoxy-2'-ethyl-α-D-glucopyranoso-[2,1-d]-Δ2'-thiazoline, 1,2-dideoxy-2'-propyl-α-D-glucopyranoso-[2,1-d]-Δ2'-thiazoline, 1,2-dideoxy-2'-butyl-α-D-glucopyranoso-[2,1-d]-Δ2'-thiazoline, 1,2-dideoxy-2'-pentyl-α-D-glucopyranoso-[2,1-d]-Δ2'-thiazoline, 1,2-dideoxy-2'-isopropyl-α-D-glucopyranoso-[2,1-d]-Δ2'-thiazoline, 1,2-dideoxy-2'-isobutyl-α-D-glucopyranoso-[2,1-d]-Δ2'-thiazoline.*

As will be appreciated by a person skilled in the art, formula (I) above may also be represented alternatively as follows:

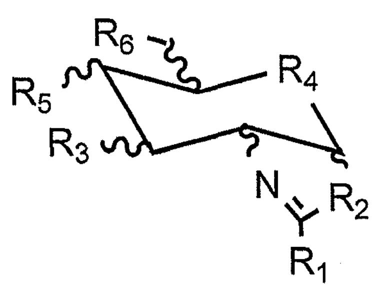

In another embodiment of the invention, the compounds have the general chemical formula (II):

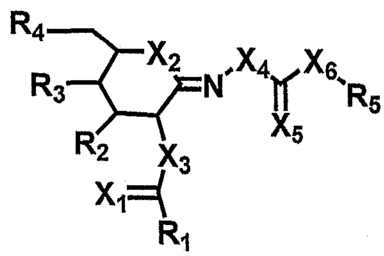

(II)

wherein $X_1$-$X_6$ are O, NH, $NR_1$, $CHR_1$, $CH_2$ or any heteroatom, $R_1$ to $R_5$ are selected from the group consisting of branched alkyl chains, unbranched alkyl chains, cycloalkyl groups, aromatic groups, alcohols, ethers, amines, substituted or unsubstituted carbamates, substituted or unsubstituted ureas, esters, amides, aldehydes, carboxylic acids, and heteroatom containing derivatives thereof, wherein said esters and amides may comprise an acyl group selected from the group consisting of branched alkyl chains, unbranched alkyl chains, cycloalkyl groups, aromatic groups, and heteroatom derivatives thereof, and pharmaceutically acceptable salts thereof.

The invention includes pharmaceutically acceptable salts of the above compounds. In some embodiments, $R_1$ is selected from the group consisting of $CH_2CH_3$, $(CH_2)_2CH_3$, $(CH_2)_3CH_3$, $(CH_2)_4CH_3$, $CH(CH_3)_2$ and $CH_2CH(CH_3)_2$, $R_2$, $R_3$ and $R_4$ are OH, $X_1$ and $X_2$ are O, $X_3$ is NH, $X_4$ and $X_5$ are O, $X_6$ is NH, and $R_5$ is $C_6H_6$. In some specific embodiments of the invention, the compounds comprise O-(2-deoxy-2-propamido-D-glucopyranosylidene)amino *N*-Phenylcarbamate, O-(2-deoxy-2-butamido-D-glucopyranosylidene)amino *N*-Phenylcarbamate, O-(2-deoxy-2-valeramido-D-glucopyranosylidene)amino *N*-Phenylcarbamate, O-(2-deoxy-2-hexamido-D-glucopyranosylidene)amino *N*-Phenylcarbamate, O-(2-deoxy-2-isobutamido-D-glucopyranosylidene)amino *N*-Phenylcarbamate, or O-(2-deoxy-2-isovaleramido-D-glucopyranosylidene)amino *N*-Phenylcarbamate.

Throughout this application, it is contemplated that the term "compounds" refers to the compounds discussed above and includes derivatives of the compounds, including acyl-protected derivatives, and pharmaceutically acceptable salts of the compounds and the derivatives. The invention also comprises prodrugs of the compounds, pharmaceutical compositions containing the compounds and a pharmaceutically acceptable carrier, and pharmaceutical compositions containing prodrugs of the compounds and a pharmaceutically acceptable carrier.

In some embodiments of the invention the compounds selectively inhibit the activity of specific glycosidases over other glycosidases. The glycosidases may comprise glycoside hydrolases. For example, the glycoside hydrolases may be family 84 glycoside hydrolases. In a specific embodiment of the invention, the glycosidase is O-GlcNAcase. The invention comprises compounds which selectively inhibit the activity of O-GlcNAcase over β-hexosaminidase. In particular, the compounds selectively inhibit the cleavage of O-GlcNAc from proteins.

The compounds of the invention are valuable tools in studying the physiological role of O-GlcNAc at the cellular and organismal level. The compounds are useful in the development of animal models to study a disease or disorder, or for studying treatment of a disease or disorder, related to deficiency or over-expression of O-GlcNAcase or accumulation or depletion of O-GlcNAc. As an example, the compounds are useful in the development of a disease model for the development of Type I or Type II diabetes.

Type II diabetes develops when humans or animals are unable to properly regulate blood glucose levels. Tissues must be able to sense and rapidly respond to changes in glucose availability as well as to signals from other components of the endocrine system. Glucose is the key nutrient in regulating insulin synthesis and secretion from the β-cells of the pancreas. Of all glucose entering into cells, 2-5% is shunted into the hexosamine biosynthetic pathway, thereby regulating cellular concentrations of the end product of this pathway, uridine diphosphate-N-acetylglucosamine (UDP-GlcNAc).[52] UDP-GlcNAc is a substrate of the nucleocytoplasmic enzyme O-GlcNAc transferase (OGTase),[53-56] which acts to post-translationally add GlcNAc to specific serine and threonine residues of numerous nucleocytoplasmic proteins. OGTase recognizes many of its substrates[57,58] and binding palmers[59, 60] through its tetratricopeptide repeat (TPR) domains.[61, 62] As described above, O-GlcNAcase,[13, 15] removes this post-translational modification to liberate proteins making the O-GlcNAc-modification a dynamic cycle occuring several times during the lifetime of a protein. [63] O-GlcNAc has been found in several proteins on known phosphorylation sites, [3, 64-66] suggesting a role for O-GlcNAc in cellular signaling. Additionally, OGTase shows unusual kinetic behaviour making it exquisitely sensitive to intracellular UDP-GlcNAc substrate concentrations and therefore glucose supply.[67] All of these data point to a logical role for O-GlcNAc levels acting as a nutrient sensing mechanism. To support such a potential role in nutrient sensing it has been shown that in peripheral tissues, elevated O-GlcNAc levels result in the development of insulin resistance. [7, 21] Indeed, it has been proposed that markedly elevated O-GlcNAc levels may lead to type II diabetes[68] in accord with a recent

study showing that a single nucleotide polymorphism is associated with the occurrence of Type II diabetes in a Mexican population. PUGNAc has been used in culture cells and in tissues to show that increased O-GlcNAc levels cause insulin resistance [7, 32-35]. By analogy, the compounds of the invention can also be used in similar studies and used to develop animal models to show the role of P-GlcNAc levels in the development of diabetes.

The compounds of the invention are also useful in the treatment of diseases or disorders related to deficiency or over-expression of O-GlcNAcase or accumulation or depletion of O-GlcNAc, or any disease or disorder responsive to glycosidase inhibition therapy. Such diseases and disorders include, but are not limited to, diabetes, neurodegenerative disorders, such as Alzheimer's disease (AD), and cancer. Such diseases and disorders may also include diseases or disorders related to the accumulation or deficiency in the enzyme OGTase.

For example, because of relationship between O-GlcNAc-levels and phosphorylation levels on tau as discussed above, the compounds of the invention may be used to study and treat AD and other tauopathies. Six isoforms of tau are found in the human brain. In AD patients, all six isoforms of tau are found in NFTs, and all are markedly hyperphosphorylated.[69, 70] Tau in healthy brain tissue bears only 2 or 3 phosphate groups, whereas those found in the brains of AD patients bear, on average, 8 phosphate groups [71, 72].

The presence of O-GlcNAc on tau has stimulated studies that correlate O-GlcNAc levels with tau phosphorylation levels. The recent interest in this field stems from the observation that O-GlcNAc modification has been found to occur on many proteins at amino acid residues that are also known to be phosphorylated. [65, 66, 73] Consistent with this observation, it has been found that increases in phosphorylation levels result in decreased O-GlcNAc levels and conversely, increased O-GlcNAc levels correlate with decreased phosphorylation levels. [74] This reciprocal relationship between O-GlcNAc and phosphorylation has been termed the "Ying-Yang hypothesis"[75] and has gained strong biochemical support by the recent discovery that the enzyme OGTase[55] forms a functional complex with phosphatases that act to remove phosphate groups from proteins.[60] Like phosphorylation, O-GlcNAc is a dynamic modification that can be removed and reinstalled several times during the lifespan of a protein. Suggestively, the gene encoding O-GlcNAcase has been mapped to a chromosomal locus that is linked to AD.[15, 76]

Very recently, it has been shown that O-GlcNAc levels of soluble tau protein from human brains affected with AD are markedly lower than those from healthy brain.[17] Furthermore, PHF from diseased brain was suggested to lack completely any O-GlcNAc modification whatsoever.[17] The molecular basis of this hypoglycosylation of tau is not known, although it may stem from increased activity of kinases and/or dysfunction of one of the enzymes involved in processing O-GlcNAc. Supporting this latter view, in both PC-12 neuronal cells and in brain tissue sections from mice, a nonselective N-acetylglucosamindase inhibitor was used to increase tau O-GlcNAc levels, whereupon it was observed that phosphorylation levels decreased.[17] The implication of these collective results is that by maintaining healthy O-GlcNAc levels in AD patients, such as by inhibiting the action of O-GlcNAcase, one should be able to block hyperphosphorylation of tau and all of the associated effects of tau hyperphosphorylation, including the formation of NFTs and downstream effects. However, because the proper functioning of the β-hexosaminidases is critical, any potential therapeutic intervention for the treatment of AD that blocks the action of O-GlcNAcase would have to avoid the concomitant inhibition of both hexosaminidases A and B. This selective inhibition is provided by compounds of the present invention.

The compounds of the invention are useful as selective inhibitors of other types of glycosidases. For example, some microbial toxins are members of family 84 glycoside hydrolases, and therefore, the compounds of the invention may be useful as antimicrobials.

The compounds of the invention are also useful in promoting differentiation of cells, such as promoting pluripotent cells into islet β-cells. For example, O-GlcNAc is known to regulate the function of several transcription factors and is known to be found on the transcription factor PDX-1. Modification of the transcription factor PDX-1 by modification of O-GlcNAc residues may affect activity of PDX-1, which in turn affects cell differentiation.

The compounds of the invention are also useful in preparing cells for stress. Recent studies have indicated that PUGNAc can be used in an animal model to reduce myocardial infarct size after left coronary artery occlusions [77].

The invention also relates to various methods of making the compounds. In one embodiment the method may comprise the steps of:

a) Acylating the hydrochloride salt of 2-amino-2-deoxy-1,3,4,6-tetra-*O*-acetyl-β-D-glucopyranose with a range of acylating agents to yield a series of 1,3,4,6-tetra-O-acetyl-2-deoxy-2-N-acyl-β-D-glucopyranose derivatives;

b) Converting the amides of the series of 1,3,4,6-tetra-O-acetyl-2-deoxy-2-N-acyl-β-D-glucopyranose derivatives to corresponding thioamides and cyclizing the thioamides to yield a series of 3,4,6-tri-O-acetyl-1,2-dideoxy-2'-alkyl-α-D-glucopyranoso-[2,1-d]-Δ2'-thiazolines; and

c) Deacylating the thiazoline compounds to yield a series of 1,2-dideoxy-2'-alkyl-α-D-glucopyranoso-[2,1-d]-Δ2'-thiazolines.

The invention also relates to methods of making selective glycosidase inhibitors comprising, for example, the steps of:

a) Selecting an inhibitor of two or more glycosidases or a class of glycosidases;

b) Modifying one or more side chains of the inhibitor by enlarging or reducing the side chain; and

c) Testing the modified inhibitor for selective inhibition of one or more of the glycosidases.

In some embodiments of the invention, the method of making selective glycoside inhibitors comprises the steps of:

a) Selecting an inhibitor of β-*N*-acetyl-glucosaminidases or β-hexosaminidases having a general formula selected from:

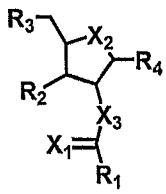
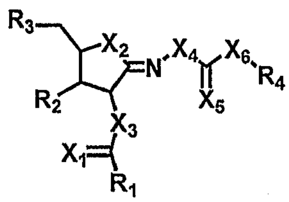
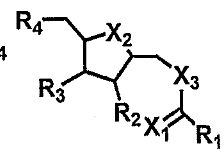

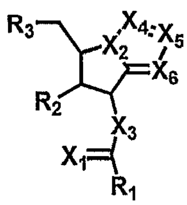
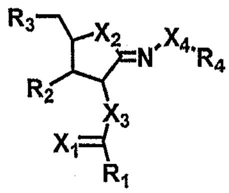
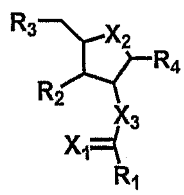

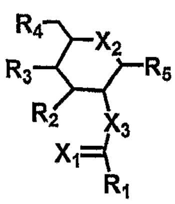
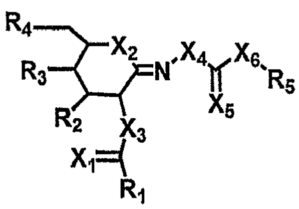
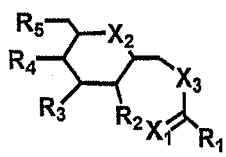

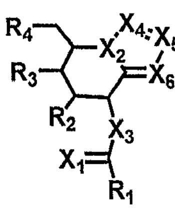
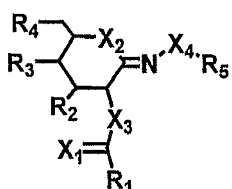
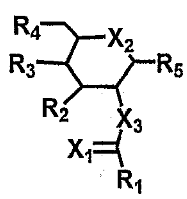
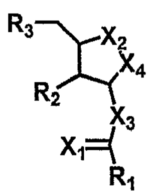
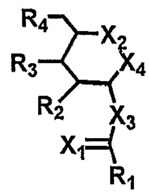

wherein $X_1$-$X_6$ are O, NH, $NR_1$, $CHR_1$, $CH_2$ or any heteroatom, $R_1$ to $R_5$ are selected from the group consisting of branched alkyl chains, unbranched alkyl chains, cycloalkyl groups, aromatic groups, alcohols, ethers, amines, substituted or unsubstituted carbamates, substituted or unsubstituted ureas, esters, amides, aldehydes, carboxylic acids, and heteroatom containing derivatives thereof, wherein said esters and amides may comprise an acyl group

selected from the group consisting of branched alkyl chains, unbranched alkyl chains, cycloalkyl groups, aromatic groups, and heteroatom derivatives thereof, and pharmaceutically acceptable salts thereof;

b) Modifying the $R_1$ chain of the inhibitor by enlarging or reducing the bulk of the side chain.; and

c) Testing the modified inhibitors for selective inhibition of one or more β-*N*-acetyl-glucosaminidases or β-hexosaminidases.

In some embodiments of the invention, the $R_1$ chain of the inhibitor can be enlarged by inserting a branched alkyl chain, an unbranched alkyl chain, a cycloalkyl group, an aromatic group or heteroatom derivatives thereof, into the side chain. In some specific embodiments, the $R_1$ chain is enlarged by inserting groups selected from the group consisting of $CH_2CH_3$, $(CH_2)_2CH_3$, $(CH_2)_3CH_3$, $(CH_2)_4CH_3$, $CH(CH_3)_2$ and $CH_2CH(CH_3)_2$.

EXAMPLES

The following examples are intended to illustrate embodiments of the invention and are not intended to be construed in a limiting manner.

<u>Example 1 : Comparative Analysis of the Catalytic Mechanisms of O-GlcNAcase and β-Hexosaminidase</u>

<u>1.1 Possible Catalytic Mechanisms of O-GlcNAcase</u>

A logical starting point for the design of inhibitors of O-GlcNAcase takes into consideration the catalytic mechanism of O-GlcNAcase and β-hexosaminidase. Although the catalytic mechanism of action of the family 20 human β-hexosaminidase A and B have been fairly well established [78], that of the family 84 O-GlcNAcase remains unknown. The inventors therefore first elucidated the catalytic mechanism of human O-GlcNAcase and secondly, used this information in designing simple inhibitors that would be potent, cell permeable, and highly selective for O-GlcNAcase over the lysosomal β-hexosaminidases.

Three realistic mechanistic alternatives exist for O-GlcNAcase and family 84 glycoside hydrolases. The first alternative is an inverting mechanism (Figure 1A) such as that found for goose lysozyme [79] from family 23 of glycoside hydrolases. This catalytic mechanism generally involves the base-catalyzed nucleophilic attack of water at the anomeric centre concomitant with the acid-catalyzed departure of the leaving group.

The second mechanistic possibility is the canonical two step double displacement mechanism that results in retention of configuration at the anomeric center (Figure 1B). This mechanism is used by most retaining β-glycosidases and involves, in the first step, attack of an enzymic nucleophile at the anomeric center with the resulting formation of a transient covalent glycosyl enzyme intermediate [80]. Departure of the aglycon leaving group is facilitated by an enzymic residue acting as a catalytic general acid. In the second step this same residue acts as a catalytic general base to facilitate the attack of a water molecule at the anomeric center, cleaving the intermediate to liberate the hemiacetal product with retained stereochemistry. β-N-acetylglucosaminidases from family 3 of glycoside hydrolases have been shown to use this mechanism [81] as have the C-type lysozymes from family 22 [82].

The third mechanistic alternative involves the nucleophilic participation of the 2-acetamido group of the substrate in place of an enzymic catalytic nucleophile (Figure 1C). This last mechanistic option is exploited by β-hexosaminidases from family 20 of glycoside hydrolases [30, 48, 83] These three mechanisms differ in several aspects. The inverting mechanism is a one step reaction that results in the formation of a product with inverted stereochemistry at the anomeric center. The other two alternatives are retaining in stereochemistry at the anomeric center and differ from each other primarily in the nature of the intermediate; in the second mechanism this species is a covalent enzyme adduct whereas in the third case it is believed to be a bicyclic oxazoline or oxazolinium ion.

A key difference between these mechanistic alternatives is the involvement of the 2-acetamido group of the substrate. This moiety may actively participate in catalysis as a nucleophile, as for the lysosomal β -hexosaminidases, or it may act as a bystander.

<u>1.2 Synthesis of Substrate Analogues</u>

To address the role of the 2-acetamido group of the substrate, several substrate analogues were synthesized bearing differing levels of fluorine substitution on the N-acetyl group (Scheme 1). The highly electronegative fluorine substituents decrease the basicity of the carbonyl group and the expected effect of such substitutions on an enzymatic reaction using anchimeric assistance would be to decrease its rate.

**Scheme 1:**

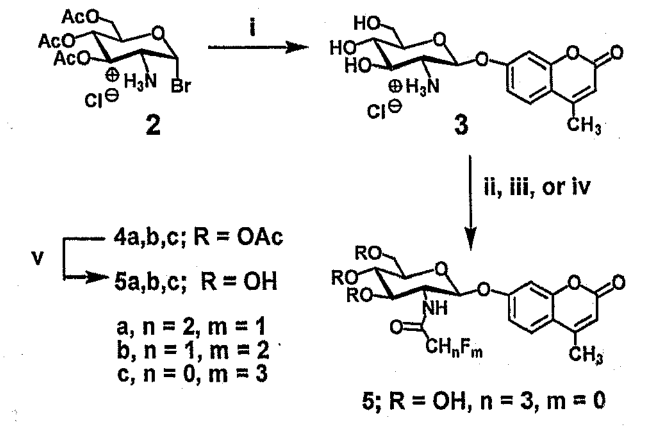


**i) a) Acetone, 4-MU, 4-MU·$Na^+$,b) $K_2CO_3$, $Et_2O$ c) HCl; ii) a) DOWEX-50 $H^+$, $NaOOCCH_2F$ b) DMF, $Et_3N$, py, DCC, 4 °C; iii) DMF, $Et_3N$, py, DCC, $F_2HCCOOH$, 4 °C; iv) DMF, $Et_3N$, py, DCC, $(F_3CCO)_2O$, 4 °C; v) a) NaOMe, MeOH, b) DOWEX-50 $H^+$.**

*1.2.1 general procedure for synthesis of substrate analogues*

All buffer salts used in this study were obtained from Sigma-Aldrich. Dry methanol and toluene were purchased from Acros Organics. Dicloromethane and triethylamine were dried by distillation over $CaH_2$ prior to use. β-Hexosaminidase was purchased from Sigma (Lot 043K3783). STZ was purchased from Sigma-Aldrich and samples were freshly dissolved just prior to assays. PUGNAc was obtainined from Toronto Research Chemicals. All other reagents were purchased from Sigma-Aldrich and used without further purification. Milli-Q (18.2 mΩ $cm^{-1}$) water was used to prepare all buffers. Synthetic reactions were monitored by TLC using Merck Kieselgel 60 $F_{254}$ aluminum-backed sheets. Compounds were detected by charring with 10% ammonium molybdate in 2 M $H_2SO_4$ and heating. Flash chromatography under a positive pressure was performed with Merck Kieselgel 60 (230-400 mesh) using the specified eluants. $^1H$ NMR spectra were recorded on a Varian AS500 Unity Innova spectrometer at 500 MHz (chemical shifts quoted relative to $CDCl_3$, $CD_3OD$ or $(CD_3)_2SO$ where appropriate). $^{19}F$ NMR spectra were recorded on a Varian AS500 Unity Innova spectrometer at 470 MHz and are proton-coupled with $CF_3CO_2H$ as a reference. $^{13}C$ NMR spectra were recorded on a Varian AS500 Unity Innova spectrometer at 125 MHz (chemical shifts quoted relative to $CDCl_3$, $CD_3OD$, or $(CD_3)_2SO$). Elemental analyses of all compounds used in cell culture and enzyme assay were performed at the Simon Fraser University Analytical Facility.

*1.2.2 Synthesis of 4-methylumbelliferone 2-deoxy-2-acetamido-β-D-glucopyranosides*

4-Methylumbelliferyl 2-amino-2-deoxy-β-D-glucopyranoside hydrochloride (3) was prepared essentially as described by Roeser and Legler [84] and was used without further purification.

*1.2.3 Synthesis of 4-Methylumbelliferyl 3,4,6-tri-O-acetyl-2-deoxy-2-fluoroacetamido-β-D-glucopyranoside* (**4a**)

To a cooled (0 ˚C) solution of the hydrochloride salt 3 (0.50 g, 1.0 mmol) in a solution of dimethylformamide (DMF, 10 mL) was added triethylamine (0.3 mL, 0.21 g, 2.1 mmol) and dry pyridine (10 mL). To a stirred mixture of dry DMF (45 mL) containing dried DOWEX-50 $H^+$ resin (6 g) was added sodium fluoroacetate (0.9 g). After one hour dicyclohexylcarbodiimide (DCC, 1.6 g, 7.8 mmol) and 30 mL of the fluoroacetic acid solution (6.0 mmol) was added *via* canula to the reaction vessel containing the hydrochloride salt 3. The resulting solution was allowed to stand for 16 h at 0 ˚C after which time the reaction was judged complete by TLC analysis. The solvent was partially removed *in vacuo* after which ethyl acetate (50 mL) and a solution of saturated sodium chloride (20 mL) were added. The organic layer was collected and the aqueous layer was extracted twice with ethyl acetate. The combined organic extracts were washed successively with water, twice with saturated sodium bicarbonate, and finally with a solution of saturated sodium chloride. The organic extracts were dried over $MgSO_4$, filtered, and the solvent removed *in vacuo* to yield a light yellow syrup. The desired product was purified using flash column silica chromatography (2:1; ethyl acetate - hexanes) to yield the partially purified desired compound as an amorphous white solid (≈ 356 mg, 0.68 mmol, 68 %) that was used in the next

step without further purification.

*1.2.4 Synthesis of 4-Methylumbelliferyl 3,4,6-tri-O-acetyl-2-deoxy-2-difluoroacetamido-β-D-glucopyranoside* (**4b**)

To a cooled (0 ˚C) solution of the hydrochloride salt **3** (0.15 g, 0.3 mmol) in a solution of dimethylformamide (DMF, 6 mL) was added triethylamine (0.09 mL, 0.063 g, 0.62 mmol) and dry pyridine (3 mL). Dicyclohexylcarbodiimide (DCC, 0.48 g, 2.3 mmol) and difluoroacetic acid (0.12 mL, 0.18 g, 1.3 mmol) was added to the reaction mixture *via* syringe. The resulting solution was allowed to stand for 16 h at 0 ˚C after which time two drops of difluoroacetic acid were added. After a further 3.5 h at RT the reaction was judged complete by TLC analysis. The solvent was partially removed *in vacuo* after which ethyl acetate (50 mL) and a solution of saturated sodium chloride (20 mL) were added. The organic layer was collected and the aqueous layer was extracted twice with ethyl acetate. The combined organic extracts were washed successively with water, twice with saturated sodium bicarbonate, and finally with a solution of saturated sodium chloride. The organic extracts were dried over $MgS_4O$, filtered, and the solvent removed *in vacuo* to yield a light yellow syrup. The desired product was purified using flash column silica chromatography using a gradient solvent system (1:1; hexanes - ethyl acetate) to yield the partially purified desired compound as a white amorphous solid (≈ 0.10 mg, 0.19 mmol, 64 %) that was used in the next step without further purification.

*1.2.5 Synthesis of 4-Methylumbelliferyl 3,4,6-tri-4-acetyl-2-deoxy-2-trifluoroacetamido-β-D-glucopyranoside* (**4c**)

To a cooled (0 ˚C) solution of the hydrochloride salt **3** (0.10 g, 0.2 mmol) in a solution of dimethylformamide (DMF, 6 mL) was added triethylamine (0.06 mL, 0.42 g, 0.41 mmol). The reaction mixture was then cooled to 0 ˚C and trifluoroacetic anhydride (0.08 mL, 0.12 g, 5.7 mmol) was added *via* syringe. The resulting solution was allowed to stand for 16 h at 0 ˚C after which time the reaction was judged complete by TLC analysis. The reaction mixture was then diluted with ethyl acetate (20 mL) and a solution of saturated sodium chloride (40 mL) was added. The organic layer was collected and the aqueous layer was extracted twice with ethyl acetate. The combined organic extracts were washed successively with water, twice with saturated sodium bicarbonate, and finally with a solution of saturated sodium chloride. The organic extracts were dried over $MgSO_4$, filtered, and the solvent removed *in vacuo* to yield a light yellow syrup. The desired product was purified using flash column silica chromatography using a gradient solvent system (1:1; hexanes - ethyl acetate) to yield the partially purified desired compound as an amorphous white solid (≈ 0.93 g, 0.17 mmol, 82 %) that was used in the next step without further purification.

*1.2.6 General procedure for the synthesis of 4-methylumbelliferyl 2-deoxy-2-fluoroacetamido-β-D-glucopyranosides* (**5a-c**)

To a solution of each glycoside in dry methanol was added a spatula tip of anhydrous sodium methoxide. The resulting basic solution was stirred under nitrogen until the reaction was judged complete by TLC analysis. Dowex-50 $H^+$ resin was added to the stirred reaction mixture until the pH of the solution became neutral. The suspension was filtered and the filter cake rinsed extensively with methanol after which the solvent from the combined filtrates was removed *in vacuo.* The desired deprotected glycosides were isolated by flash column silica chromatography using the following solvent systems: ethyl acetate - methanol - water (12:1:1) for the *N*-tri- and *N*-difluoroacetyl derivatives (**5b** and **5c**) and ethyl acetate - methanol (1:1) for the *N*-monofluoroacetyl derivative **(5a).** Products were recrystallized from ethanol and diethyl ether to yield the desired products with the overall yields over two steps of 66% for the *N*-trifluoroacetyl derivative **(5c),** 37% for the *N*-difluoroacetyl derivative (**5b**), and 45% for the *N*-fluoroacetyl derivative **(5a).**

*4-Methylumbelliferyl 2-deoxy-2-fluoroacetamido-β-D-glucopyranoside* (MuGlcNAc-F) (5a) - $^1$H-NMR (500 MHz, d6-DMSO) δ 7.71 (1 H, d, $J_{H5AR\text{-}H6AR}$ = 8.8, H-$5_{AR}$), 7.00 (1 H, $J_{H8AR\text{-}H6AR}$ = 2.4, H-$8_{AR}$), 6.96 (1 H, dd, H-$6_{AR}$), 6.26 (1 H, d, $J_{H3AR\text{-}CH3}$ = 1.1, H-$3_{AR}$), 5.21 (1 H, d, $J_{H1\text{-}H2}$ = 8.5, H-1), 4.81 (2 H, d, $J_{H\text{-}F}$ = 47.0, $CH_2F$), 3.86 (1 H, dd, $J_{H2\text{-}H3}$ = 9.9, H-2), 3.74 (1-H, dd, $J_{H6\text{-}H6'}$ = 11.7, $J_{H6\text{-}H5}$ = 1.7, H-6), 3.53-3.46 (2 H, m, H-3, H-6'), 3.38 (1 H, ddd, $J_{H5\text{-}H4}$ = 9.6, $J_{H5\text{-}H6'}$ = 6.0, H-5), 3.20 (1 H, dd, $J_{H4\text{-}H3}$ = 8.9, H-4), 2.39 (1 H, d, $CH_3$) ppm; $^{19}$F-NMR (500 MHz, d6-DMSO) -225.24 ppp (dd, $J_{F\text{-}H}$ = 53). Analytical calculated for $C_{18}H_{20}FNO_8$; C, 54.41; H, 5.07; N, 3.53; Experimental C, 54.20; H, 4.97; N 3.59.

*4-Methylumbelliferyl 2-deoxy-2-difluoroacetamido-β-D-glucopyranoside (MuGlcNAc-$F_2$) (**5b**)* - $^1$H-NMR (500 MHz, d6-DMSO) δ 7.69 (1 H, d, $J_{H5AR\text{-}H6AR}$ = 8.8, H-$5_{AR}$), 6.97 (1 H, $J_{H8AR\text{-}H6AR}$ = 2.4, H-$8_{AR}$), 6.92 (1 H, dd, H-$6_{AR}$), 6.24 (1 H, d, $J_{H3AR\text{-}CH3}$ = 1.2, H-$3_{AR}$), 6.21 (1 H, d, $J_{H\text{-}F}$ = 53.6 Hz, $CHF_2$), 5.16 (1 H, d, $J_{H1\text{-}H2}$ = 8.5, H-1), 3.79 (1 H, dd, $J_{H2\text{-}H3}$ = 10.3, H-2), 3.72 (1-H, dd, $J_{H6\text{-}H6'}$ = 11.8, $J_{H6\text{-}H5}$ = 1.9, H-6), 3.53-3.42 (2 H, m, H-3, H-5, H-6'), 3.20 (1 H, dd, $J_{H4\text{-}H5}$ = 9.6 Hz, $J_{H4\text{-}H3}$ = 8.9 Hz, H-4), 2.38 (1 H, d, $CH_3$) ppm; $^{19}$F-NMR (500 MHz, d6-DMSO) -127.32 (d, J = 54 Hz); Analytical calculated for $C_{18}H_{19}F_2NO_8$; C, 52.05; H, 4.61; N, 3.37; Experimental C, 51.92; H, 4.62; N, 3.31.

*4-Methylumbelliferyl 2-deoxy-2-trifluoroacetamido-β-D-glucopyranosid e(MuGlcNAc-$F_3$) (**5c**)* -$^1$H-NMR (500 MHz, d6-DMSO) δ 7.70 (1 H, d, $J_{H5AR\text{-}H6AR}$ = 8.8, H-$5_{AR}$), 6.86 (1 H, $J_{H8AR\text{-}H6AR}$ = 2.3, H-$8_{AR}$), 6.91 (1 H, dd, H-$6_{AR}$),

6.25 (1 H, d, $J_{H3AR-CH3}$ = 1.2, H-$3_{AR}$), 5.16 (1 H, d, $J_{H2-H2}$ = 8.5, H-1), 3.80 (1 H, dd, $J_{H2-H3}$ = 10.2, H-2), 3.72 (1-H, dd, $J_{H6-H6'}$= 11.7, $J_{H6-H5}$ = 1.8, H-6), 3.50-3.42 (2 H, m, H-6', H-3, H-5), 3.22 (1 H, dd, $J_{H4-H5}$ = 9.5 Hz, $J_{H4-H3}$ = 8.9 Hz, H-4), 2.38 (1 H, d, $CH_3$) ppm; $^{19}$F-NMR (500 MHz, d6-DMSO) -77.29; Analytical calculated for $C_{18}H_{18}F_3NO_8$; C, 49.89; H, 4.19; N, 3.23; Experimental C, 49.80; H, 4.29; N, 3.11.

## 1.3 Kinetic analyses of O-GlcNAcase and β-hexosaminidase

### *1.3.1 Experimental procedure for kinetic analyses*

All assays were carried out in triplicate at 37 °C for 30 minutes using a stopped assay procedure in which the enzymatic reactions (25 μL) were quenched by the addition of a 6 fold excess (150 μL) of quenching buffer (200 mM glycine, pH 10.75). Assays were initiated by the addition, *via* syringe, of enzyme (3 μL), and in all cases the final pH of the resulting quenched solution was greater than 10. Time dependent assay of β-hexosaminidase and O-GlcNAcase revealed that both enzymes were stable over this period in their respective buffers; 50 mM citrate, 100 mM NaCl, 0.1% BSA, pH 4.25 and 50 mM $NaH_2PO_4$, 100 mM NaCl, 0.1% BSA, pH 6.5. The progress of the reaction at the end of thirty minutes was determined by measuring the extent of 4-methylumbelliferone liberated as determined by fluorescence measurements using a Varian CARY Eclipse Fluoresence-Spectrophotometer 96-well plate system and comparison to a standard curve of 4-methylumbelliferone under identical buffer conditions. Excitation and emission wavelengths of 368 and 450 nM were used, respectively, with 5 mm slit openings. Human placental β-hexosaminidase was purchased from Sigma-Aldrich (Lot 043K3783). The cloning and expression of *O*-GlcNAcase is described in the literature [85], Both enzymes were dialyzed against PBS buffer and their concentrations determined using the Bradford assay. The concentration (μg/μl) of β-hexosaminidase and *O*-GlcNAcase used in assays with fluorinated substrates were as follows: for 4-methylumbelliferyl 2-acetamido-2-deoxy-β-D-glucopyranoside (MuGlcNAc) **(5):** 0.00077, 0.0126; MuGlcNAc-F **(5a):** 0.0031, 0.0189; MuGlcNAc-$F_2$ **(5b):** 0.0154, 0.0756, and for MuGlcNAc-$F_3$ **(5c):** 0.0154, 0.01523. In addition, β-hexosaminidase and O-GlcNAcase were used at a concentration (μg/μL) of 0.0154 and 0.0378, respectively to test the inhibitors using substrate **5** at a concentration of 0.64 mM. All inhibitors were tested at eight concentrations ranging from 5 times to 1/5$^{th}$ $K_I$ with the exception of the assay of inhibitor **8e** with β-hexosaminidase, where such high concentrations of inhibitor could not be reached owing to the high $K_I$ value of **8e.** $K_I$ values were determined by linear regression of data in Dixon plots. Where necessary, assays were carried out in triplicate and error bars are included in plots of the data.

### *1.3.2 Results from kinetic analyses using substrate analogues*

These compounds were first tested with the lysosomal human β- hexosamindase since this enzyme is known to proceed via a mechanism involving anchimeric assistance (Figure 2A). Although Michaelian saturation kinetics were not observed for all substrates (Table 1), $V_{max}[E]_o/K_M$, which is proportional to the second order rate constant governing the enzyme catalyzed reaction, could be determined from the initial slope of the Michaelis-Menten plot (Figure 2A Inset). A plot of log $V_{max}[E]_o/K_M$ against the Taft electronic parameter (σ*) of the N-acyl substituent shows a negative linear correlation on increasing fluorine substitution (Figure 2C). As expected, decreasing the basicity of the carbonyl oxygen has a deleterious effect on catalysis. The steep negative slope of the Taft-like linear free energy analysis (given by the reaction constant, p = -1.0 ± 0.1) suggests that the carbonyl oxygen acts as a nucleophile, attacking the anomeric center.

Table 1: Michaelis-Menten parameters for the β-hexosaminidase and O-GlcNAcase catalyzed hydrolysis of a series of 4-methylumbelliferone 2-N-acetyl-2-deoxy-β-D-glucopyranosides.

| Substrate | σ*[a] | Enzyme | $K_M$ (mM) | $V_{max}$ $[E]_o$ (μmol min$^{-1}$ mg$^{-1}$) | $V_{max}$ $[E]_o$ / $K_M$ (μmol mM$^{-1}$ min$^{-1}$ mg$^{-1}$) |
|---|---|---|---|---|---|
| MuGlcNAc **(5)** | 0.0 | Hexosaminidase | 0.88 ± 0.05 | 8.3 ± 0.2 | 9.5 ± 0.8 |
| | | *O*-GlcNAcase | 0.43 ± 0.04 | 0.74 ± 0.02 | 1.7 ± 0.2 |
| MuGlcNAc-$F_1$ **(5a)** | 0.8 | Hexosaminidase | 3.8 ± 0.4[b] | 3.3 ± 0.2[b] | 0.40 ± 0.07[d] |
| | | *O*-GlcNAcase | 0.49 ± 0.06 | 0.60 ± 0.03 | 1.2 ± 0.2 |
| MuGlcNAc-$F_2$ **(5b)** | 2.0 | Hexosaminidase | ND[c] | ND[c] | 0.069 ± 0.001[d] |
| | | *O*-GlcNAcase | 0.45 ± 0.05 | 0.16 ± 0.01 | 0.36 ± 0.06 |

(continued)

| Substrate | $\sigma^{*a}$ | Enzyme | $K_M$ (mM) | $V_{max}$ $[E]_o$ (μmol $min^{-1}$ $mg^{-1}$) | $V_{max}$ $[E]_o$ / $K_M$ (μmol $mM^{-1}$ $min^{-1}$ $mg^{-1}$) |
|---|---|---|---|---|---|
| MuGlcNAc-$F_3$ **(5c)** | 2.8 | Hexosaminidase | ND[c] | ND[c] | 0.0077 ± 0.0010[d] |
| | | *O*-GlcNAcase | 0.38 ± 0.03 | 0.033 ± 0.001 | 0.0077 ± 0.0008 |

[a]The Taft electronic parameters (σ*) used for each N-acyl substituent were obtained from Hansch and Leo [86].
[b]Values were estimated by non-linear regression of the Michelis-Menten data. Note that substrate concentrations assayed matched but did not exceed $K_M$ due to limited substrate solubility.
[c]These values could not be determined as saturation kinetics were not observed owing to limitations in substrate solubility.
[d]Values were determined by linear regression of the second order region of the Michelis-Menten plot.

This data is consistent with a mechanism involving electrophilic migration of the anomeric center and consequent oxocarbenium-ion like transition states that have been generally proposed for enzyme-catalyzed glycoside hydrolysis [80,82] . For O-GlcNAcase, Michaelian saturation kinetics were observed for all four substrates, therefore, both kinetic parameters were determined (Table 1, Figure 2B). For O-GlcNAcase the value of $V_{max}[E]_o/K_M$ is also dependant on the extent of fluorine substitution but the slope is more shallow (p = -0.42 ± 0.08, Figure 2C). On the basis of these results it appears that O-GlcNAcase, in common with lysosomal β-hexosaminidase, uses a catalytic mechanism involving anchimeric assistance.

The reaction constant (ρ), which is the slope of the correlations in the Taft-like analyses, is an indication of the sensitivity of the reaction to different substituents. This constant can be considered a function of both an electronic component (ρ*, which is governed by sensitivity of the reaction to the electronic parameter of the substituents, σ*) and a steric component (δ, which is governed by sensitivity of the reaction to the steric Taft parameters of the substituent, $E_s$) according to the following equation.

$$\rho = \rho^* + \delta \qquad \text{(Eqn 1)}$$

The difference between the slopes measured for lysosomal β-hexosaminidase and O-GlcNAcase may thus reflect the position of the transition state along the reaction coordinate or may indicate that the lysosomal β-hexosaminidase has a more sterically constrained active site architecture than does O-GlcNAcase. Indeed, a common misconception is that fluorine (147 pm Van der Waals radius and 138 pm C-F bond length) is often considered to have a negligible difference in size as compared to hydrogen (120 pm Van der Waals radius and 109 pm C-H bond length). Therefore, it is possible that unfavorable steric interactions between the substrate and the active site of human β-hexosaminidase may play an additional role, beyond electronics, in discriminating between the varying levels of fluorine substitution. Indeed, the recent crystal structures of human hexosaminidase B revealed a carefully structured pocket that tightly nestles the acetamido group between three tryptophan residues [78, 87]. For O-GlcNAcase, no three-dimensional structure is available. The relatively constant $K_M$ values measured for all of the substrate analogues suggest, however, that steric effects are not a major contributor and that the electronic effect of the N-acyl-fluorine substituents predominate. An earlier study on an isolated enzyme of unknown family using two para-nitrophenyl 2-acetamido-2-deoxy-β-D-glucopyanoside (pNP-GlcNAc) with either two or three fluorine substituents in the acetamido group yielded a ρ* of -1.41 ± 0.1. [88] This value is even greater than that found for the lysosomal β-hexosaminidase in this study (ρ*=-1.0 ± 0.1) suggesting that the enzyme Kosman and Jones studied from Aspergillus niger is more likely a member of family 20 of glycoside hydrolases than family 84.

*1.3.3 Results from kinetic analyses using NAG-Thiazoline as an inhibitor*

As a further test of whether O-GlcNAcase uses a catalytic mechanism involving anchimeric assistance, the inhibitor NAG-thiazoline (**9a**) was tested with this enzyme. NAG-thiazoline, designed as a mimic of the bicyclic oxazoline intermediate, has been previously demonstrated to function as an inhibitor of family 20 hexosaminidases [30, 48]. Using pNP-GlcNAc as a substrate, NAG-thiazoline was found to be a potent inhibitor of family 84 human O-GlcNAcase and a clear pattern of competitive inhibition was observed (Figure 3). Non-linear regression revealed a $K_I$ value of 180 nM at pH 7.4 and analysis using MU-GlcNAc (**5**) revealed a $K_I$ value of 70 nM at pH 6.5 (Table 2). NAG-Thiazoline is therefore

a potent inhibitor of O-GlcNAcase, binding approximately 21000-fold more tightly than the parent saccharide, GlcNAc ($K_I$ =1.5 mM) at pH 6.5. This potent inhibition may be attributed to the resemblance of NAG-thiazoline to a putative oxazoline intermediate or a structurally related transition state. Indeed, the observed inhibition data is similar to that measured by the inventors for the family 20 human lysosomal β-hexosaminidase ($K_I$ = 70 nM, 17000-fold more tightly than GlcNAc for which the $K_I$ value is 1.2 mM) and strongly supports the Taft-like analysis indicating that O-GlcNAcase, in common with the family 20 β-hexosaminidases, uses a catalytic mechanism involving anchimeric assistance. Glycoside hydrolases from families 18 [89] and 56 [90] , which are endo-glycosidases acting to cleave oligosaccharide chains, have also been shown to use a mechanism involving anchimeric assistance [91]. Thus families 18, 20, 56 and 84 are all comprised of retaining glycosidases that use a catalytic mechanism involving anchimeric assistance from the acetamido group of the substrate. This is in marked contrast to retaining β-glycosidases from families 3 and 22 that also act on substrates bearing an acetamido group at the 2-position of their respective substrates [81, 82] as well as the inverting family 23 inverting endo-glycosidases.

Table 2: Inhibition constants and selectivity of inhibitors for both *O*-GlcNAcase and β-Hexosaminidase. All $K_I$ values were determined using linear regression of a Dixon plots.

| Compound | *O*-GlcNAcase $K_I$ (μM) | β-Hexosaminidase $K_I$ (μM) | Selectivity Ratio (β-Hex $K_I$ / *O*-GlcNAcase $K_I$) |
|---|---|---|---|
| GlcNAc | 1500 | 1200 | 0.8 |
| STZ | $1500^2$ | $47000^2$ | 31 |
| PUGNAc | 0.046 | 0.036 | 0.8 |
| **9a** | 0.070 | 0.070 | 1 |
| **9b** | 0.12 | 32 | 270 |
| **9c** | 0.23 | 340 | 1500 |
| **9d** | 1.5 | 4600 | 3100 |
| **9e** | 57 | 11000 | 100 |
| **9f** | 1.6 | 720 | 700 |
| **9g** | 5.7 | 4000 | 190 |

Example 2: Design and Synthesis of a First Embodiment of Selective O-GlcNAcase Inhibitors

2.1 Design of a First Embodiment of Selective O-GlcNAcase Inhibitors

With the mechanism of human O-GlcNAcase, and by extension other members of family 84 of glycoside hydrolases, established, attention was turned to using this information in the design of inhibitors that would be selective for this enzyme over the human lysosomal β-hexosaminidase. Because both β-hexosaminidase and O-GlcNAcase use a mechanism involving anchimeric assistance, NAG-thiazoline was chosen as a scaffold that could be elaborated to generate the required selectivity. Three observations provided a starting point in the design of the inhibitor. The first is that the slope of the Taft-like analysis for the lysosomal enzyme is much steeper than that measured for O-GlcNAcase thereby suggesting that the bulk of the N-acyl group may be a determinant in substrate recognition (*vide supra*). The second, and related, consideration is that the structure of the human lysosomal ß-hexosaminidase B reveals a snug pocket into which the methyl group of the acetamido substituent is poised [78] . The third is that STZ, which bears a bulky N-acyl substituent shows some selectivity for O-GlcNAcase over β-hexosaminidase [46] .

2.2 Synthesis of a First Embodiment of Selective O-GlcNAcase Inhibitors

A series of seven inhibitors were prepared in which the thiazoline ring was elaborated with aliphatic chains of increasing length in the expectation that these compounds would allow the discriminative inhibition of O-GlcNAcase over lysosomal hexosaminidase. The synthesis of this panel of inhibitors is outlined in Scheme 2. This facile synthetic route enables the production of quantities of inhibitor from commercially available starting materials in three steps or from the inexpensive starting material 2-amino-2-deoxy-glucopyranose in six steps.

**Scheme 2:**

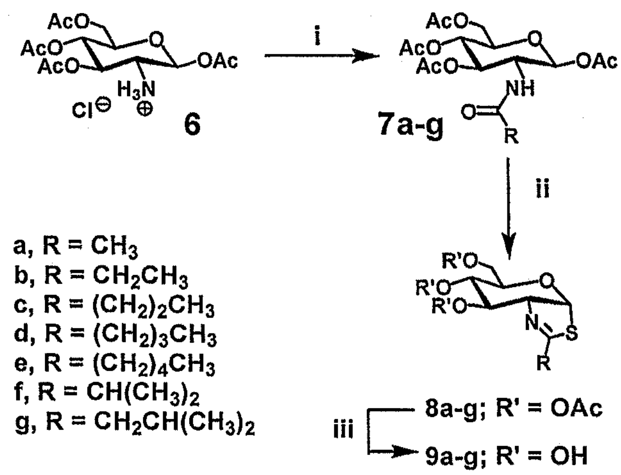


**i) RCOCl, $Et_3N$, $CH_2Cl_2$, 0 °C; ii) Lawesson's Reagent, Tol, Δ, iii) a) NaOMe, MeOH, b) DOWEX-50 $H^+$.**

*2.2.1 General procedure for synthesis of compounds*

The general procedures are the same as for the synthesis of the substrate analogues set out above.

*2.2. 2 General procedure for the synthesis of 1,3,4,6-tetra-O-acetyl-2-deoxy-2-N-acyl-β-D-glucopyranose* **(7a-g)**

To a solution of the hydrochloride salt of 2-amino-2-deoxy-1,3,4,6-tetra-*O*-acetyl-β-D-glucopyranose **(6)** [92] in 1 volume of dry dichloromethane was added 2 equivalents of dry triethylamine at which time the starting material dissolved. The reaction mixture was cooled to 0 ˚C and 1.2 equivalents of the appropriate acyl chloride was added via syringe. The resultant mixture was stirred for approximately two hours at room temperature. When the reaction mixture was judged complete by TLC analysis, 5 volumes of ethyl acetate were added. The resulting organic phase was washed successively with water, 1 M NaOH, and saturated sodium chloride. The organic phase was dried over $MgSO_4$, filtered, and concentrated to yield a white crystalline solid. The material was recrystallized using a mixture of ethyl acetate and hexanes to yield the desired *N*-acylated materials in yields ranging from 46 to 74 %.

*1,3,4,6-tetra-O-acetyl-2-deoxy-2-N-propyl-β-D-glucopyranose (**7b**)- $^1H$* NMR (500M Hz, $CDCl_3$) δ: 5.68 (1 H, d, $J_{H1,H2}$ = 8.8 Hz, H-1), 5.42 (1 H, d, $J_{NH,H2}$ = 6.7 Hz, NH), 5.14 (1 H, dd, $J_{H3,H4}$ = 8.7 Hz, H-3), 5.12 (1 H, dd, $J_{H4,H5}$ = 8.7 Hz, H-4), 4.34 (1 H, ddd, $J_{H2,H3}$ = 8.7 Hz, H-2), 4.27 (1 H, dd, $J_{H6,H6'}$ = 12.5 Hz, $J_{H5,H6}$ = 4.6 Hz, H-6), 4.12 (1 H, dd, $J_{H5,H6'}$ = 2.1 Hz, H-6'), 3.79-3.75 (1 H, m, H-5), 2.12 (3 H, s, OAc), 2.10 (3 H, s, OAc), 2.04 (3 H, s, OAc), 2.02 (3 H, s, OAc), 2.15-2.10 (2 H, m, H-7), 0.90 (3 H, t, $J_{H8,H7}$ = 7.4 Hz, H-8) ppm. $^{13}C$ NMR (125 MHz, MeOD) δ: 174.1, 171.5, 170.9, 169.8, 169.5, 92.9, 73.2, 72.8, 67.9, 61.8, 53.1, 30.0, 21.1, 21.0, 20.9, 20.8, 10.1 ppm.

*1,3,4,6-tetra-O-acetyl-2-deoxy-2-N-butyl-β-D-glucopyranose (7**c**)*- $^1H$ NMR (500 MHz, $CDCl_3$) δ: 5.73 (1 H, d, $J_{H1,H2}$ = 8.8 Hz, H-1), 5.58 (1 H, d, $J_{NH,H2}$ = 9.5 Hz, NH), 5.21-5.14 (2 H, m, H-3/H-4), 4.38 (1 H, ddd, H-2), 4.31 (1 H, dd, $J_{H6,H6'}$ = 12.5 Hz, H-6), 4.16 (1 H, dd, $J_{H5,H6'}$ = 2.2 Hz, H-6'), 3.84 (1 H, ddd, $J_{H5,H6}$ = 4.7 Hz, H-5), 2.16 (3 H, s, OAc), 2.13 (3 H, s, OAc), 2.18-2.10 (2 H, m, H-7), 2.08 (3 H, s, OAc), 2.05 (3 H, s, OAc), 1.64 (2 H, ddd, H-8), 0.94 (3 H, t, $J_{H8,H9}$ = 7.4 Hz, H-9) ppm. $^{13}C$ NMR (125 MHz, MeOD) δ: 173.1, 171.4, 170.9, 169.8, 169.5, 92.9, 73.2, 72.8, 67.9, 61.9, 53.0, 38.8, 21.1, 21.0, 20.9, 20.8, 19.2, 13.7 ppm.

*1,3,4,6-tetra-O-acetyl-2-deoxy-2-N-pentyl-β-D-glucopyranose (**7d**)-$^1H$* NMR (500 MHz, $CDCl_3$) δ: 5.68 (1 H, d, $J_{H1,H2}$ = 8.8 Hz, H-1), 5.50 (1 H, d, $J_{NH,H2}$ = 9.5 Hz, NH), 5.18-5.12 (2 H, m, H-3/H-4), 4.36-4.30 (1 H, m, H-2), 4.26 (1 H, dd, $J_{H6,H6'}$ = 12.5 Hz, H-6), 4.13 (1 H, dd, $J_{H5,H6'}$ = 2.2 Hz, H-6'), 3.82-3.77 (1 H, m, $J_{H5,H6}$ = 4.5 Hz, H-5), 2.11 (3 H, s, OAc), 2.11 (2 H, m, H-7), 2.09 (3 H, s, OAc), 2.04 (3 H, s, OAc), 2.02 (3 H, s, OAc), 1.56 (2 H, dd, $J_{H7,H8}$ = 7.5 Hz, H-8), 1.52 (2 H, dd, $J_{H7',H8}$ = 7.5 Hz, H-8), 1.26 (2 H, ddq, $J_{H8,H9}$ = 7.5 Hz, H-9), 0.94 (3 H, dd, $J_{H9,H10}$ = 7.5 Hz, H-10) ppm. $^{13}C$ NMR (125 MHz, MeOD) δ: 173.4, 171.4, 170.9, 169.8, 169.5, 92.9, 73.2, 72.7, 67.9, 61.9, 53.0, 36.7, 27.9, 22.4, 21.1, 21.0, 20.9, 20.8, 13.9 ppm.

*1,3,4,6-tetra-O-acetyl-2-deoxy-2-N-hexyl-β-D-glucopyranose (**7e**)*- $^1H$ NMR (500 MHz, $CDCl_3$) δ: 5.69 (1 H,

d, $J_{H1,H2}$ = 8.8 Hz, H-1), 5.52 (1 H, d, $J_{NH,H2}$ = 9.5 Hz, NH), 5.14 (2 H, m, H-3/H-4), 4.37-4.30 (1 H, m, H-2), 4.26 (1 H, dd, $J_{H6,H6'}$ = 12.5 Hz, H-6), 4.15-4.08 (1 H, m, H-6'), 3.82-3.77 (1 H, m, $J_{H5,H6}$ = 4.7 Hz, H-5), 2.11 (3 H, s, OAc), 2.09 (3 H, s, OAc), 2.04 (3 H, s, OAc), 2.02 (3 H, s, OAc), 1.58-1.52 (2 H, m, H-7), 1.33-1.08 (6 H, m, H-8, H-9, H-10), 0.87 (3 H, t, $J_{H11,H10}$ = 7.1 Hz, H-11) ppm. $^{13}$C NMR (125 MHz, MeOD) δ: 173.4, 171.4, 170.9, 169.8, 169.5, 92.9, 73.2, 72.8, 67.9, 61.9, 60.6, 53.0, 36.9, 31.4, 25.5, 22.5, 21.1, 21.0, 20.9, 20.8, 14.1 ppm.

*1,3, 4, 6-tetra-O-acetyl-2-deoxy-2-N-isobutyl-β-D-glucopyranose* (***7f***)- $^1$H NMR (500 MHz, $CDCl_3$) δ: 5.70 (1 H, d, $J_{H1,H2}$ = 8.8 Hz, H-1), 5.58 (1 H, d, $J_{NH,H2}$ = 9.6 Hz, NH), 5.18-5.12 (2 H, m, H-3/H-4), 4.36-4.30 (1 H, m, H-2), 4.27 (1 H, dd, $J_{H6,H6'}$= 12.5 Hz, H-6), 4.12 (1 H, dd, $J_{H5,H6'}$= 2.2 Hz, H-6'), 3.84-3.87 (1 H, m, $J_{H5,H6}$ = 4.8 Hz, H-5), 2.10 (3 H, s, OAc), 2.08 (3 H, s, OAc), 2.28 (1 H, m, H-7), 2.04 (3 H, s, OAc), 2.03 (3 H, s, OAc), 1.08 (6 H, t, $J_{H8,H7}$ = 2.8 Hz, H-8) ppm. $^{13}$C NMR (125 MHz, MeOD) δ: 177.2, 171.5, 170.9, 169.8, 169.5, 93.0, 73.3, 72.7, 67.9, 61.9, 52.9, 36.0, 21.0, 21.0, 20.8, 20.8, 19.6 ppm.

*1,3,4,6-tetra-O-acetyl-2-deoxy-2-N-isopentyl-β-D-glucopyranose (**7g**)*- $^1$H NMR (500 MHz, $CDCl_3$) δ: 5.67 (1 H, d, $J_{H1,H2}$ = 8.8 Hz, H-1), 5.58 (1 H, d, $J_{NH,H2}$ = 9.5 Hz, NH), 5.18-5.10 (2 H, m, H-3/H-4), 4.38-4.32 (1 H, m, H-2), 4.27 (1 H, dd, $J_{H6,H6'}$ = 12.5 Hz, H-6), 4.12 (1 H, $_1$, $J_{H5,H6'}$ = 2.2 Hz, H-6'),3.82-3.78 (1 H, m, $J_{H5,H6}$ = 4.6 Hz, H-5), 2.10 (3 H, s, OAc), 2.08 (3 H, s, OAc), 2.06-2.01 (2 H, m, H-7), 2.04 (3 H, s, OAc), 2.$_0$3 (3 H, s, OAc), 1.98 (1 H, ddd, H-8), 0.94 (6 H, d, $J_{H8,H9}$ = 6.5 Hz, H-9) ppm. $^{13}$C NMR (125 MHz, MeOD) δ: 172.6, 171.4, 170.9, 169.8, 169.5, 92.9, 73.2, 72.7, 67.9, 61.8, 52.8, 46.2, 26.3, 22.4, 22.3, 21.1, 21.0, 20.9, 20.8 ppm.

*2.2. 3 General procedure for the synthesis of 3,4,6-tri-O-acetyl-1, 2-dideoxy-2'-alkyl-α-D-glucopyranoso-[2,1-d]-Δ2'-thiazoline (3,4,6-tri-O-acetyl-NAG-thiazoline analogues)* (**8a-g**)

To a solution of the appropriate 1,3,4,6-tetra-O-acetyl-2-N-acyl-2-deoxy-β-D-glucopyranose (**7a-g**) in anhydrous toluene was added Lawesson's Reagent (0.6 equivalents) and the reaction mixture was refluxed for 2 h after which time the reaction was judged to be complete by TLC analysis. The solution was cooled to room temperature and the solvent was removed in vacuo. The residue was dissolved in toluene and the desired material was isolated by flash column silica chromatography using a solvent system of hexanes and ethyl acetate in ratios ranging from 4:1 to 1:2 as appropriate. Products were isolated in yields ranging from 62 to 83 %. 3,4,6-tri-O-acetyl-1,2-dideoxy-2'-ethyl-α-D-glucopyranoso-[2,1-d]-Δ2'-thiazoline (**8a**) has been previously prepared using similar reaction conditions as described above.[30] All spectral characterization agreed with the literature values.

*3,4,6-tri-O-acetyl-1,2-dideoxy-2'-ethyl-α-D-glucopyranoso-[2,1-d]-Δ2'-thiazoline (**8b**)* -$^1$H NMR (500 MHz, $CDCl_3$)δ:6.28(1H, d, $J_{H1,H2}$ = 7.4 Hz, H-1), 5.60 (1 H, dd, $J_{H3,H4}$ = 3.3 Hz, H-3), 4.97 (1 H, d, $J_{H4,H5}$ = 9.3 Hz, H-4), 4.56-4.52 (1 H, m, H-2), 4.18-4.10 (2 H, m, H-6/ H-6'), 3.58 (1 H, ddd, $J_{H5,H6}$ = 3.2 Hz, H-5), 2.74-2.67 (1 H, m, H-7), 2.14 (3 H, s, OAc), 2.11 (3 H, s, OAc), 2.09 (3 H, s, OAc), 1.29 (3 H, t, $J_{H8,H7}$ = 7.60 Hz, H-8) ppm.

*3,4,6-tri-O-acetyl-1,2-dideoxy-2'propyl-α-D-glucopyranoso-[2,1-d]-Δ2'-thiazoline (8c)* - $^1$H NMR (500 MHz, $CDCl_3$) δ: 6.26 (1 H, d, $J_{H1,H2}$ = 7.2 Hz, H-1), 5.58 (1 H, dd, $J_{H3,H4}$ = 3.3 Hz, H-3), 4.96 (1 H, d, $J_{H4,H5}$ = 9.2 Hz, H-4), 4.54-4.50 (1 H, m, H-2), 4.16-4.08 (2 H, m, H-6/H-6'), 3.58 (1 H, ddd, $J_{H5,H6}$ = 3.3 Hz, H-5), 2.70-2.58 (2 H, m, H-7), 2.14 (3 H, s, OAc), 2.08 (3 H, s, OAc), 2.07 (3 H, s, OAc), 1.76-1.69 (2 H, m, H-8), 1.00 (3 H, t, $J_{H9,H8}$ = 7.4 Hz, H-9) ppm.

*3,4,6-tri-O-acetyl-1,2-dideoxy-2'-butyl-α-D-glucopyranoso-[2,1-d]-Δ2'-thiazoline (**8d**)* - $^1$H NMR (500 MHz, $CDCl_3$) δ: 6.21 (1 H, d, $J_{H1,H2}$ = 7.2 Hz, H-1), 5.57 (1 H, dd, $J_{H3,H4}$ = 3.3 Hz, H-3), 4.94 (1 H, d, $J_{H4,H5}$ = 9.4 Hz, H-4), 4.48-4.44 (1 H, m, H-2), 4.12-4.07 (2 H, m, H-6/H-6'), 3.53 (1 H, ddd, $J_{H5,H6}$ = 3.0 Hz, H-5), 2.60-2.57 (2 H, m, H-7), 2.12 (3 H, s, OAc), 2.07 (6 H, s, OAc), 1.67-1.63 (2 H, m, H-8), 1.40 (2 H, ddd, $J_{H9,H8}$ = 7.3 Hz, H-9), 0.92 (3 H, t, $J_{H10,H9}$ = 7.4 Hz, H-10) ppm.

*3,4,6-tri-O-acetyl-1,2-dideoxy-2'-pentyl-α-D-glucopyranoso-[2,1-d]-Δ2'-thiazoline* (***8e***) - $^1$H NMR (500 MHz, $CDCl_3$) δ: 6.24 (1 H, d, $J_{H1,H2}$ = 7.2 Hz, H-1), 5.60 (1 H, s, H-3), 4.96 (1 H, d, $J_{H4,H5}$ = 9.4 Hz, H-4), 4.52-4.49 (1 H, m, H-2), 4.14-4.10 (2 H, m, $J_{H6,H6'}$ = 12.2 Hz H-6/H-6'), 3.56 (1 H, ddd, $J_{H5,H6}$ =3.1 Hz, $J_{H5,H6'}$, = 5.6 Hz, H-5), 2.65-2.60 (2 H, m, H-7), 2.14 (3 H, s, OAc), 2.09 (3 H, s, OAc), 2.08 (3 H, s, OAc), 1.71-1.68 (2 H, m, H-8), 1.38-1.33 (4 H, m, H-9/H-10), 0.91 (3 H, t, $J_{H11,H10}$ = 6.9 Hz, H-11) ppm.

3,4, *6-tri-O-acetyl-1,2-dideoxy-2'-isopropyl-α-D-glucopyranoso-[2,1-d]-Δ2'-thiazoline (**8f**)* -$^1$H NMR (500 MHz, $CDCl_3$) δ: 6.27 (1 H, d, $J_{H1,H2}$ = 7.2 Hz, H-1), 5.60 (1 H, m, H-3), 4.97 (1 H, d, $J_{H4,H5}$ = 9.3 Hz, H-4), 4.55-4.49 (1 H, m, H-2), 4.18-4.11 (2 H, m, H-6/H-6'), 3.60 (1 H, ddd, $J_{H5,H6}$ = 3.1 Hz, H-5), 2.55 (2 H, s, H-7), 2.15 (3 H, s, OAc), 2.09 (3 H, s, OAc), 2.08 (3 H, s, OAc), 1.07 (6 H, t, $J_{H8,H7}$ = 6.6 Hz, H-8) ppm.

*3,4,6-tri-O-acetyl-1,2-dideoxy-2'-isobutyl-α-D-glucopyranoso-[2,1-d]-Δ2'-thiazoline (**8g**)* - $^1$H NMR (500 MHz, $CDCl_3$) δ: 6.26 (1 H, d, $J_{H1,H2}$ = 7.2 Hz, H-1), 5.60 (1 H, dd, $J_{H3,H4}$ = 3.3 Hz, H-3), 4.97 (1 H, d, $J_{H4,H5}$ = 9.3 Hz, H-4), 4.56-4.50 (1 H, m, H-2), 4.16-4.10 (2 H, m, H-6/H-6'), 3.62-3.58 (1 H, m, $J_{H5,H6}$ = 3.1 Hz, H-5), 2.57-2.52 (2 H, m, H-7), 2.15 (3 H, s, OAc), 2.09 (3 H, s, OAc), 2.08 (3 H, s, OAc), 1.28-1.23 (2 H, m, H-8), 1.02 (6 H, t, $J_{H9,H8}$ = 6.6 Hz, H-9) ppm.

*2.2.4 General procedure for the synthesis of 1,2-dideoxy-2'-alkyl-α-D-glucopyranoso-[2,1-d]-Δ2'-thiazoline (NAG-thiazoline analogues)* (**9a-g**)

To a solution of the appropriate protected thiazoline (**8a-g**) in dry methanol was added a spatula tip of anhydrous sodium methoxide. The basic solution was stirred until the reaction was judged complete by TLC analysis (typically 2 hours). A solution of glacial acetic acid in methanol (1:20) was added dropwise to the reaction mixture until the pH of the solution was found to be neutral. The solvent was then removed *in vacuo* and the desired materials (**9a-g**) was isolated by flash column silica chromatography using a solvent system of ethyl acetate and methanol in ratios ranging from 2:1 to 6:1 as appropriate. Products were isolated in yields ranging from 86 to 99 %. 1,2-dideoxy-2'-ethyl-α-D-glucopyranoso-[2,1-d]-Δ2'-thiazoline (**9a**) has been previously prepared using similar reaction conditions as described above [30]. All spectral characterization agreed with the literature values as did the elemental analysis of the sample used in these assays. Analytical calculated for $C_8H_{13}O_4NS$; C, 43.82; H, 5.98; N, 6.39; Experimental C, 43.45; H, 6.23; N, 6.18.

*1,2-dideoxy-2'-ethyl-α-D-glucopyranoso-[2,1-d]-Δ2'-thiazoline (**9b**)* - $^1$H NMR (500 MHz, MeOD) δ: 6.31 (1 H, d, $J_{H1,H2}$ = 7.0 Hz, H-1), 4.29-4.26 (1 H, m, $J_{H2,H3}$ = 4.32 Hz, H-2), 4.09 (1 H, dd, $J_{H3,H4}$= 4.32 Hz, H-3), 3.70 (1 H, dd, $J_{H6,H6'}$= 12.07 Hz, H-6), 3.57 (1 H, dd, H-6') 3.52 (1 H, dd, $J_{H4,H5}$ = 9.10 Hz, H-4), 3.32-3.29 (1 H, m, $J_{H5,H6}$ = 6.29 Hz, $J_{H5,H6'}$ = 2.21 Hz, H-5), 2.52 (2 H, dd, $J_{H7,H8}$ = 7.59 Hz, H-7), 1.18 (3 H, t, H-8) ppm. $^{13}$C NMR (125 MHz, MeOD) δ: 175.1, 89.4, 80.2, 75.7, 74.5, 71.4, 62.5, 28.9,11.1 ppm; Analytical calculated for $C_9H_{15}O_4NS$; C, 46.34; H, 6.48; N, 6.00; Experimental C, 45.95; H, 6.33; N, 5.93.

*1,2-dideoxy-2'propyl-α-D-glucopyranoso-[2,1-d]-Δ2'-thiazoline (**9c**)* - $^1$H NMR (500 MHz, MeOD) δ: 6.32 (1 H, d, $J_{H1,H2}$ = 7.0 Hz, H-1), 4.28 (1 H, m, $J_{H2,H3}$ = 4.4 Hz, H-2), 4.09 (1 H, dd, $J_{H3,H4}$ = 4.4 Hz, H-3), 3.72 (1 H, dd, $J_{H6,H6'}$ = 12.0 Hz, H-6), 3.59 (1 H, dd, H-6'), 3.53 (1 H, dd, $J_{H4,H5}$ = 9.1 Hz, H-4), 3.33 (1 H, m, $J_{H5,H6}$ = 6.3 Hz, $J_{H5,H6'}$ = 2.5 Hz, H-5), 2.51-2.48 (1 H, m, $J_{H7,H8}$ = 7.3 Hz, $J_{H7,H7'}$ = 14.9 Hz, H-7), 2.49-2.47 (1 H, m, H-7'), 1.68-1.65 (2 H, m, $J_{H8,H9}$ = 7.4 Hz, H-8) 0.98 (3 H, dd, H-9); Analytical calculated for $C_{10}H_{17}O_4NS$; C, 48.57; H, 6.93; N, 5.66; Experimental C, 48.32; H, 6.77; N, 5.45.

*1,2-dideoxy-2'-butyl-α-D-glucopyranoso-[2,1-d]-Δ2'-thiazoline (**9d**)* -$^1$H NMR (500 MHz, MeOD) δ: 6.31 (1 H, d, $J_{H1,H2}$ = 7.0 Hz, H-1), 4.30-4.28 (1 H, m, $J_{H2,H3}$ = 4.4 Hz, H-2), 4.09 (1 H, dd, $J_{H3,H4}$ = 4.4 Hz, H-3), 3.71 (1 H, dd, $J_{H6,H6'}$ = 12.0 Hz, H-6), 3.59 (1 H, dd, H-6'), 3.53 (1 H, dd, $J_{H4,H5}$ = 9.1 Hz, H-4), 3.35-3.30 (1 H, m, $J_{H5,H6}$ = 6.3 Hz, $J_{H5,H6'}$ = 2.5 Hz, H-5), 2.58-2.53 (1 H, m, $J_{H7,H8}$ = 7.8 Hz, $J_{H7,H7'}$ = 14.8 Hz, H-7) 2.53-2.50 (1 H, m, $J_{H7',H8}$ = 7.6 Hz, H-7') 1.60 (2 H, ddd, $J_{H8,H9}$ = 14.9 Hz, H-8) 1.37 (2 H, ddd, $J_{H9,H10}$ = 7.4, H-9) 0.92 (3 H, t, H-10) ppm. $^{13}$C NMR (125 MHz, MeOD) δ: 175.5, 89.4, 79.1, 75.2, 74.2, 71.5, 62.7, 36.9, 20.4, 15.2 ppm; Analytical calculated for $C_{11}H_{19}O_4NS$; C, 50.55; H, 7.33; N, 5.36; Experimental C, 50.68; H, 7.12; N, 5.13.

*1,2-dideoxy-2'-pentyl-α-D-glucopyranoso-[2,1-d]-Δ2'-thiazoline (**9e**)* - $^1$H NMR (500 MHz, MeOD) δ: 6.20 (1 H, d, $J_{H1,H2}$ = 6.9 Hz, H-1), 4.29-4.27 (1 H, m, $J_{H2,H3}$ = 6.0 Hz, H-2), 4.09 (1 H, dd, $J_{H3,H4}$ = 4.4 Hz, H-3), 3.72 (1 H, dd, $J_{H6,H6'}$ = 12.0 Hz, H-6), 3.59 (1 H, dd, H-6'), 3.53 (1 H, dd, $J_{H4,H5}$ = 9.1 Hz, H-4), 3.35-3.31 (1 H, m, $J_{H5,H6}$ = 6.3, $J_{H5,H6'}$ = 2.4 Hz, H-5), 2.52 (2 H, ddd, $J_{H7,H8}$ = 6.2 Hz, H-7), 1.37-1.32 (2 H, m, H-8), 1.35-1.30 (2 H, m, H-9), 1.66-1.63 (2 H, m, $J_{H10,H11}$ = 7.1 Hz, H-10), 0.90 (3 H, t, H-11) ppm. $^{13}$C NMR (125 MHz, MeOD) δ: 174.8, 89.3, 79.2, 75.5, 73.4, 71.5, 62.2, 35.4, 30.3, 28.8, 22.1, 13.1 ppm; Analytical calculated for $C_{12}H_{21}O_4NS$; C, 52.34; H, 7.69; N, 5.09; Experimental C, 52.48; H, 7.67; N, 4.40.

*1,2-dideoxy-2'-isopropyl-α-D-glucopyranoso-[2,1-d]-Δ2'-thiazoline (**9f**)* - $^1$H NMR (500 MHz, MeOD) δ: 6.31 (1 H, d, $J_{H1,H2}$ = 7.0 Hz, H-1), 4.29 (1 H, m, $J_{H2,H3}$ = 4.4 Hz, H-2), 4.11 (1 H, dd, $J_{H3,H4}$ = 4.4 Hz, H-3), 3.60 (1 H, dd, $J_{H6',H6'}$= 12.0 Hz, H-6), 3.72 (1 H, dd, H-6'), 3.55 (1 H, dd, $J_{H4,H5}$ = 9.1 Hz, H-4), 3.35-3.32 (1 H, m, $J_{H5,H6}$ = 6.4 Hz, $J_{H5,H6'}$ = 2.4 Hz, H-5), 2.87-2.83 (2 H, m, $J_{H7,H8}$ = 6.9 Hz, H-7), 1.24 (3 H, d, H.8), 1.21 (3 H, d, H-8') ppm. $^{13}$C NMR (125 MHz, MeOD) δ: 175.2, 89.5, 79.7, 75.6, 72.3, 70.8, 62.3, 35.1, 30.3, 22.5, 13.8 ppm; Analytical calculated for $C_{10}H_{17}O_4NS$; C 48.57; H 6.93; N 5.66; Experimental C 48.40; H 6.70; N 5.33.

*1,2-dideoxy-2'-isobutyl-α-D-glucopyranoso-[2,1-d]-Δ2'-thiazoline D (**9g**)* - $^1$H NMR (500 MHz, MeOD) δ: 6.38 (1 H, d, $J_{H1,H2}$ = 7.1 Hz, H-1), 4.28-4.24 (1 H, m, $J_{H2',H3}$ = 6.0 Hz, H-2), 4.06 (1 H, dd, $J_{H3,H4}$ = 6.0 Hz, H-3), 3.71 (1 H, dd, $J_{H6,H6'}$= 12.0 Hz, H-6), 3.58 (1 H, dd, H-6'), 3.58 (1 H, dd, $J_{H4,H5}$ = 9.2 Hz, H-4), 3.35-3.30 (1 H, m, $J_{H5,H6}$ = 6.3 Hz, $J_{H5,H6'}$ = 2.4 Hz, H-5), 2.46-2.40 (1 H, m, $J_{H7,H8}$ = 7.3 Hz, $J_{H7,H7'}$ = 14.1 Hz, H-7) 2.37-2.33 (1 H, m, $J_{H7',H8}$ = 7.3 Hz, H-7') 2.00 (2 H, ddd, $J_{H8,H9}$ = 6.7 Hz, H-8) 0.97 (3 H, d, H-9) ppm; Analytical calculated for $C_{11}H_{19}O_4NS$; C, 50.55; H, 7.33; N, 5.36; Experimental C, 50.68; H, 7.12; N, 5.13.

2.3 Kinetic Analyses Using Inhibitors **9a-g**

*2.3.1 Experimental procedure for kinetic analyses*

All assays were carried out in triplicate at 37 °C for 30 minutes using a stopped assay procedure in which the enzymatic reactions (25 μL) were quenched by the addition of a 6 fold excess (150 μL) of quenching buffer (200 mM

glycine, pH 10.75). Assays were initiated by the addition, *via* syringe, of enzyme (3 μL), and in all cases the final pH of the resulting quenched solution was greater than 10. Time dependent assay of β-hexosaminidase and O-GlcNAcase revealed that both enzymes were stable over this period in their respective buffers; 50 mM citrate, 100 mM NaCl, 0.1% BSA, pH 4.25 and 50 mM $NaH_2PO_4$, 100 mM NaCl, 0.1% BSA, pH 6.5. The progress of the reaction at the end of thirty minutes was determined by the measuring the extent of 4-methylumbelliferone liberated as determined by fluorescence measurements using a Varian CARY Eclipse Fluoresence-Spectrophotometer 96-well plate system and comparison to a standard curve of 4-methylumbelliferone under identical buffer conditions. Excitation and emission wavelengths of 368 and 450 nM were used, respectively, with 5 mm slit openings. The possible time dependent inactivation of O-GlcNAcase was assayed by incubating 10 mM STZ with 0.016 mg/mL O-GlcNAcase in the presence of 50 mM $NaH_2PO_4$, 100 mM NaCl, 1% BSA, 5 mM β-mercaptoethanol, pH 6.5 or 0.036 mg/mL β-hexosaminidase in the presence of 50 mM citrate, 100 mM NaCl, 0.1% BSA, pH 4.25. At several time intervals the residual enzyme activity contained in the inactivation mixture was assayed. Assays were carried out as described above for each enzyme except that reactions were initiated by the addition of an aliquot of the reaction mixture into the assay mixture containing 5.7 mM MU-GlcNAc and the appropriate buffer for each enzyme. The stability of STZ was first tested by following its decomposition in deuterated water by NMR. The half-life of STZ in aqueous solution at room temperature was well greater than 6 hours as determined by following its decomposition by NMR at room temperature. Human placental β-hexosaminidase was purchased from Sigma-Aldrich (Lot 043K3783). The cloning and expression of *O*-GlcNAcase is described in the literature [85]. Both enzymes were dialyzed against PBS buffer and their concentrations determined using the Bradford assay. The concentration (μg/μl) of β-hexosaminidase and O-GlcNAcase used in assays with fluorinated substrates were as follows: for 4-methylumbelliferyl 2-acetamido-2-deoxy-β-D-glucopyranoside (MuGlcNAc) (**5**): 0.00077, 0.0126; MuGlcNAc-F (**5a**): 0.0031, 0.0189; MuGlcNAc-$F_2$ (**5b**): 0.0154, 0.0756, and for MuGlcNAc-$F_3$ (**5c**): 0.0154, 0.01523. In addition, β-hexosaminidase and O-GlcNAcase were used at a concentration (μg/μL) of 0.0154 and 0.0378, respectively to test the inhibitors using substrate **5** at a concentration of 0.64 mM. All inhibitors were tested at eight concentrations ranging from 5 times to 1/5$^{th}$ $K_I$ with the exception of the assay of inhibitor **8e** with β-hexosaminidase, where a such high concentrations of inhibitor could not reached owing to the high $K_I$ value of **8e.** $K_I$ values were determined by linear regression of data in Dixon plots. Where necessary, assays were carried out in triplicate and error bars are included in plots of the data.

*2.3.2 Results from kinetic analyses using inhibitors **9a-g***

Analysis of the inhibition of human β-hexosaminidase reveals that increasing the chain length resulted in a marked decrease in the potency of these inhibitors (Figure 4 and Table 2). The inclusion of even one methylene unit (compound **9b**) resulted in a 460-fold increase in the value of $K_I$ for β-hexosaminidase as compared to the parent compound NAG-thiazoline (**9a**). Further increases in the chain length lead to still greater increases in $K_I$ values. For O-GlcNAcase, however, the situation is markedly different (Figure 4 and Table 2). Increases in chain length are much better tolerated and the inclusion of two methylene units yields a compound (**9c**) for which the $K_I$ value ($K_I$ = 230 nM) is only 3-fold greater than that measured for the parent compound **(9a)** NAG-thiazoline ($K_I$ = 70 nM). Branching of the aliphatic chain also does not abrogate binding since both compounds **9f** and **9g** are good inhibitors of O-GlcNAcase. From analysis of the data it can be seen that compounds **9b, 9c,** and **9f** are potent inhibitors of O-GlcNAcase and show a remarkable selectivity for O-GlcNAcase over lysosomal hexosaminidase. Indeed, the selectivity ratio for O-GlcNAcase over β-hexosaminidase is 3100-fold for compound **9d**, 1500-fold for compound **9c**, and 700-fold for compound **9f** (Figure 2 and Table 2).

*2.3.3 Results from kinetic analyses using existing inhibitors*

Existing inhibitors of O-GlcNAcase were also tested for their inhibitory properties and selectivities. The $K_I$ values for STZ (Table 2) with both O-GlcNAcase ($K_I$ = 1.5 mM) and β-hexosaminidase ($K_I$ = 47 mM) were determined and the value measured for O-GlcNAcase was found to be consistent with previous determinations of the $IC_{50}$ value that were in the range of 1 to 2.5 mM [42, 46]. The selectivity of STZ for O-GlcNAcase over β-hexosaminidase is surprisingly modest (31-fold) given the bulk of the N-acyl group of this compound. Perhaps the thiazoline compounds demonstrate greater selectivity than STZ by virtue of the fact that they may emulate a transition state or tightly bound intermediate. The possible STZ induced irreversible inactivation of O-GlcNAcase and β-hexosaminidase was also investigated. Irreversible inhibitors result in the time dependent loss of enzyme activity as the inactivator modifies the protein. To first check the stability of STZ in aqueous solution and the purity of the commercially available material, the time dependent decomposition of STZ freshly dissolved in deuterated water was monitored by NMR. STZ decomposed over time with a half-life clearly greater than 6 hours (see Figure 7). In the inventors' hands, freshly dissolved STZ did not act as a time dependent inactivator of O-GlcNAcase nor of β-hexosaminidase over a period of six hours (see Figure 8). The selectivity of PUGNAc was also investigated. The $K_I$ value for PUGNAc was measured ($K_I$ = 46 nM) with O-GlcNAcase and found

to be in close agreement with the previously determined value ($K_I$ = 50 nM)[13]. This compound, however, shows no selectivity for O-GlcNAcase as compared to β-hexosaminidase ($K_I$ = 36 nM).

These data also support the view, vide supra, that the steric bulk of the N-acyl group of the fluorine-substituted substrates does not contribute greatly to the slope of the Taft-like analysis (ρ = -0.42) measured with O-GlcNAcase. The slope (ρ= -1.0) measured with lysosomal β-hexosaminidase, however, appears likely to be a composite of both electronic and steric effects according to equation 1. The sensitivity of the reaction to the electronic effect of the fluorine substituents (ρ*) may therefore be the same for both enzymes but the significant sensitivity of the lysosomal β-hexosaminidase catalyzed reaction to the steric effect of the substrates (δ) results in an apparently steeper slope for β-hexosaminidase than that measured for O-GlcNAcase. Together, the Taft-like linear free energy analysis and the selective inhibition data suggest that the active site of O-GlcNAcase has considerably more space in the region surrounding the 2-acetamido group of the substrate than does lysosomal ß-hexosamindiase.

<u>Example 3: Design and Synthesis of a Second Embodiment of Selective O-GlcNAcase Inhibitors</u>

3.1 Design of a Second Class of Selective O-GlcNAcase Inhibitors

Based on the above observations, modifications in the pendant *N*-acyl chain ofPUGNAc were made to yield a second embodiment of potent and selective inhibitors for *O*-GlcNAcase based on a different inhibitor scaffold. Such inhibitors may have different pharmacokinetic properties and be valuable tools in dissecting the role of the *O*-GlcNAc post-translational modification at the cellular and organismal level.

3.2 Synthesis of a Second Embodiment of Selective O-GlcNAcase Inhibitors

The synthesis of a series of six inhibitors using PUGNAc as a scaffold is outlined in Scheme 3.

## Scheme 3:

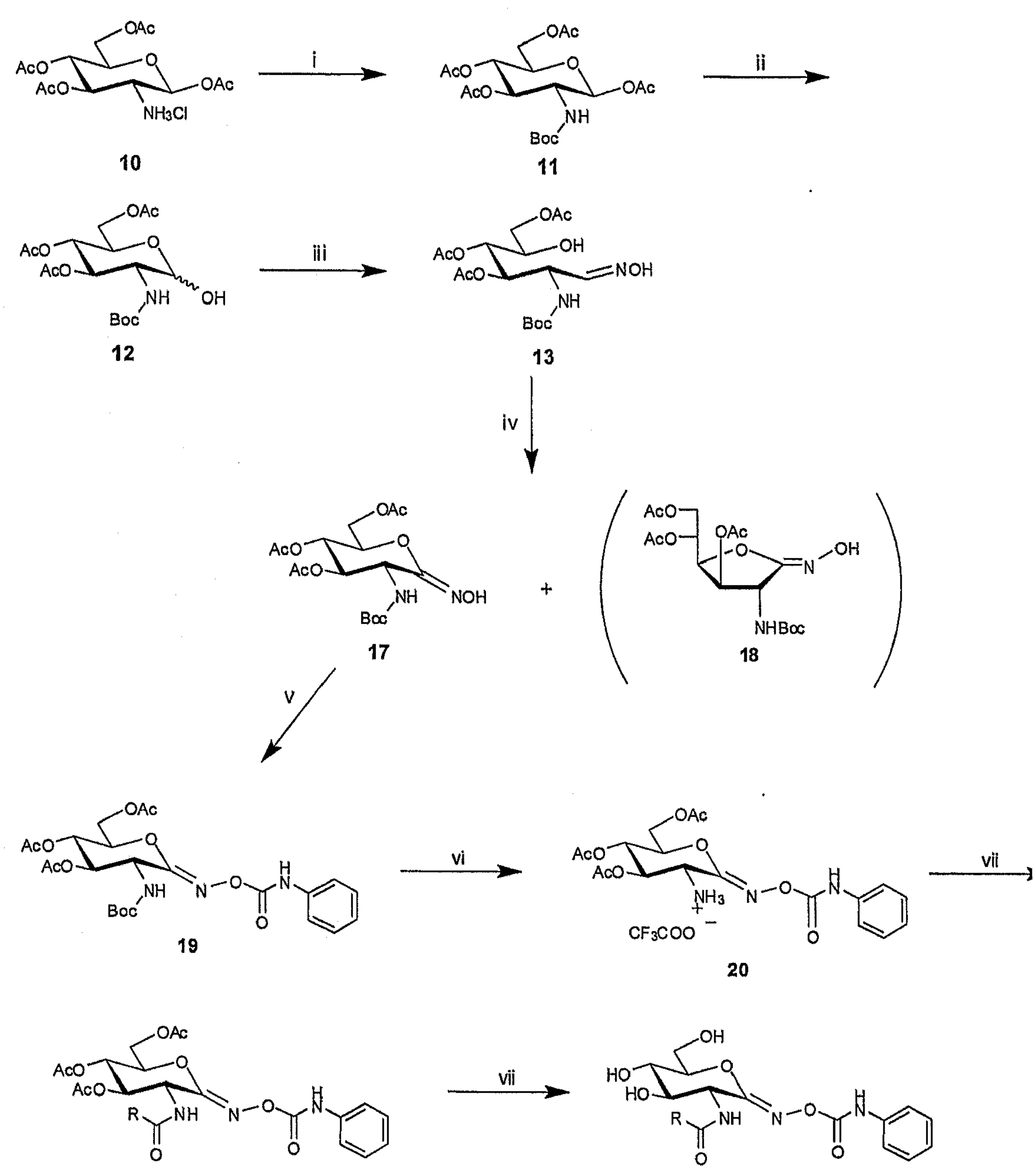


10, 11, 12, 13, 17, 18, 19, 20

21a R = $CH_3$
21b R = $CH_2CH_3$
21c R = $(CH_2)_2CH_3$
21d R = $(CH_2)_3CH_3$
21e R = $(CH_2)_4CH_3$
21f R = $CH(CH_3)_2$
21g R = $CH_2CH(CH_3)_2$

2 R = $CH_3$
22a R = $CH_2CH_3$
22b R = $(CH_2)_2CH_3$
22c R = $(CH_2)_3CH_3$
22d R = $(CH_2)_4CH_3$
22e R = $CH(CH_3)_2$
22f R = $CH_2CH(CH_3)_2$

i) $Boc_2O$, $Et_3N$, THF; ii) $(NH_4)_2CO_3$, THF, MeOH; iii) $NH_2OH.HCl$, $C_5H_5N$, MeOH; iv) DBU, NCS, $CH_2Cl_2$; v) PhNCO, $Et_3N$, THF; vi) $CF_3COOH$, $CH_2Cl_2$; vii) RC(O)Cl, $C_5H_5N$. viii) $NH_3$, MeOH

Starting from the readily accessible hydrochloride **10**[93], the Boc group was introduced to protect the amine moiety to give the tetraacetate **11**[94] (Scheme 3). With the tetraacetate **11** in hand, selective de-*O*-acetylation using

$(NH_4)_2CO_3$ afforded the hemiacetal **12** in excellent yield. Treating this hemiacetal **12** with $NH_2OH.HCl$ yields the crude *E* and *Z* oximes **13** in good yield.

The next reaction, an oxidative ring closure, has been reported by Mohan and Vasella to be quite temperamental (Scheme 4).

**Scheme 4:**

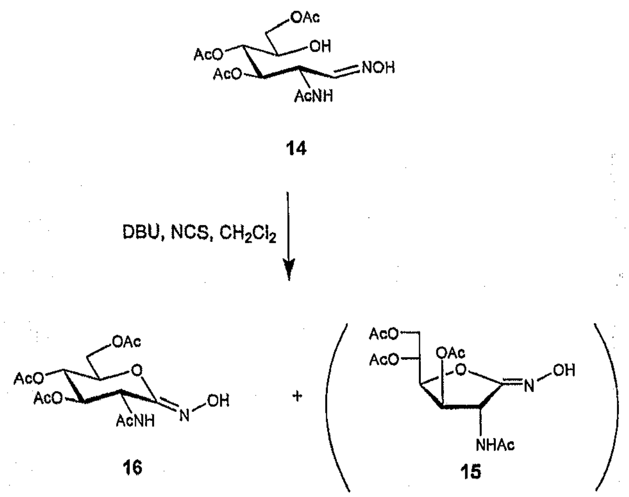


14

16 + 15

When oxidising the oxime **14** using 1,8-diazabicyclo[5.4.0]undec-7-ene (DBU) and *N*-chlorosuccinimide (NCS) careful control of the temperature of the reaction mixture during the addition of the NCS was critical to avoid formation of the undesired 1,4-lactone oxime **15**. [95] Consistent with these observations several trial reactions with **14** show that the temperature is indeed critical to successfully produce the 1,5-lactone oxime **16** over the undesired 1,4-lactone oxime **15**. Indeed, at slightly elevated temperatures (-20 ˚C) only the 1,4-lactone oxime **15** is obtained.

Referring back to Scheme 3, treating the oxime **13** with DBU/NCS under the literature conditions[95] provided a mixture of the desired 1,5-lactone **17** and the unwanted 1,4-lactone oxime **18** in a 4:1 ratio. It was found, however, that having NCS already in solution with **13** and only then adding DBU to the mixture furnished exclusively **17**, albeit in a somewhat lower yield. One complication encountered here is that succinimide, a by-product of these reactions, is difficult to separate from **17.** Furthermore, spectral analysis of **17** is complicated due to broadening of the signals obtained in the $^1H$ n.m.r. spectrum.

Nonetheless, treating impure hydroximolactone **17** with phenyl isocyanate yields pure carbamate **19** in good yield after purification by flash chromatography. Indeed, the Boc protecting group is smoothly removed using anhydrous trifluoroacetic acid in dichloromethane. Treating the resulting crude amine salt **20** with the appropriate acyl chloride yields amides **21a-g** in good overall yields. Detailed analyses of **21a** and **21b** support the identities of this entire *N*-acylated series of compounds. Subsequent de-*O*-acetylation of **21a** with saturated ammonia in methanol affords PUGNAc in good yield. **22a** was obtained from **21b** in a similar fashion. To highlight the ease of these conversions, the common intermediate, **20** can be treated with a range of acyl chlorides to provide **21c-g** as crude products. Immediate de-*O*-acetylation of these crude intermediates readily furnishes the triols **22b-f**, in good yield.

*3.2.1 General procedure for synthesis of compounds*

All solvents were dried prior to use. Synthetic reactions were monitored by TLC using Merck Kieselgel 60 $F_{254}$ aluminium-backed sheets. Compounds were detected by charring with a 10% concentrated sulfuric acid in ethanol solution and heating. Flash chromatography under a positive pressure was performed with Merck Kieselgel 60 (230-400 mesh) using specified eluants. $^1H$ and $^{13}C$ NMR spectra were recorded on a Bruker AMX400 at 400 MHz (100 MHz for $^{13}C$) or a Varian AS500 Unity Innova spectrometer at 500 MHz (125 MHz for $^{13}C$) (chemical shifts quoted relative to $CDCl_3$ or $CD_3OD$ where appropriate). Elemental analyses of all synthesized compounds used in enzyme assays were performed at the Simon Fraser University or the University of British Columbia Analytical Facility.

*3.2.2 Synthesis of (E)- and (Z)-3, 4, 6-Tri-O-acetyl-2-tert-butoxycarbonamido-2-deoxy-D-glucose Oxime (**13***

Hydroxylamine hydrochloride (3.2 g, 46 mmol) was added to the hemiacetal **12**[93] (12 g, 31 mmol) and pyridine (6.3 mL, 77 mmol) in MeOH (200 mL) and the resulting solution stirred at reflux (2 h). The solution was concentrated and co-evaporated with toluene (2 x 20 mL). The residue was taken up in EtOAc and washed with water (2 x 50 mL), brine (50 mL), dried ($MgSO_4$), filtered and concentrated to give the presumed oxime **13** (9.5 g). The residue was used without further purification.

*3.2.3 Synthesis of 3, 4, 6-Tri-O-acetyl-2-tert-butoxycarbonamido-2-deoxy-D-gluconohydroximo-1,5-lactone (**17**)*

(a) 1,8-Diazabicyclo[5.4.0]undec-7-ene (0.98 mL, 6.6 mmol) was added to the crude oxime **13** (2.5 g, 5.9 mmol) in $CH_2Cl_2$ (60 mL) at -45 ˚C and the mixture stirred (5 min). *N*-chlorosuccinimide (0.87 g, 6.5 mmol) was then added to the solution in such a way that the temperature did not go above -40 ˚C and the resulting mixture was allowed to stir for 30 minutes at this temperature and then was allowed to warm to room temperature over two hours. The mixture was quenched with water and diluted with EtOAc (100 mL). The organic layer was separated and washed with water (2 x 50 mL), brine (1 x 50 mL), dried ($MgSO_4$) filtered and concentrated. Flash chromatography of the resultant residue (EtOAc/hexanes 2:3) gave the title compound **17** as a colourless oil (1.7 g, 68%). The $^1H$ and $^{13}C$ NMR spectra appeared to show the compound of interest but was contaminated with the 1,4-lactone oxime **18** and succinimide.

(b) 1,8-Diazabicyclo[5.4.0]undec-7-ene (3.0 mL, 20 mmol) was added to the oxime **13** (7.7 g, 18 mmol) and *N*-chlorosuccinimide (2.7 g, 20 mmol) in $CH_2Cl_2$ (110 mL) at -45 ˚C in such a way that the temperature did not go above -40 ˚C and the resulting mixture was allowed to stir for 30 minutes at this temperature and then was allowed to warm to room temperature over two hours. The mixture subsequently treated as in (a) to give the title compound **17** (4 g, 52%†). $R_f$ 0.16 (EtOAc/hexane 1:1); $\delta_H$ (500 MHz, $CD_3OD$): 9.12 (*br* s, 1H, NOH), 5.33-5.18 (m, 3H, H3, H4, NH), 4.60-4.52 (m, 1H, H2), 4.33 (dd, *J*= 3.5, 12.5, 1H, H6), 4.33 (dd, *J* = 2.0, 1H, H6), 4.19 (ddd, *J*= 9.5, 1H, H5), 2.08 (s, 3H, $CH_3$), 2.03 (s, 3H, $CH_3$), 2.02 (s, 3H, $CH_3$), 1.40 (s, 9H, $C(CH_3)_3$). $\delta_C$ (125 MHz, $CD_3OD$): 171.5, 170.5, 170.3, 169.0 (4C, C=O), 150.7 (C1), 76.5, 72.4, 67.3 (3C, C3, C4, C5), 61.5 (C6), 50.8 (C2), 29.6 ($C(CH_3)_3$), 28.2 ($C(CH_3)_3$), 20.7, 20.6, 20.5 (3C, $CH_3$).

*3.2.4 Synthesis of O-(3,4,6-Tri-O-acetyl-2-tert-butoxycarbonamido-2-deoxy-D-glucopyranosylidene)amino N-Phenylcarbamate (**19**)*

Phenyl isocyanate (0.5 mL, 3.7 mmol) was added to the lactone **17** (1.3 g, 3.1 mmol) and $Et_3N$ (1.3 mL, 9.3 mmol) in THF (50 mL) and the solution stirred (r.t., 3 h). Concentration followed by flash chromatography of the resultant residue (EtOAc/hexanes 1:4) yielded the carbamate **19** as a colourless oil (1.2 g, 71%). $R_f$ 0.65 (EtOAc/hexane 1:1); $\delta_H$ (500 MHz, $CDCl_3$): 7.83 (*br* s, 1H, PhN*H*), 7.42 (m, 2H, Ar), 7.32 (m, 2H, Ar), 7.11 (m, 1H, Ar), 5.38-5.30 (m, 2H, H3, H4), 5.18 (*br* s, 1H, NH), 4.62 (m, 1H, H2), 4.45-4.40 (m, 2H, H5, H6), 4.31 (dd, *J*= 3.5, 13.5, 1H, H6), 2.12 (s, 3H, $CH_3$), 2.09 (s, 3H, $CH_3$), 2.07 (s, 3H, $CH_3$), 1.44 (s, 9H, $C(CH_3)_3$). $\delta_C$ (125 MHz, $CDCl_3$): 171.1, 170.3, 170.0, 169.1 (4C, C=O), 155.0 (CONHPh), 151.4 (C1), 13 6.8, 129.1, 124.2, 119.4 (4C, Ar), 77.2, 71.0, 67.2 (3C, C3, C4, C5), 61.2 (C6), 51.1 (C2), 30.6 ($C(CH_3)_3$), 28.2 ($C(CH_3)_3$), (20.7, 20.6, 20.5 (3C, $CH_3$); Anal. calcd for $C_{24}H_{31}N_3O_{11}$: C, 53.63; H, 5.81; N, 7.82. Found: C, 53.72; H, 5.78; N, 7.78%.

*3.2.5 General procedure for the synthesis of O-(3,4,6-Tri-O-acetyl-2-acylamido-2-deoxy-D-glucopyranosylidene)amino N-Phenylcarbamates (**21a-g**)*

Trifluoroacetic acid (13 mmol) was added to the carbamate **19** (1 mmol) in $CH_2Cl_2$ (10 mL) at 0 ˚C and the solution stirred (2 h). Pyridine (200 mmol) was then slowly added to the solution and the resulting mixture left to stand (0 ˚C, 10 min). The appropriate acyl chloride (3 mmol) was then added at 0 ˚C and the solution allowed to stand at 4 ˚C overnight. Concentration of the mixture gave a yellowish residue which was dissolved in EtOAc (30 mL) and washed with (2 x 20 mL), brine (1 x 20 mL), dried ($MgSO_4$) filtered and concentrated. For the presumed intermediate tri-*O*-acetates **21c-g** these were carried through without further purification. Flash chromatography of the residues presumably **21a** and **21b** (EtOAc/hexanes 1:1) gave the desired acyl derivatives **21a** and **21b** in yields of 48% and 42% respectively.

***O*-(2- acetamido- 3,4,6- tri- O- acetyl- 2- deoxy- D- glucopyranosylidene) amino *N*-Phenylcarbamate (21a)**-Gave $^1H$ and $^{13}C$ NMR spectra consistent with that found in the literature. [96] (48%) $R_f$ 0.2 (EtOAc/hexane 7:3).

**O-(3,4,6-Tri-*O*-acetyl-2-deoxy-2-propamido-$_D$-glucopyranosylidene)amino *N*-Phenylcarbamate (21b)**-(42%) $R_f$ 0.11 (EtOAc/hexane 1:1); $\delta_H$ (500 MHz, $CDCl_3$): 7.71 (*br* s, 1H, PhN*H*), 7.41 (m, 2H, Ar), 7.32 (m, 2H, Ar), 7.11 (m, 1H, Ar), 6.59 (d, *J*= 7.5, 1H, NH), 5.40 (dd, *J*= 9.0, 1H, H3), 5.35 (dd, *J*= 9.0, 1H, H4), 4.89 (dd, 1H, H2), 4.53 (ddd, *J*= 3.0, 4.0, 1H, H5), 4.43 (dd, *J*= 13.0, 1H, H6), 4.32 (dd, 1H, H6), 2.22 (q, *J*= 7.5, $CH_2$), 2.13 (s, 3H, $COCH_3$), 2.07 (s, 3H, $COCH_3$), 2.05 (s, 3H, $COCH_3$), 1.14 (t, 3H, $CH_3$). $\delta_C$ (125 MHz, $CDCl_3$): 174.2, 170.4, 170.2, 169.1 (4C,

C=O), 155.1 (CONHPh), 151.7 (C1), 13 6.8, 129.2, 124.3, 119.2 (4C, Ar), 77.2, 71.2, 67.1 (3C, C3, C4, C5), 61.2 (C6), 49.6 (C2), 29.4 ($CH_2$), 20.7, 20.6, 20.5 (3C, $COCH_3$), 9.7 ($CH_3$).

*3.2.6 General procedure for the synthesis of O-(2-acylamido-2-deoxy-$_D$-glucopyranosylidene)amino N-Phenylcarbamates (**PUGNAc, 22**α**-f**)*

A saturated solution of ammonia in MeOH (2 mL) was added to the carbamate (0.3 mmol) in MeOH (10 mL) and the solution left to stand (r.t., 2 h). Concentration followed by flash chromatography of the residue (MeOH/EtOAc 3:97) gave the desired triols **PUGNAc, 22a-f** in yields ranging from 21% to 32%.

**O-(2-Acetamido-2-deoxy-$_D$-glucopyranosylidene) amino *N*-Phenylcarbamate (PUGNAc)**-(32%) Gave $^1H$ and $^{13}C$ NMR spectra consistent with that found in the literature. [96] $R_f$ 0.15 (MeOH/EtOAc 1:19)

***O*-(2-deoxy-2-propamido-$_D$-glucopyranosylidene) amino *N*-Phenylcarbamate (22a)**-(32%) $R_f$ 0.11 (MeOH/EtOAc 1:19); $\delta_H$ (500 MHz, $CD_3OD$): 7.41 (m, 2H, Ar), 7.26 (m, 2H, Ar), 7.03 (m, 1H, Ar), 4.54 (m, 1H, H2), 3.96-3.93 (m, 2H, H5, H6), 3.83 (dd, *J*= 4.0, 12.5, 1H, H6), 3.75-3.71 (m, 2H, H3, H4), 2.31 (q, *J*= 7.5, $CH_2$), 1.16 (t, 3H, $CH_3$); $\delta_C$ (125 MHz, $CD_3OD$): 177.5 (C=O), 159.5 (CONHPh), 154.7 (C1), 139.3, 129.9, 124.8, 120.3 (4C, Ar), 84.1, 74.4,69.9 (3C, C3, C4, C5), 61.8 (C6), 52.9 (C2), 30.2 ($CH_2$), 10.2 ($CH_3$); Anal. calcd for $C_{16}H_{21}N_3O_7.H_2O$: C, 50.00; H, 5.77; N, 10.93. Found: C, 50.02; H, 5.78; N, 10.76%.

*O*-**(2-deoxy-2-butamido-$_D$-glucopyranosylidene)amino** *N*-**Phenylcarbamate (22b)**-(26%) $R_f$ 0.26 (MeOH/EtOAc 1:19); $\delta_H$ (500 MHz, $CD_3OD$): 7.41 (m, 2H, Ar), 7.27 (m, 2H, Ar), 7.03 (m, 1H, Ar), 4.54 (m, 1H, H2), 3.94-3.92 (m, 2H, H5, H6), 3.83 (dd, *J*= 4.5, 13.0, 1H, H6), 3.74-3.70 (m, 2H, H3, H4), 2.26 (t, *J*= 6.0, $COCH_2$), 1.66 (m, 2H, $CH_2CH_3$), 0.96 (t, *J*= 7.0, 3H, $CH_3$); $\delta_C$ (100 MHz, $CD_3OD$): 176.7 (C=O), 159.5 (CONHPh), 154.7 (C1), 139.3, 130.0, 124.8, 120.3 (4C, Ar), 84.1, 74.5, 69.9 (3C, C3, C4, C5),61.8 (C6), 52.9 (C2), 39.1 ($CH_2$), 20.3 ($CH_2$), 14.1 ($CH_3$); Anal. calcd for $C_{17}H_{23}N_3O_7$: C, 53.54; H, 6.08; N, 11.02. Found: C, 53.52; H, 6.09; N, 10.90%.

***O*-(2-deoxy-2-valeramido-n-glucopyranosylidene)amino *N*-Phenylcarbamate (22c)-**(23%) $R_f$ 0.43 (MeOH/EtOAc 1:19); $\delta_H$ (400 MHz, $CD_3OD$): 7.45 (m, 2H, Ar), 7.30 (m, 2H, Ar), 7.03 (m, 1H, Ar), 4.58 (m, 1H, H2), 3.99-3.94 (m, 2H, H5, H6), 3.87 (dd, *J*= 4.0, 12.4, 1H, H6), 3.79-3.75 (m, 2H, H3, H4), 2.33 (t, *J*= 7.2, $COCH_2$), 1.64 (m, 2H, $CH_2CH_2$), 1.40 (m, 2H, $CH_2CH_2$), 0.92 (t, *J*= 7.2, 3H, $CH_3$); $\delta_C$ (100 MHz, $CD_3OD$): 176.8 (C=O), 159.5 (CONHPh), 154.7 (C1), 139.3, 130.0, 124.8, 120.4 (4C, Ar), 84.1, 74.3, 69.9 (3C, C3, C4, C5), 61.8 (C6), 52.9 (C2), 37.0 ($CH_2$), 29.0 ($CH_2$), 23.5 ($CH_2$), 14.2 ($CH_3$); Anal. calcd for $C_{18}H_{25}N_3O_7.2H_2O$: C, 50.11; H, 6.34; N, 9.79. Found: C, 50.25; H, 6.04; N, 9.94%.

***O*-(2-deoxy-2-hexamido-$_D$-glucopyranosylidene)amino *N*-Phenylcarbamate 22d-**(26%) $R_f$ 0.48 (MeOH/EtOAc 1:19); $\delta_H$ (500 MHz, $CD_3OD$): 7.43 (m, 2H, Ar), 7.28 (m, 2H, Ar), 7.05 (m, 1H, Ar), 4.55 (m, 1H, H2), 3.97-3.92 (m, 2H, H5, H6), 3.85 (dd, *J*= 4.0, 12.5, 1H, H6), 3.77-3.72 (m, 2H, H3, H4), 2.30 (t, *J*= 7.5, $COCH_2$), 1.66 (m, 2H, $COCH_2CH_2$), 1.36-1.27 (m, 4H, $CH_2CH_2$), 0.87 (t, *J*= 6.5, 3H, $CH_3$); $\delta_C$ (125 MHz, $CD_3OD$): 176.8 (C=O), 159.5 (CONHPh), 154.7 (C1), 139.3, 130.0, 124.8, 120.4 (4C, Ar), 84.1, 74.3, 69.9 (3C, C3, C4, C5), 61.8 (C6), 52.9 (C2), 37.2 ($CH_2$), 32.6 ($CH_2$), 26.6 ($CH_2$), 23.5 ($CH_2$), 14.3 ($CH_3$); Anal. calcd for $C_{19}H_{27}N_3O_7$: C, 55.74; H, 6.65; N, 10.26. Found: C, 55.55; H, 6.86; N, 9.99%.

***O*-(2-deoxy-2-isobutamido-$_D$-glucopyranosylidene) amino *N*-Phenylcarbamate (22e)**-(29%) $R_f$ 0.22 (MeOH/EtOAc 1:19); $\delta_H$ (500 MHz, $CD_3OD$): 7.42 (m, 2H, Ar), 7.27 (m, 2H, Ar), 7.03 (m, 1H, Ar), 4.52 (m, 1H, H2), 3.96-3.92 (m, 2H, H5, H6), 3.83 (dd, *J*= 4.5, 12.5, 1H, H6), 3.77-3.71 (m, 2H, H3, H4), 2.54 (m, *J*= 7.0, CH), 1.16 (d, 3H, $CH_3$), 1.14 (d, 3H, $CH_3$); $\delta_C$ (125 MHz, $CD_3OD$): 180.6 (C=O), 159.4 (CONHPh), 154.7 (C1), 139.3, 130.1, 124.8, 120.4 (4C, Ar), 84.1, 74.3, 69.9 (3C, C3, C4, C5), 61.8 (C6), 52.8 (C2), 36.4 (CH), 19.9, 19.8 (2C, $CH_3$); Anal. calcd for $C_{17}H_{23}N_3O_7$: C, 53.54; H, 6.08; N, 11.02. Found: C, 53.17; H, 6.18; N, 10.87%.

***O*-(2-deoxy-2-isovaleramido-$_D$-glucopyranosylidene)amino *N*-Phenylcarbamate (22f)-** (23%) $R_f$ 0.47 (MeOH/EtOAc 1:19); $\delta_H$ (400 MHz, $CD_3OD$): 7.44 (m, 2H, Ar), 7.30 (m, 2H, Ar), 7.06 (m, 1H, Ar), 4.59 (m, 1H, H2), 3.98-3.95 (m, 2H, H5, H6), 3.86 (dd, *J*= 4.4, 12.8, 1H, H6), 3.77-3.74 (m, 2H, H3, H4), 2.17 (d, *J*= 7.5, $COCH_2$), 2.12, (m, 1H, $CH(CH_3)_2$), 0.98 (m, 6H, $CH_3$); $\delta_C$ (100 MHz, $CD_3OD$): 176.0 (C=O), 159.5 (CONHPh), 154.7 (C1), 139.3, 130.0, 124.8, 120.4 (4C, Ar), 84.1, 74.5, 69.9 (3C, C3, C4, C5), 61.8 (C6), 52.9 (C2), 46.5 ($CH_2$), 27.5 ($CH(CH_3)_2$), 23.0, 22.9 (2C, $CH_3$); Anal. calcd for $C_{18}H_{25}N_3O_7$: C, 54.68; H 6.37; N, 10.63. Found: C, 54.33; H, 6.48; N, 10.56%.

3.3 Kinetic Analysis Using Inhibitors **22a-f**

*3.3.1 Experimental procedure for kinetic analyses*

All assays were carried out in triplicate at 37 ˚C for 30 minutes by using a stopped assay procedure in which the enzymatic reactions (50 μL) were quenched by the addition of a 4-fold excess (200 μL) of quenching buffer (200 mM glycine, pH 10.75). Assays were initiated by the careful addition, *via* pipette, of enzyme (5 μL), and in all cases the final pH of the resulting quenched solution was greater than 10. Time-dependent assay of *O*-GlcNAcase and β-hexosaminidase revealed that enzymes were stable in their respective buffers over the period of the assay: 50 mM $NaH_2PO_4$, 100 mM NaCl, 0.1% BSA, pH 6.5 and 50 mM citrate, 100 mM NaCl, 0.1% BSA, pH 4.25. The progress of the reaction at the end of 30 minutes was determined by measuring the extent of 4-nitrophenol liberated as determined by UV measurements at 400 nm using a 96-well plate (Sarstedt) and 96-well plate reader (Molecular Devices). Human placental β-hexosaminidase was purchased from Sigma (lot043K3783), and *O*-GlcNAcase was overexpressed and purified freshly, prior to use. [85] *O*-GlcNAcase and β-hexosaminidase were used in the inhibition assays at a concentration (μg/μL) of 0.0406 and 0.012, respectively using substrate pNP-GlcNAc at a concentration of 0.5 mM. All inhibitors were tested at seven concentrations ranging from 3 times to 1/3 $K_I$, with the exception of the assay of inhibitor **22d** with β-hexosaminidase, where such high concentrations of inhibitor could not be reached because of its high $K_I$ value. $K_I$ values were determined by linear regression of data from Dixon plots.

*3.3.2 Results from kinetic analyses using inhibitors* **22a-f**

As stated above, PUGNAc is a potent competitive inhibitor of both *O*-GlcNAcase[13, 50] and β-hexosaminidase[28, 51]. For the human enzymes the respective $K_I$ values are 46 nM and 36 nM. [13, 49] The selectivity of inhibitors **22a-f** were evaluated and compared to PUGNAc. Using *p*NP-GlcNAc as a substrate it was found that these compounds **22a-f** are inhibitors of both human *O*-GlcNAcase and human β-hexosaminidase (Table 3).

**Table 3.** Inhibition constants and selectivities of inhibitors for *O*-GlcNAcase and β-hexosaminidase.

| Compound | *O*-GlcNAcase $K_I$ (μM) | β-Hexosaminidase $K_I$ (μM) | Selectivity ratio (β-Hexosaminidase $K_I$/ *O*-GlcNAcase) |
|---|---|---|---|
| **PUGNAc** | 0.046 | 0.036 | 1 |
| **22a** | 1.2 | 1.2 | 1 |
| **22b** | 2.4 | 26 | 11 |
| **22c** | 40 | 220 | 6 |
| **22d** | 210 | >900 | ≥5 |
| **22e** | 9 | 20 | 2 |
| **22f** | 190 | >1200 | ≥6 |

Analysis of the inhibition of human β-hexosaminidase reveals that increasing the chain length of the *N*-acyl group results in a marked decrease in the potency of these inhibitors (Table 3). The inclusion of only one methylene group (compound **22a**) results in a 33-fold increase in the $K_I$ value for β-hexosaminidase as compared to the parent PUGNAc. Additional increases in chain length lead to still greater increases in $K_I$ values. Furthermore, *O*-GlcNAcase tolerates increases in chain length better than β-hexosaminidase (Table 3).

For these two human enzymes, *O*-GlcNAcase and β-hexosaminidase, direct comparison of the $K_I$ values of the previously prepared NAG-thiazoline derivatives, **9b-g**, [49] reveals that the modified thiazolines are more selective and potent inhibitors than the corresponding PUGNAc-based compounds, **22a-f**. These observations point to the importance of the position of the acyl group of the inhibitor and potentially, the hybridisation of the anomeric carbon. The thiazolines, **NAG-thiazoline** and **9b-g**, with a $sp^3$ hybridised anomeric carbon, comprise a bicyclic scaffold that restricts movement of the acyl chain as compared to the acyl chain on the corresponding PUGNAc derivatives, PUGNAc and **22a-f**, that have a $sp^2$ hybridised anomeric carbon. As well, the precise positioning of the side chain within the active sites must vary between these two sets of compounds. Together, these two factors must contribute to both the overall somewhat poorer inhibition and lesser selectivity of the PUGNAc-derived compounds compared to the thiazoline derivatives. Consistent with these observations is that streptozotocin (STZ), a poor inhibitor of O-GlcNAcase ($K_I$ = 1.5 mM), [46, 47, 49] also has a bulky, freely rotating acyl chain and shows moderate selectivity for O-GlcNAcase over β-hexosaminidase. [46, 47, 49]

These differences between the tiazoline-based compounds, **NAG-thiazoline** and **9b-g**, and the PUGNAc derivatives, PUGNAc and **22a-f**, may stem from the former compounds emulating the presumed bicyclic-like transition state involved in the catalytic mechanism of *O*-GlcNAcase[49] and β-hexosaminidase[30, 48, 83, 97]. In contrast, the position of the *N*-acyl group of the PUGNAc analogues and STZ may resemble that of the natural substrate *N*-acetyl-$_D$-glucopyranoside. The different hybridisation of the anomeric carbons of the PUGNAc and thiazoline derivatives may also contribute to the precise positioning of these *N*-acyl groups.

All of the above prepared compounds were inhibitors of both human *O*-GlcNAcase and human β-hexosaminidase. These compounds, however, exploit differences in active site architectures between these two enzymes, which

results in them being selective for *O*-GlcNAcase. Despite the lower selectivity of the PUGNAc analogues as compared to the thiazoline derivatives, these compounds may have different pharmacokinetic properties owing to their different scaffold. Accordingly, both the thiazoline and PUGNAc derivatives may prove to be valuable tools for dissecting the role of the *O*-GlcNAc post-translational modification at the cellular and organismal level. Indeed, elaboration of other glycosidase inhibitors has yielded clinically useful compounds targeting entirely different enzymes. [98] As well, using the strategy outlined here, systematic elaboration of other β-*N*-acetyl-glucosaminidase inhibitor scaffolds[99, 100] may also yield selective inhibitors for human *O*-GlcNAcase.

Example 4: Evaluation of selective inhibitors in cell culture

Having demonstrated the selectivity of these compounds *in vitro* the use of the compounds in living cells was evaluated.

4.1 Experimental Procedures

*4.1.1 Cell Culture and Inhibition*

COS-7 cells were cultured in DMEM medium (Invitrogen) supplemented with 5-10% FBS (Invitrogen). Aliquots of inhibitors (50 μL of a stock in 95% ethanol) were delivered onto tissue culture plates and the ethanol was evaporated. The cells were incubated at 37˚C for 40 hours at which time they reached approximately 80 % confluence. The time dependent accumulation of *O*-GlcNAc-modified proteins in response to treatment with 50 μM of compound **9a, 9c** or **9g** in cells was studied as follows. COS-7 cells were cultured to 25 % confluence in 5% FBS and an aliquot (100 μL) of inhibitor dissolved in media and filter sterilized was added to each plate to yield a final concentration of 50 μM of inhibitor. COS-7 cells (2 x 10 cm plates) were harvested at the appropriate times by scraping and were pooled by centrifugation (200 × g, 10 min). Cells were washed once with PBS, pH 7.0 (10 mL) and pelleted (200 × g, 10 min). The cells could be frozen at -80˚C at this point. Control cultures without inhibitors were treated in the same manner.

*4. 1. 2 Western Blot Analyses*

COS-7 cells were cultured in the presence of inhibitors **9a, 9c,** or **9g** as described above to approximately 90 % of confluence. A culture of control cells was treated in the same manner as follows but the cultures contained no inhibitor. Cells were harvested as described above. Frozen cells were thawed at 4˚C, and cold lysis buffer (1 mL of 50 mM Tris, pH 8.0 containing 150 mM NaCl, 1 mM EDTA, 1 mM PMSF, 1% NP-40, 0.5% sodium deoxycholate, and 1 mM of inhibitor **9f**) was added. After 10 minutes at 4 ˚C the solution was centrifuged at 14,000 rpm in an Eppendorf 5415C microcentrifuge and the supernatant was collected. SDS/PAGE loading buffer was added to an aliquot (15 μL) of each sample, and after heating at 96 ˚C aliquots were loaded onto 10% or 12% Tris·HCl polyacrylamide gels. After electrophoresis, the samples were electroblotted to nitrocellulose membrane (0.45 μm, Bio-Rad). Transfer was verified by visual inspection of the transfer of prestained markers (Dual Colour Precision Plus Protein Standard - Biorad). The membrane was blocked by using 5% BSA (fraction V, Sigma) in PBS (blocking buffer A for samples probed with mouse anti-*O*-GlcNAc monoclonal IgM antibody (MAb CTD 110.6 - Covance)) or 5% low-fat dry powdered milk (blocking buffer B for samples probed with anti-β-actin), pH 7.4, containing 0.1% Tween 20 for 1 h at room temperature or overnight at 4˚C. The blocking solution was decanted, and a solution of blocking buffer A containing MAb CTD 110.6 (1:2500 of the stock) or blocking buffer B containing mouse monoclonal anti-β-actin IgG (Clone AC-40 - Sigma) was added (1:1000 dilution) as appropriate. The membrane was incubated at room temperature for 1 h or overnight at 4˚C after which the blocking buffer was decanted and the membrane was rinsed with PBS, pH 7.4, containing 0.1 % Tween 20 (wash buffer). Membranes were then rinsed for 2 × 5 min and 3 × 15 min with wash buffer. For immunological detection of *O*-GlcNAc, the membrane was incubated in blocking buffer A for 1 hour at RT and, after washing, the membrane was incubated with a secondary goat anti-mouse-IgM-HRP-conjugate (1:2500, Santa Cruz Biotech) for one hour at RT or 4 ˚C overnight in blocking solution. For detection of β-actin levels, the membrane was incubated with a secondary goat anti-mouse-IgG-HRP conjugate (1:100000, Sigma) for one hour at RT or 4 ˚C overnight in blocking solution B. Membranes were washed and detection of membrane bound goat anti-mouse-IgG-HRP conjugate was accomplished by chemiluminescent detection using the SuperSignal West Pico Chemiluminescent Detection Kit (Pierce) and film (Kodak Biomax MR).

4.2 Results from Cell Studies

COS-7 cells incubated in plates with 50 μM of inhibitor **9a, 9c,** or **9g** revealed no abnormalities in proliferation rate or morphology as compared to control cells (data not shown). Cellular levels of O-GlcNAc-modified proteins within cells cultured for 40 hours in the presence of inhibitors **9a, 9c** or **9g**, or in their absence was carried out using the O-GlcNAc directed monoclonal antibody (64) mAbCTD110.6. Marked increases in cellular levels of O-GlcNAc-modified proteins within the cells were observed as compared to the control (Figure 5A) indicating that these compounds readily gain access to the interior of the cell where they act to block O-GlcNAcase function. The speed with which the thiazoline inhibitor (**9c**) acts to block O-GlcNAcase action within COS-7 cells was also probed. By monitoring the level of O-GlcNAc-modified proteins using Western blot analysis, a clear time dependent increase in levels of O-GlcNAc-modified proteins was found. Even after one hour of exposure to inhibitor (**9c**) increases in levels of O-GlcNAc-modified proteins were observed. The inhibitor appears to quickly enter into the cells where it immediately blocks O-GlcNAcase activity resulting in a time-dependent accumulation of O-GlcNAc-modified proteins (Figure 6). After an initial rapid rise, the level of O-GlcNAc-modified proteins in cells treated with inhibitor (**9c**) appears to asymptotically approach a steady state within the cell (Figure 6). This behaviour is consistent with the similar time dependent increase previously observed for HT29 cells incubated with PUGNAc [50]. Western blot analysis of blots probed with α-β-actin (Figure 5B) followed by the appropriate secondary HRP-conjugate revealed that in all cases the sample loading was equivalent. Also worth noting is that the parent compound NAG-Thiazoline (**9a**) has previously been demonstrated to enter into cells where it exerts an effect on lysosomal β-hexosaminidase [101].

Example 5: Use of Selective O-GlcNAcase Inhibitors to Develop an Animal Model for Studying Type II Diabetes

5.1 Animal Studies Showing Effect of But-NAG-thiazoline (***9c***) on Various Tissue Types and Glucose Clearance

*5.1.1 Experimental Procedure*

Eight give-week old healthy Sprague-Dawley rats (Charles River) were caged in pairs. The animals were allowed to acclimatize to their new surrounding at the Simon Fraser University Animal Care Facilities (SFU ACF) for one week. At 3:00 pm, four rats were given a tail vein injection of 400 μl of inhibitor **9c** dissolved in PBS buffer (pH 7.4) at a dose of 50 mg $kg^{-1}$. The remaining four rats were given a tail vein injection of 400 μl of PBS as a control. All solutions were pre-sterilized through a 0.2 μm filter (Millipore) and injected with 0.5 mL syringes containing a 28 gauge x 0.5" needle (Terumo) as bolus over 10 seconds. Food was withdrawn at approximately 11:00 pm. At 8:30 am the following morning, the rats were given a second injection. After three hours the rats were anesthetized with isoflurane and a 100 μL blood sample was taken from the jugular vein to measure the fasting blood glucose levels. An intravenous glucose tolerance test (IVGTT) was then performed. A 0.5 mL injection of a 50% w/v solution of glucose dissolved in PBS (pre-sterilized) was administered via the tail vein over 30 seconds. At 10, 20, 30, 40, 60, and 90 minutes after the injection of glucose, a 100 μL sample of blood was taken from the jugular vein. Immediately following blood sampling, a small aliquot of the blood was used to measure the blood glucose concentration using a glucometer (Accu-Check Advantage, Roche) and the remaining blood was stored on ice for 20 minutes and spun down to isolate the serum. Following completion of the IVGTT, the rats were sacrificed with 1:1 mixture of $CO_2/O_2$. Tissue samples (brain, muscle, liver, spleen, pancreas, fat) were immediately harvested and flash frozen with liquid nitrogen and stored at -80°C. To homogenize the tissues to obtain a cell extract, tissue were kept frozen while they were ground with a mortar and pestle into a fine powder. 100 mg of the powder was then homogenized in 1 mL of cold lysis buffer (50 mM Tris, 0.5 % sodium deoxycholate, 0.1 % SDS, 1% nonidet P-40, 1 mM EDTA, 1 mM PMSF, and 1 mM butyl-NAG-thiazoline) using two 10 second pulse on a Jenke and Kunkel Ultra-Turrax tissue homogenizer tissue homogenize. Tissues were then spun down at 13,000 rpm in a microcentrifuge (eppendrof) to remove the cell debris. The soluble cell extract was then analyzed for levels O-GlcNAc modified proteins using Western blot analysis as described above for the cell studies.

*5.1.2 Effect of But-NAG-thiazoline (9c) on Various Tissue Types*

Rats injected with varying doses of inhibitor **9c** via the tail vein showed that intravenous administration of inhibitor **9c** affects cellular levels of O-GlcNAc modified proteins in various tissue types (Figure 9). Samples of brain tissue (Figure 9A) showed that there were marked increases in cellular levels of O-GlcNAc modified proteins when rats are injected with 50, 120 and 300 mg/kg doses of inhibitor 9c as compared to controls. This indicates that inhibitor 9c readily gains access to the interior of brain cells where it acts to block O-GlcNAcase function. Similar results were observed for tissue samples of muscle and liver (Figures 9B and 9C).

From the results, it appears that the inhibitor is only inhibiting O-GlcNAcase in a semi dose-dependent manner. This shows that 50 mg/kg of inhibitor 9c is enough to elicit the maximal increase in O-GlcNAc modified proteins. As well, these results show that the inhibitor is able to gain access to a wide variety of tissues in less than 24 hours and retains an effect for a minimum of 6 hours. It should also be noted that rats used in this study that were treated with the inhibitor did not appear to show any discomforts compared to the control rat.

*5.1.3 Effect of But-NAG-thiazoline on Glucose Clearance*

Having shown that the inhibitor **9c** is capable of inhibiting *O*-GlcNAcase in rats, attention was turned to addressing whether or not increased levels of *O*-GlcNAc modified proteins alters the ability of the rats to maintain glucose homeostasis. An intravenous glucose tolerance test (IVGTT) was used as a means of measuring the ability of the animal to respond to a challenge of 1 g $kg^{-1}$ of glucose. The results clearly indicate that short term exposure of the inhibitor (< 24 hours) does not affect the glucose clearance rate in rats (Figure 10A). It should be noted that these results are statistically significant, as they were done with four rats treated with the inhibitor as well as four rats treated with a control injection of PBS buffer. Western blot analyses indicates that the four rats treated with the inhibitor in the IVGTT did have increased levels of O-GlcNAc modified proteins (Figure 10B).

This result is surprising, given that it has been shown previously that elevated levels of *O*-GlcNAc modified proteins in cultured adipocytes cells results in insulin resistance. This result is surprising, given that it has been shown previously that elevated levels of *O*-GlcNAc modified proteins in cultured adipocytes cells results in insulin resistance. The inventors hypothesize that the differences between these results stem from one of two possibilities. First, the results obtained from a whole organism is more physiologically relevant than experiments conducted using cultured cells, and therefore insulin resistance does not develop in peripheral tissues. The second possibility is that the inhibitor 9c actually affects the pancreatic β-cells in a way that causes them to secrete more insulin. This effect would overcome insulin insensitivity in the peripheral tissues, possibly resulting in normal glucose clearance as was observed. Support for this second hypothesis comes from the fact that a number of proteins involved in producing and transporting insulin within the β-cells are themselves modified by *O*-GlcNAc, such as two of the transcription factors (pdx-1 and sp1) that stimulate insulin production.

5.2 Animal Studies Showing Clearance Rate of But-NAG-thiazoline (**9c**)

*5.2.1 Experimental Procedure*

To obtain information for how rapid the inhibitor is cleared from tissues, five 1 0-week old Sprague-Dawley rats were given a tail vein injection of the inhibitor (50 mg $kg^{-1}$), The animals were sacrificed sequentially 3, 7, 24, 27, and 32 hours following the injection. Two additional rats were used as controls. One rat was sacrificed immediately before the others received their injections in order to obtain information about the normal levels of *O*-GlcNAc modified proteins. Also, one rat was injected with sterilized PBS (pH 7.4) along with the other rats and sacrificed at the end of the experiment (32 hours). Tissue collection, homogenization, and western blot analyses were done in the same matter as described above.

*5.2.2 Clearance rate results*

Rats injected with inhibitor **9c** showed that intravenous administration of inhibitor **9c** affects cellular levels of O-GlcNAc modified proteins in various tissue types as early as three hours following injection (Figure 11). Samples of brain tissue (Figure 11A) showed that there were marked increases in cellular levels of-GlcNAc modified proteins after three hours following injection as compared to controls. This indicates that inhibitor **9c** readily gains access to the interior of brain cells where it acts to block O-GlcNAcase function. Cellular levels of O-GlcNAc modified proteins remained high after seven hours. The levels dropped after 24 hours and appeared to return to normal levels after about 32 hours. Similar results were observed for muscle tissue samples (Figure 11B).

5.3 Animal Studies Showing Oral Availability of But-NAG-thiazoline (**9c**)

*5.3.1 Experimental Procedure*

To determine if the inhibitor 9c is orally available, it was incorporated into the chow (Lab Diet 5001 Rodent Diet, PMI Nutrition International, LLC). To make the rat chow, 600 g of the ground chow was mixed with 355 ml of water and 5 mL of the inhibitor dissolved in ethanol. Small pieces were then prepared with a pasta machine (Pasta Express, Creative) and dehydrated overnight at 37 ˚C (Snackmaster Dehydrator, American Harvest). Five sets of chow were made with the following amounts of inhibitor: 0 mg $kg^{-1}$ $day^{-1}$, 100 or 1000 mg $kg^{-1}$ $day^{-1}$ of the deprotected (polar) inhibitor, and 100 or 1000 mg $kg^{-1}$ $day^{-1}$ of the protected (non-polar) inhibitor. These numbers are based on data obtained from previous studies that show that a 6-week old rat eats approximately 25 g food per day. Ten five-week old healthy Sprague-Dawley rats (two per set of food) were then allowed to feed on the rat chow for three days. The animals were then sacrificed and tissues were collected, homogenized, and analyzed as described above.

*5.3.2 Oral availability results*

Rats were fed two different doses of protected (non-polar) and deprotected (polar) forms of inhibitor **9c** for three days. Western blot analyses showed that oral administration of both forms of inhibitor **9c** affects cellular levels of O-GlcNAc modified proteins in various tissue types (Figure 12). Samples of brain tissue (Figure 12A) showed that there were marked increases in cellular levels of O-GlcNAc modified proteins when rats are fed 100 mg/kg/day doses of either the protected or deprotected forms of inhibitor **9c.** Cellular levels of O-GlcNAc modified proteins within brain tissue was even greater when rats were fed the larger dose of 1000 mg/kg/day doses of either the protected or deprotected forms of inhibitor **9c.** This indicates that inhibitor **9c** readily gains access to the interior of brain cells where it acts to block O-GlcNAcase function even when it is orally administered in a dose dependant manner. Similar results were observed for other types of tissue samples, including muscle, pancreas, fat and spleen (Figure 12B-12E).

Disclosed is a compound having the general chemical formula (I) and pharmaceutically acceptable salts thereof:

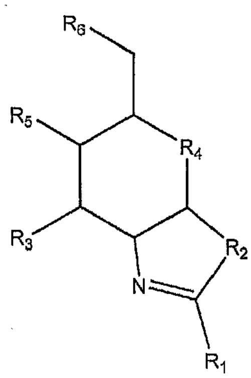

(I)

wherein $R_3$, $R_5$, $R_6$, are selected from the group consisting of branched alkyl chains, unbranched alkyl chains, cycloalkyl groups, aromatic groups, alcohols, ethers, amines, substituted or unsubstituted carbamates, substituted or unsubstituted ureas, esters, amides, aldehydes, carboxylic acids, and heteroatom containing derivatives thereof, wherein said esters and amides may comprise an acyl group selected from the group consisting of branched alkyl chains, unbranched alkyl chains, cycloalkyl groups, aromatic groups, and heteroatom derivatives thereof, and; wherein $R_2$ and $R_4$ are $CH_2$, $CHR_1$, NH, $NR_1$, or any heteroatom, and $R_1$ is selected from the group consisting of H, ethers, amines, branched alkyl chains, unbranched alkyl chains, cycloalkyl groups, aromatic groups, and heteroatom derivatives thereof.

Further disclosed is the above compound, wherein $R_3$, $R_5$, and $R_6$ are OH, $R_2$ is S, $R_1$ is selected from the group consisting of $CH_2CH_3$, $(CH_2)_2CH_3$, $(CH_2)_3CH_3$, $(CH_2)_4CH_3$, $CH(CH_3)_2$ and $CH_2CH(CH_3)_2$, and $R_4$ is O.

Also disclosed is the above compound, wherein $R_3$, $R_5$, and $R_6$ are OH, $R_2$ is S, and $R_1$ is $CH_2CH_3$, and $R_4$ is O.

Disclosed is also the above compound, wherein $R_3$, $R_5$, and $R_6$ are OH, $R_2$ is S, and $R_1$ is $(CH_2)_2CH_3$, and $R_4$ is O.

Further disclosed is the above compound, wherein $R_3$, $R_5$, and $R_6$ are OH, $R_2$ is S, and $R_1$ is $(CH_2)_3CH_3$, and $R_4$ is O.

Also disclosed is the above compound, wherein $R_3$, $R_5$, and $R_6$ are OH, $R_2$ is S, and $R_1$ is $(CH_2)_4CH_3$, and $R_4$ is O.

Likewise disclosed is the above compound, wherein $R_3$, $R_5$, and $R_6$ are OH, $R_2$ is S, and $R_1$ is $CH(CH_3)_2$, and $R_4$ is O.

Moreover disclosed is the above compound, wherein $R_3$, $R_5$, and $R_6$ are OH, $R_2$ is S, and $R_1$ is $CH_2CH(CH_3)_2$, and $R_4$ is O.

Furthermore disclosed is the above compound, wherein the compound is selected from the group consisting of 1,2-dideoxy-2'-ethyl-α-D-glucopyranoso-[2,1-d]-Δ2'- thiazoline, 1,2-dideoxy-2'-propyl-α-D-glucopyranoso-[2,1-d]-Δ2'-thiazoline, 1,2-dideoxy-2'-butyl-α-D-glucopyranoso-[2,1-d]-Δ2'-thiazoline, 1,2-dideoxy-2'-pentyl-α-D-glucopyranoso-[2,1-d]-Δ2'-thiazoline, 1,2-dideoxy-2'-isopropyl-α-D-glucopyranoso-[2,1-d]-Δ2'-thiazoline, and 1,2-dideoxy-2'-isobutyl-α-D- glucopyranoso-[2,1-d]-Δ2'-thiazoline, and pharmaceutically acceptable salts thereof.

Further disclosed is a prodrug comprising one or more of the above compounds.

Also disclosed is a prodrug comprising one or more of the above compounds, wherein $R_3$, $R_5$, and $R_6$ comprise an acyl group selected from the group consisting of branched alkyl chains, unbranched alkyl chains, cycloalkyl groups, aromatic groups, and heteroatom derivatives thereof.

Disclosed is also a pharmaceutical composition comprising one or more of the above compounds and a pharmaceutically acceptable carrier.

Further disclosed is a pharmaceutical composition comprising one or more of the above prodrugs and a pharmaceutically acceptable carrier.

Also disclosed is a pharmaceutical composition comprising one or more of the above compounds and a pharmaceutically acceptable carrier.

Moreover, a method of inhibiting a glycosidase is disclosed, comprising administering one or more of the above compounds.

Also disclosed is the above method, wherein the glycosidase comprises glycoside hydrolases.

Further disclosed is the above method, wherein the glycoside hydrolases comprise family 84 glycoside hydrolases.

Disclosed is further the above method, wherein the glycoside hydrolase comprises O-GlcNAcase.

Furthermore disclosed is a method of selectively inhibiting cleavage of O-GlcNAc comprising administering one or more of the above compounds.

Also disclosed is a method of selectively inhibiting cleavage of O-GlcNAc comprising administering one or more of the above prodrugs.

Moreover disclosed is a method of developing an animal model for studying a disease or disorder, or for studying treatment of a disease or disorder, related to deficiency or over-expression of O-GlcNAcase, or accumulation or depletion of O-GlcNAc, comprising administering the above compound to an animal.

Further disclosed is the above method, wherein the disease or disorder is selected from the group consisting of diabetes, neurodegenerative diseases, Alzheimer diseases, and cancer.

Also disclosed is an animal model developed according to the above method.

Likewise disclosed is a method of treating a disorder responsive to glycosidase inhibition therapy comprising administering the above compound.

Furthermore disclosed is a method of treating a disorder responsive to glycosidase inhibition therapy comprising administering the above prodrug.

Also disclosed is a method of promoting differentiation of islet β-cells, comprising administering the above compound.

Disclosed is also a method of preparing cells for stress, comprising administering the above compound.

Further disclosed is a method of making the above compound, comprising the steps of:

a) Acylating the salt of 2-amino-2-deoxy-1,3,4,6-tetra-*O*-acetyl-β-D-glucopyranose with a range of acylating agents to yield a series of 1,3,4,6-tetra-*O*-acetyl-2-deoxy-2-N-acyl-β-D-glucopyranose derivatives;

b) Converting the amides of the series of 1,3,4,6-tetra-*O*-acetyl-2-deoxy-2-N-acyl-β-D-glucopyranose derivatives to corresponding thioamides and cyclizing the thioamide to yield a series of 3,4,6-tri-*O*-aceryl-1,2-dideoxy-2'-alkyl-α-D-glucopyranoso-[2,1-d]-Δ2'-thiazolines; and

c) Deacylating the thiazoline compounds to yield a series of 1,2-dideoxy-2'-alkyl-α-D-glucopyranoso-[2,1-d]-Δ2'-thiazolines

Also disclosed is a method of making selective glycosidase inhibitors comprising the steps of:

a) Selecting an inhibitor of two or more glycosidases or a class of glycosidases;

b) Modifying one or more side chains of the inhibitor by enlarging or reducing the side chain; and

c) Testing the modified inhibitor for selective inhibition of one or more of the glycosidases. Further disclosed is a method of making the above selective glycosidase inhibitors, comprising the steps of:

a) Selecting an inhibitor of β-*N*-acetyl-glucosaminidases or β-hexosaminidases having a general formula selected from:

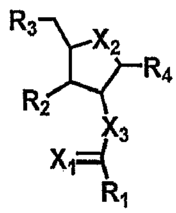
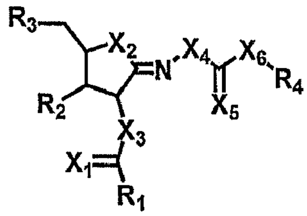
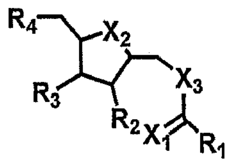

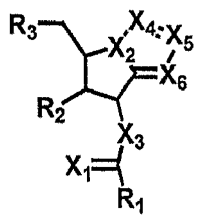
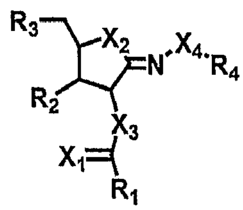
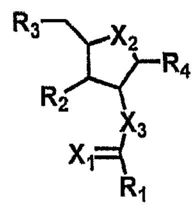

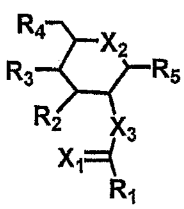
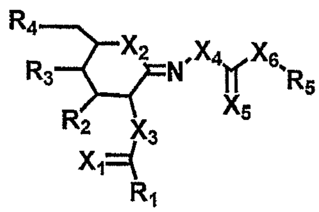
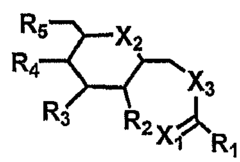

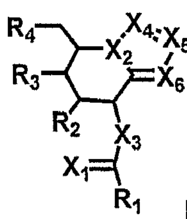
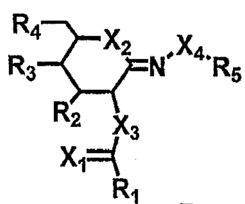
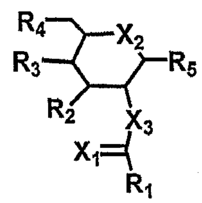

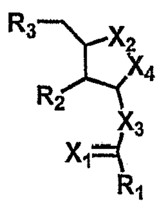
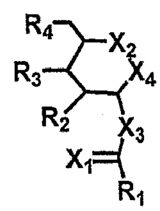

wherein $X_1$-$X_6$ are O, NH, $NR_1$, $CHR_1$, $CH_2$ or any heteroatom, R1 to R are selected from the group consisting of branched alkyl chains, unbranched alkyl chains, cycloalkyl groups, aromatic groups, alcohols, ethers, amines, substituted or unsubstituted carbamates, substituted or unsubstituted ureas, esters, amides, aldehydes, carboxylic acids, and heteroatom containing derivatives thereof, wherein said esters and amides may comprise an acyl group selected from the group consisting of branched alkyl chains, unbranched alkyl chains, cycloalkyl groups, aromatic groups, and heteroatom derivatives thereof, and pharmaceutically acceptable salts thereof;

b) Modifying the R1 side chain of the inhibitor by enlarging or reducing the side chain; and

c) Testing the modified inhibitors for selective inhibition of one or more β-*N*-acetyl-glucosaminidases or β-hexosaminidases.

Further disclosed is the above method of making selective glycosidase inhibitors, wherein the side chain of the inhibitor are enlarged by inserting a branched alkyl chain, an unbranched alkyl chain, a cycloalkyl group, an aromatic group, or heteroatom derivatives thereof.

Also disclosed is the above method of making selective glycosidase, wherein the side chain $R_1$ of the inhibitors is enlarged by inserting a group selected from the group consisting of $CH_2CH_3$, $(CH_2)_2CH_3$, $(CH_2)_3CH_3$, $(CH_2)_4CH_3$, $CH(CH_s)_2$ and $CH_2CH(CH_3)_2$.

Furthermore disclosed is a selective glycosidase inhibitor comprising a compound selected from the group consisting of the following structures:

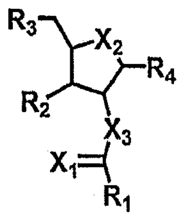
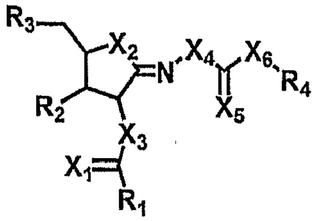
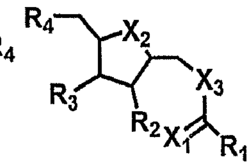
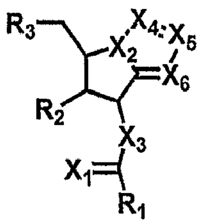
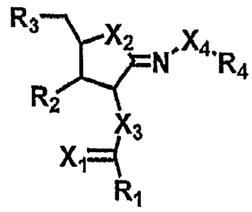
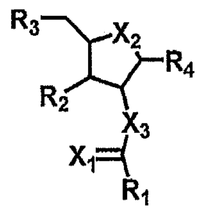
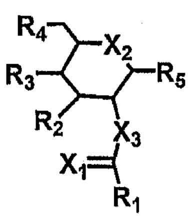
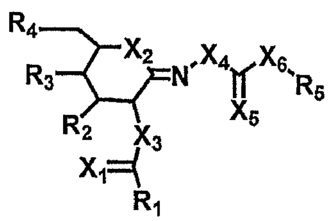
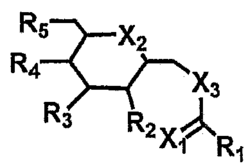
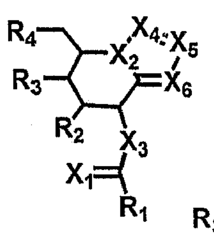
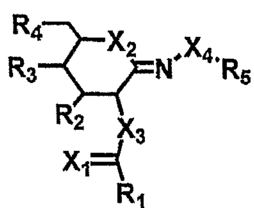
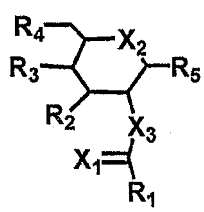
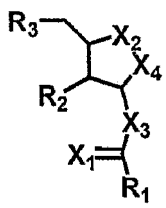
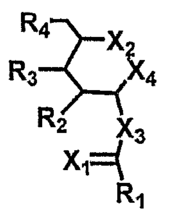

wherein $X_1$-$X_6$ are O, NH, $NR_1$, $CHR_1$, $CH_2$ or any heteroatom, $R_1$ to $R_5$ are selected from the group consisting of branched alkyl chains, unbranched alkyl chains, cycloalkyl groups, aromatic groups, alcohols, ethers, amines, substituted or unsubstituted carbamates, substituted or unsubstituted ureas, esters, amides, aldehydes, carboxylic acids, and heteroatom containing derivatives thereof, wherein said esters and amides may comprise an acyl group selected from the group consisting of branched alkyl chains, unbranched alkyl chains, cycloalkyl groups, aromatic groups, and heteroatom derivatives thereof, and pharmaceutically acceptable salts thereof.

Likewise disclosed is a compound having the general chemical formula (II) and pharmaceutically acceptable salts thereof:

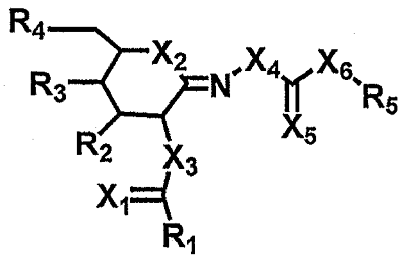

(II)

wherein $X_1$-$X_6$ are O, NH, $NR_1$, $CHR_1$, $CH_2$ or any heteroatom, $R_1$ to $R_5$ are selected from the group consisting of branched alkyl chains, unbranched alkyl chains, cycloalkyl groups, aromatic groups, alcohols, ethers, amines, substituted or unsubstituted carbamates, substituted or unsubstituted ureas, esters, amides, aldehydes, carboxylic acids, and heteroatom containing derivatives thereof, wherein said esters and amides may comprise an acyl group selected from the group consisting of branched alkyl chains, unbranched alkyl chains, cycloalkyl groups, aromatic groups, and heteroatom derivatives thereof, and pharmaceutically acceptable salts thereof.

Disclosed is also an inhibitor which selectively inhibits O-GlcNAcase, wherein said inhibitor is selected from the group of compounds defined above.

## REFERENCES

[1] C. R. Torres, G. W. Hart, J Biol Chem 1984,259, 3308-3317.
[2] S. P. Jackson, R. Tjian, Cell 1988, 55, 125-133.
[3] W. G. Kelly, M. E. Dahmus, G. W. Hart, J Biol Chem 1993, 268, 10416-10424.
[4] M. D. Roos, K. Su, J. R. Baker, J. E. Kudlow, Mol Cell Biol 1997,17, 6472-6480.
[5] N. Lamarre-Vincent, L. C. Hsieh-Wilson, J Am Chem Soc 2003, 125, 6612-6613.
[6] F. Zhang, K. Su, X. Yang, D. B. Bowe, A. J. Paterson, J. E. Kudlow, Cell 2003,115, 715-725.
[7] K. Vosseller, L. Wells, M. D. Lane, G. W. Hart, Proc Natl Acad Sci U S A 2002, 99, 5313-5318.
[8] W. A. Lubas, M. Smith, C. M. Starr, J. A. Hanover, Biochemistry 1995, 34, 1686-1694.
[9] L. S. Griffith, B. Schmitz, Biochem Biophys Res Commun 1995, 213, 424-431.
[10] R. N. Cole, G. W. Hart, J Neurochem 1999, 73, 418-428.
[11] I. Braidman, M. Carroll, N. Dance, D. Robinson, Biochem J 1974, 143, 295-301.
[12] R. Ueno, C. S. Yuan, Biochim Biophys Acta 1991, 1074, 79-84.
[13] D. L. Dong, G. W. Hart, J Biol Chem 1994, 269, 19321-19330.
[14] C. Toleman, A. J. Paterson, T. R. Whisenhunt, J. E. Kudlow, J Biol Chem 2004.
[15] Y. Gao, L. Wells, F. I. Comer, G. J. Parker, G. W. Hart, J Biol Chem 2001, 276, 9838-9845.
[16] L. Wells, Y. Gao, J. A. Mahoney, K. Vosseller, C. Chen, A. Rosen, G. W. Hart, J Biol Chem 2002, 277, 1755-1761.
[17] F. Liu, K. Iqbal, I. Grundke-Iqbal, G. W. Hart, C. X. Gong, Proc Natl Acad Sci U S A 2004, 101, 10804-10809.
[18] T. Y. Chou, G. W. Hart, Adv Exp Med Biol 2001, 491, 413-418.
[19] B. Henrissat, A. Bairoch, Biochem J 1996, 316 (Pt 2), 695-696.
[20] B. Henrissat, A. Bairoch, Biochem J 1993, 293 (Pt 3), 781-788.
[21] D. A. McClain, W. A. Lubas, R. C. Cooksey, M. Hazel, G. J. Parker, D.C. Love, J. A. Hanover, Proc Natl Acad Sci U S A 2002, 99, 10695-10699.
[22] L. Wells, K. Vosseller, G. W. Hart, Science 2001, 291, 2376-2378.
[23] J. A. Hanover, FASEB J 2001, 15, 1865-1876.
[24] P. J. Yao, P. D. Coleman, J Neurosci 1998, 18, 2399-2411.
[25] B. Triggs-Raine, D. J. Mahuran, R. A. Gravel, Adv Genet 2001, 44, 199-224.
[26] D. Zhou, J. Mattner, C. Cantu Iii, N. Schrantz, N. Yin, Y. Gao, Y. Sagiv, K. Hudspeth, Y. Wu, T. Yamashita, S. Teneberg, D. Wang, R. Proia, S. B. Levery, P. B. Savage, L. Teyton, A. Bendelac, Science 2004.
[27] G. Legler, E. Lullau, E. Kappes, F. Kastenholz, Biochim Biophys Acta 1991, 1080, 89-95.
[28] M. Horsch, L. Hoesch, A. Vasella, D. M. Rast, Eur J Biochem 1991, 197, 815-818.
[29] J. Liu, A. R. Shikhman, M. K. Lotz, C. H. Wong, Chem Biol 2001, 8, 701-711.

[30] S. Knapp, D. J. Vocadlo, Z. N. Gao, B. Kirk, J. P. Lou, S. G. Withers, J. Am. Chem. Soc. 1996, 118, 6804-6805.
[31] V. H. Lillelund, H. H. Jensen, X. Liang, M. Bols, Chem Rev 2002, 102, 515-553.
[32] R. J. Konrad, I. Mikolaenko, J. F. Tolar, K. Liu, J. E. Kudlow, Biochem J 2001, 356, 31-41.
[33] K. Liu, A. J. Paterson, F. Zhang, J. McAndrew, K. Fukuchi, J. M. Wyss, L. Peng, Y. Hu, J. E. Kudlow, J Neurochem 2004, 89, 1044-1055.
[34] G. Parker, R. Taylor, D. Jones, D. McClain, J Biol Chem 2004, 279, 20636-20642.
[35] E. B. Arias, J. Kim, G. D. Cartee, Diabetes 2004, 53, 921-930.
[36] A. Junod, A. E. Lambert, L. Orci, R. Pictet, A. E. Gonet, A. E. Renold, Proc Soc Exp Biol Med 1967, 126, 201-205.
[37] R. A. Bennett, A. E. Pegg, Cancer Res 1981, 41, 2786-2790.
[38] K. D. Kroncke, K. Fehsel, A. Sommer, M. L. Rodriguez, V. Kolb-Bachofen, Biol Chem Hoppe Seyler 1995, 376, 179-185.
[39] H. Yamamoto, Y. Uchigata, H. Okamoto, Nature 1981, 294, 284-286.
[40] K. Yamada, K. Nonaka, T. Hanafusa, A. Miyazaki, H. Toyoshima, S. Tarui, Diabetes 1982, 31, 749-753.
[41] V. Burkart, Z. Q. Wang, J. Radons, B. Heller, Z. Herceg, L. Stingl, E. F. Wagner, H. Kolb, Nat Med 1999, 5, 314-319.
[42] M. D. Roos, W. Xie, K. Su, J. A. Clark, X. Yang, E. Chin, A. J. Paterson, J. E. Kudlow, Proc Assoc Am Physicians 1998, 110, 422-432.
[43] Y. Gao, G. J. Parker, G. W. Hart, Arch Biochem Biophys 2000, 383, 296-302.
[44] R. Okuyama, M. Yachi, Biochem Biophys Res Commun 2001, 287, 366-371.
[45] N. E. Zachara, N. O'Donnell, W. D. Cheung, J. J. Mercer, J. D. Marth, G. W. Hart, J Biol Chem 2004, 279, 30133-30142.
[46] J. A. Hanover, Z. Lai, G. Lee, W. A. Lubas, S. M. Sato, Arch Biochem Biophys 1999, 362, 38-45.
[47] K. Liu, A. J. Paterson, R. J. Konrad, A. F. Parlow, S. Jimi, M. Roh, E. Chin, Jr., J. E. Kudlow, Mol Cell Endocrinol 2002, 194, 135-146.
[48] B. L. Mark, D. J. Vocadlo, S. Knapp, B. L. Triggs-Raine, S. G. Withers, M. N. James, J Biol Chem 2001, 276, 10330-10337.
[49] M. S. Macauley, G. E. Whitworth, A. W. Debowski, D. Chin, D. J. Vocadlo, J Biol Chem 2005, 280, 25313-25322.
[50] R. S. Haltiwanger, K. Grove, G. A. Philipsberg, J Biol Chem 1998, 273, 3611-3617.
[51] D. J. Miller, X. Gong, B. D. Shur, Development 1993, 118, 1279-1289.
[52] S. Marshall, W. T. Garvey, R. R. Traxinger, Faseb J 1991, 5, 3031 -3036.
[53] R. S. Haltiwanger, G. D. Holt, G. W. Hart, J Biol Chem 1990, 265, 2563-2568.
[54] L. K. Kreppel, M. A. Blomberg, G. W. Hart, J Biol Chem 1997, 272, 9308-9315.
[55] W. A. Lubas, D. W. Frank, M. Krause, J. A. Hanover, J Biol Chem 1997, 272, 9316-9324.
[56] W. A. Lubas, J. A. Hanover, J Biol Chem 2000, 275, 10983-10988.
[57] S. P. Iyer, Y. Akimoto, G. W. Hart, J Biol Chem 2003, 278, 5399-5409.
[58] K. Brickley, M. J. Smith, M. Beck, F. A. Stephenson, J Biol Chem 2005, 280, 14723-14732.
[59] X. Yang, F. Zhang, J. E. Kudlow, Cell 2002, 110, 69-80.
[60] L. Wells, L. K. Kreppel, F. I. Comer, B. E. Wadzinski, G. W. Hart, J Biol Chem 2004, 279, 38466-38470.
[61] S. P. Iyer, G. W. Hart, J Biol Chem 2003, 278, 24608-24616.
[62] M. Jinek, J. Rehwinkel, B. D. Lazarus, E. Izaurralde, J. A. Hanover, E. Conti, Nat Struct Mol Biol 2004, 11, 1001-1007.
[63] E. P. Roquemore, M. R. Chevrier, R. J. Cotter, G. W. Hart, Biochemistry 1996, 35, 3578-3586.
[64] K. Kamemura, B. K. Hayes, F. I. Corner, G. W. Hart, J Biol Chem 2002, 277, 19229-19235.
[65] X. Cheng, G. W. Hart, J Biol Chem 2001, 276, 10570-10575.
[66] X. Cheng, R. N. Cole, J. Zaia, G. W. Hart, Biochemistry 2000, 39, 11609-11620.
[67] L. K. Kreppel, G. W. Hart, J Biol Chem 1999, 274, 32015-32022.
[68] N. E. Zachara, G. W. Hart, Biochim Biophys Acta 2004, 1673, 13-28.
[69] M. Goedert, M. G. Spillantini, N. J. Cairns, R. A. Crowther, Neuron 1992, 8, 159-168.
[70] M. Goedert, M. G. Spillantini, R. Jakes, D. Rutherford, R. A. Crowther, Neuron 1989, 3, 519-526.
[71] E. Kopke, Y. C. Tung, S. Shaikh, A. C. Alonso, K. Iqbal, I. Grundke-Iqbal, J Biol Chem 1993, 268, 24374-24384.
[72] H. Ksiezak-Reding, W. K. Liu, S. H. Yen, Brain Res 1992, 597, 209-219.
[73] T. Y. Chou, G. W. Hart, C. V. Dang, J Biol Chem 1995, 270, 18961-18965.
[74] L. S. Griffith, B. Schmitz, Eur JBiochem 1999, 262, 824-831.
[75] K. Kamemura, G. W. Hart, Prog Nucleic Acid Res Mol Biol 2003, 73, 107-136.
[76] L. Bertram, D. Blacker, K. Mullin, D. Keeney, J. Jones, S. Basu, S. Yhu, M. G. McInnis, R. C. Go, K. Vekrellis, D. J. Selkoe, A. J. Saunders, R. E. Tanzi, Science 2000, 290, 2302-2303.
[77] U. J. G. Conference, in US/Japan Glyco 2004 Conference, Honolulu, Hawaii, 2004.
[78] B. L. Mark, D. J. Mahuran, M. M. Cherney, D. Zhao, S. Knapp, M. N. James, J Mol Biol 2003, 327, 1093-1109.

[79] R. Kuroki, L. H. Weaver, B. L. Matthews, Proc. Natl. Acad. Sci. USA 1999, 96, 8949-8954.
[80] D. L. Zechel, S. G. Withers, Acc Chem Res 2000, 33, 11-18.
[81] D. J. Vocadlo, C. Mayer, S. He, S. G. Withers, Biochemistry 2000, 39, 117-126.
[82] D. J. Vocadlo, G. J. Davies, R. Laine, S. G. Withers, Nature 2001, 412, 835-838.
[83] I. Tews, A. Perrakis, A. Oppenheim, Z. Dauter, K. S. Wilson, C. E. Vorgias, Nat Struct Biol 1996, 3, 638-648.
[84] K. R. Roeser, G. Legler, Biochim Biophys Acta 1981, 657, 321-333.
[85] M. S. Macauley, K. A. Stubbs, D. J. Vocadlo, J Am Chem Soc 2005, 127, 17202-17203.
[86] C. Hansch, A. Leo, Substituent constants for correlation analysis in chemistry and biology., Wiley, New York, 1979.
[87] T. Maier, N. Strater, C. G. Schuette, R. Klingenstein, K. Sandhoff, W. Saenger, J Mol Biol 2003, 328, 669-681.
[88] C. S. Jones, D. J. Kosman, J Biol Chem 1980, 255, 11861-11869.
[89] A. C. Terwisscha van Scheltinga, S. Armand, K. H. Kalk, A. Isogai, B. Henrissat, B. W. Dijkstra, Biochemistry 1995, 34, 15619-15623.
[90] Z. Markovic-Housley, G. Miglierini, L. Soldatova, P. J. Rizkallah, U. Muller, T. Schirmer, Structure Fold Des 2000, 8, 1025-1035.
[91] B. L. Mark, M. N. G. James, Can. J Chem. 2002, 80, 1064-1074.
[92] M. Bergmann, L. Zervas, Ber. Dtsch. Chem. Ges. 1999, 64B, 975-980.
[93] A. C. Cunha, L. O. R. Pereira, d. S. M. C. B. V., V. F. Ferreira, J. Chem. Ed. 1999, 76, 79-80.
[94] P. Boullanger, M. Jouineau, B. Bouammali, D. Lafont, G. Descotes, Carbohydr, Res. 1990, 202, 151-164.
[95] H. Mohan, A. Vasella, Nelv Chim Acta 2000, 83, 114-118.
[96] T. D. Heightman, A. T. Vasella, Angew Chem Int Edit 1999, 38, 750-770.
[97] D. J. Vocadlo, S. G. Withers, Biochemistry 2005, 44, 12809-12818.
[98] M. Jeyakumar, R. A. Dwek, T. D. Butters, F. M. Platt, Nat Rev Neurosci 2005, 6, 713-725.
[99] R. J. van den Berg, W. Donker-Koopman, J. H. van Boom, H. M. Aerts, D. Noort, Bioorg Med Chem 2004, 12, 891-902.
[100] E. Shitara, Y. Nishimura, F. Kojima, T. Takeuchi, Bioorg Med Chem 1999, 7, 1241-1246.
[101] M. B. Tropak, S. P. Reid, M. Guiral, S. G. Withers, D. Mahuran, J Biol Chem 2004, 279, 13478-13487.

## Claims

**1.** A compound comprising the general chemical formula (II):

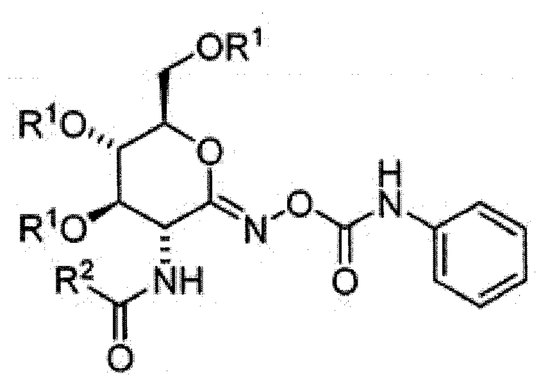

(II)

wherein
$R^1$ is selected from the group consisting of H and $COCH_3$; and
$R^2$ is selected from the group consisting of branched alkyl chains, unbranched alkyl chains, and cycloalkyl groups, or a pharmaceutically acceptable salt thereof.

**2.** The compound of claim 1, wherein $R^2$ is selected from the group consisting of a $C_2$-$C_{20}$ alkyl chain, a $C_2$-$C_{20}$ branched alkyl chain, and a $C_3$-$C_{10}$ cycloalkyl group.

**3.** The compound of claim 1, wherein the compound is selected from the group consisting of:

O-(2-deoxy-2-propamido-D-glucopyranosylidene)amino *N*-Phenylcarbamate (IUPAC name: N-((3R,4R,5S,6R)-4,5-dihydroxy-6-(hydroxymethyl)-2-(phenylcarbamoyl oxyimino)-tetrahydro-2H-pyran-3-yl)propionamide),
O-(2-deoxy-2-butamido-D-glucopyranosylidene)amino N-Phenylcarbamate (IUPAC name: N-((3R,4R,5S,6R)-4,5 -dihydroxy-6-(hydroxymethyl)-2-(phenylcarbamoyloxyimino)-tetrahydro-2H-pyran-3-yl)butyramide),
O-(2-deoxy-2-valeramido-D-glucopyranosylidene)amino N-Phenylcarbamate (IUPAC name: N-((3R,4R,5S,6R)-4,5-dihydroxy-6-(hydroxymethyl)-2-(phenylcarbamoyl oxyimino)-tetrahydro-2H-pyran-3-yl)pentanamide),
O-(2-deoxy-2-hexamido-D-glucopyranosylidene)amino *N*-Phenylcarbamate (IUPAC name: N-((3R,4R,5S,6R)-4,5 -dihydroxy-6-(hydroxymethyl)-2-(phenylcarbamoyloxymino)-tetrahydro-2H-pyran-3-yl)hexanamide),
O-(2-deoxy-2-isobutamido-D-glucopyranosylidene)amino *N*-Phenylcarbamate (IUPAC name: N-((3R,4R,5S,6R)-4,5-dihydroxy-6-(hydroxymethyl)-2-(phenylcarbamoyl oxyimino)-tetrahydro-2H-pyran-3-yl)isobutyramide),
O-(2-deoxy-2-isovaleramido-D-glucopyranosylidene)amino *N*-Phenylcarbamate (IUPAC name: N-((3R,4R,5S,6R)-4,5-dihydroxy-6-(hydroxymethyl-2-(phenylcarbamoyl oxyimino)-tetrahydro-2H-pyran-3-yl)-3-methylbutanamide,
(2R,3S,4R,5R)-2-(acetoxymethyl)-6-(phenylcarbamoyloxyimino)-5-propionamido-tetrahydro-2H-pyran-3,4-diyl diacetate,
(2R,3S,4R,5R)-2-(acetoxymethyl)-5-butyramido-6-(phenylcarbamoyloxyimino)-tetrahydro-2H-pyran-3,4-diyl diacetate,
(2R,3S,4R,5R)-2-(acetoxymethyl)-5-pentanamido-6-(phenylcarbamoyloxyimino)-tetrahydro-2H-pyran-3,4-diyl diacetate,
(2R,3S,4R,5R)-2-(acetoxymethyl)-5-hexanamido-6-(phenylcarbamoyloxyimino)-tetrahydro-2H-pyran-3,4-diyl diacetate,
(2R,3S,4R,5R)-2-(acetoxymethyl)-5-isobutyramido-6-(phenylcarbamoyloxyimino)-tetrahydro-2H-pyran-3,4-diyl diacetate, and
(2R, 3S, 4R, 5R)- 2-(acetoxymethyl)- 5-(3- methylbutanamido)- 6-(phenylcarbamoyloxyimino)-tetrahydro- 2H-pyran-3,4-diyl diacetate, or
a pharmaceutically acceptable salt thereof.

**4.** The compound of claim 1, wherein the compound comprises a prodrug.

**5.** The compound of claim 4 wherein the prodrug is selected from the group consisting of:

(2R,3S,4R,5 R)-2-(acetoxymethyl)-6-(phenylcarbamoyloxyimino)-5-propionamido-tetrahydro-2H-pyran-3,4-diyl diacetate,
(2R,3S,4R,5R)-2-(acetoxymethyl)-5-butyramido-6-(phenylcarbamoyloxyimino)-tetrahydro-2H-pyran-3,4-diyl diacetate,
(2R,3S,4R,5R)-2-(acetoxymethyl)-5-pentanamido-6-(phenylcarbamoyloxyimino)-tetrahydro-2H-pyran-3,4-diyl diacetate,
(2R,3S,4R,5R)-2-(acetoxymethyl)-5-hexanamido-6-(phenylcarbamoyloxyimino)-tetrahydro-2H-pyran-3,4-diyl diacetate,
(2R,3S,4R,5R)-2-(acetoxymethyl)-5-isobutyramido-6-(phenylcarbamoyloxyimino)-tetrahydro-2H-pyran-3,4-diyl diacetate, and
(2R, 3S, 4R, 5R)- 2-(acetoxymethyl)- 5-(3- methylbutanamido)- 6-(phenylcarbamoyloxyimino)-tetrahydro- 2H-pyran-3,4-diyl diacetate,
or a pharmaceutically acceptable salt thereof.

**6.** The compound of any one of claims 1 to 5 wherein the compound or a pharmaceutically acceptable salt thereof is provided as a pharmaceutical composition in combination with a pharmaceutically acceptable carrier.

**7.** The compound of any one of claims 1 to 5 or a pharmaceutically acceptable salt thereof for use in treating a disorder selected from one or more of the group consisting of diabetes, a neurodegenerative disease, a tauopathy, and cancer.

**8.** The compound of claim 7 wherein the tauopathy is Alzheimer disease.

## Figure 1

A

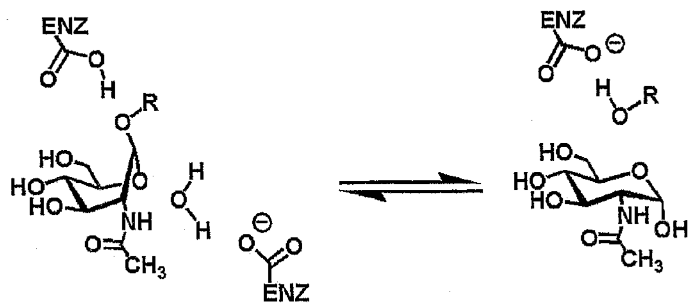

B

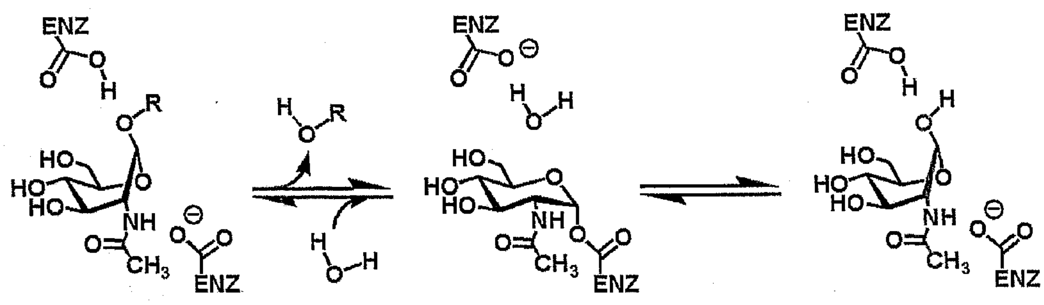

C

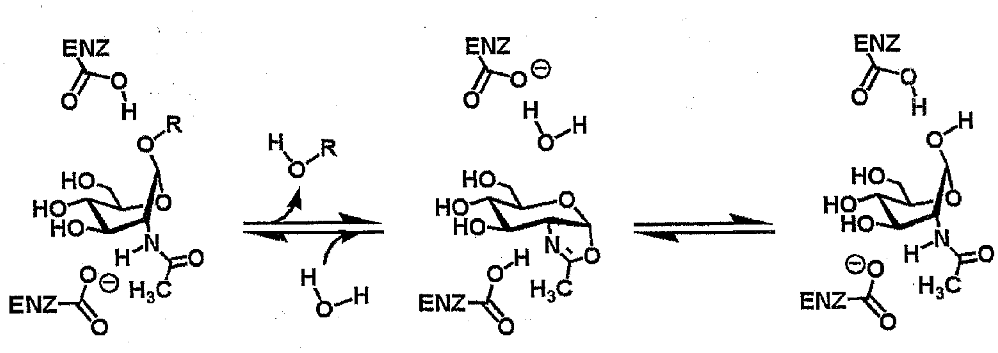

# Figure 2

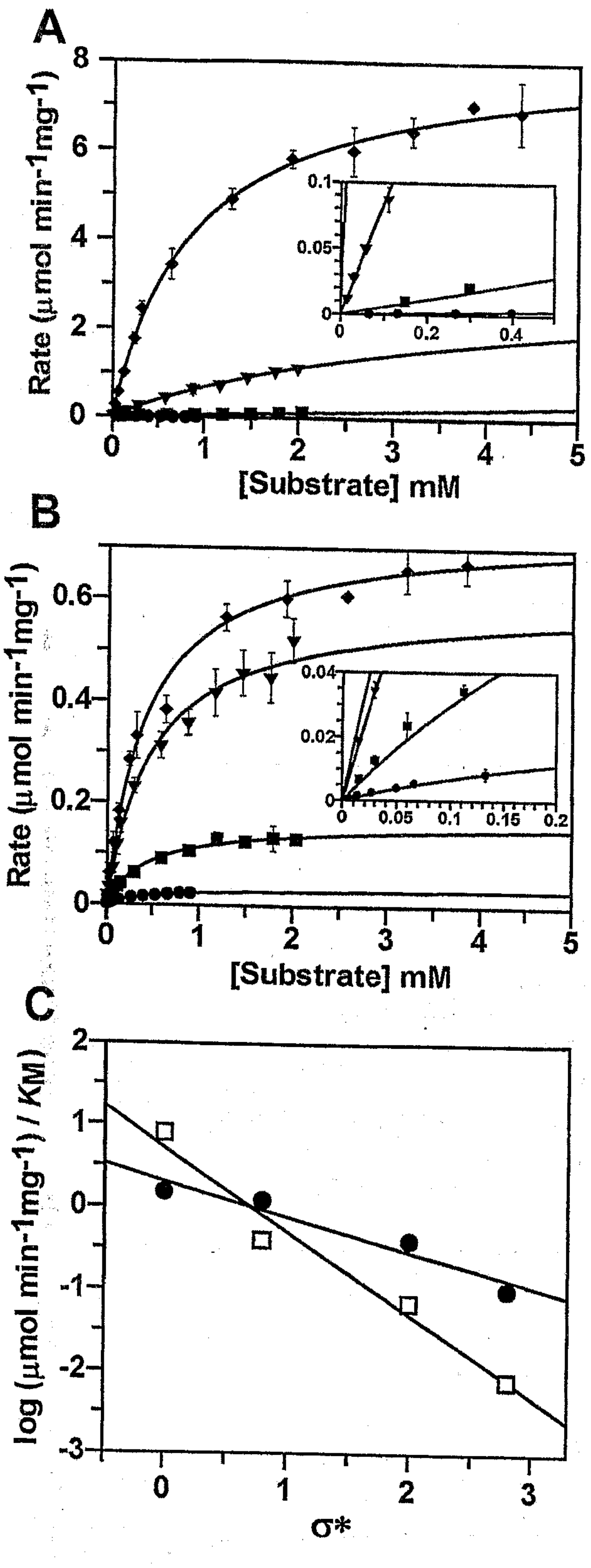
A
Rate (μmol min-1mg-1)
[Substrate] mM
B
Rate (μmol min-1mg-1)
[Substrate] mM
C
log (μmol min-1mg-1) / KM)
σ*

Figure 3

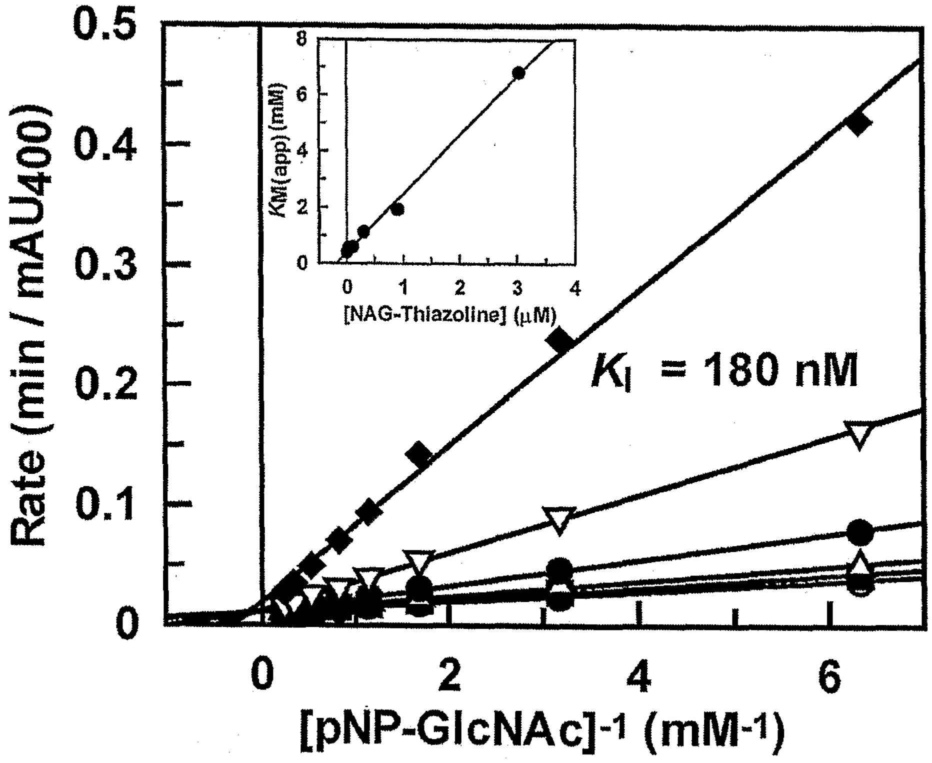
0.5
0.4
0.3
0.2
0.1
0
Rate (min / mAU400)
0
2
4
6
[pNP-GlcNAc]-1 (mM-1)
KI = 180 nM
KM(app) (mM)
8
6
4
2
0
0
1
2
3
4
[NAG-Thiazoline] (μM)

Figure 4

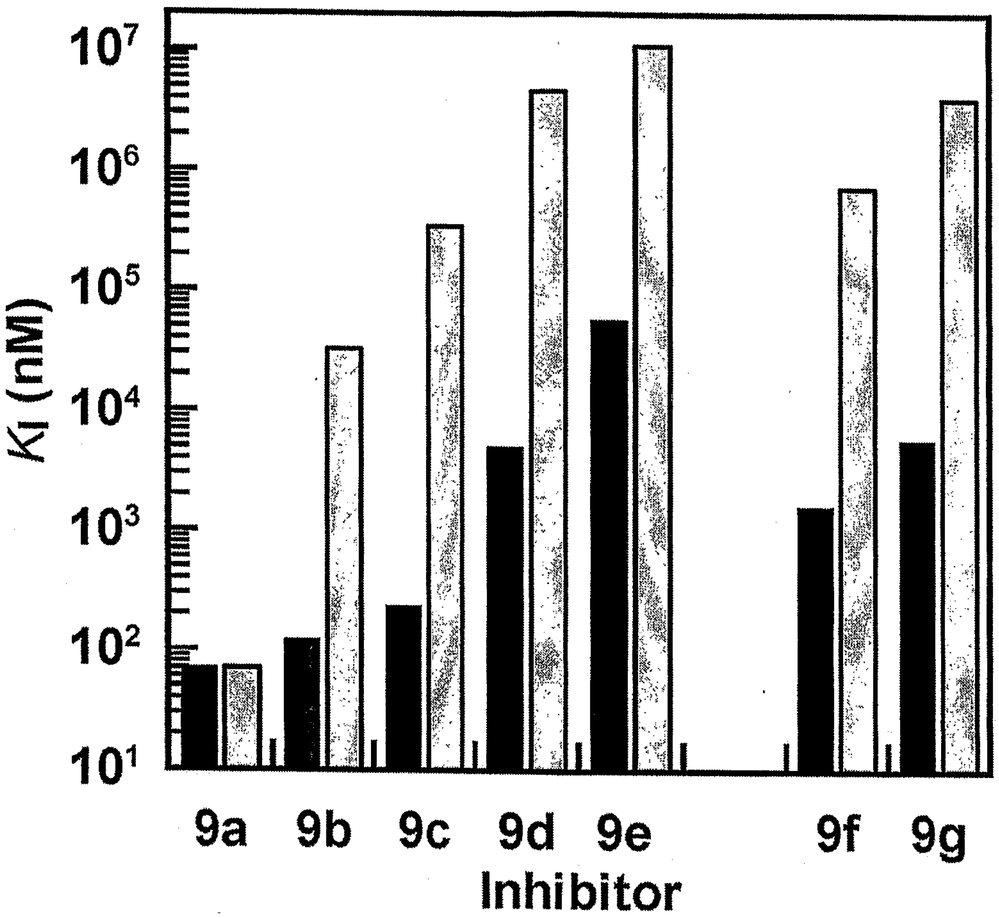

Figure 5

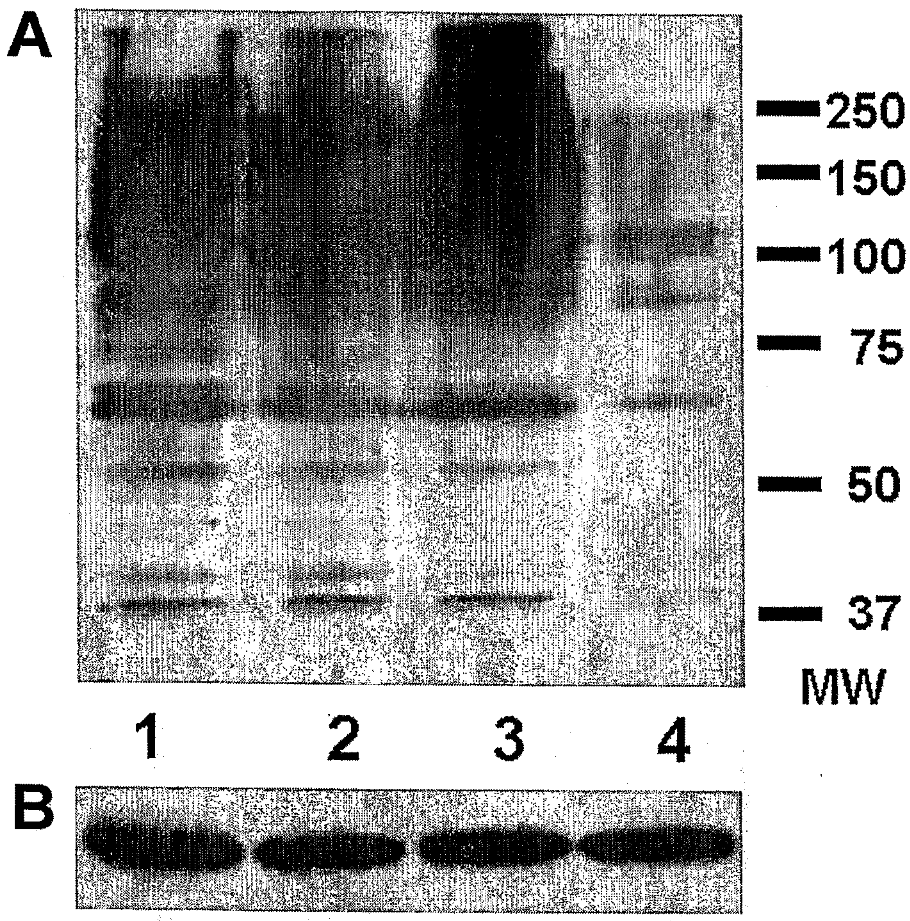

# Figure 6

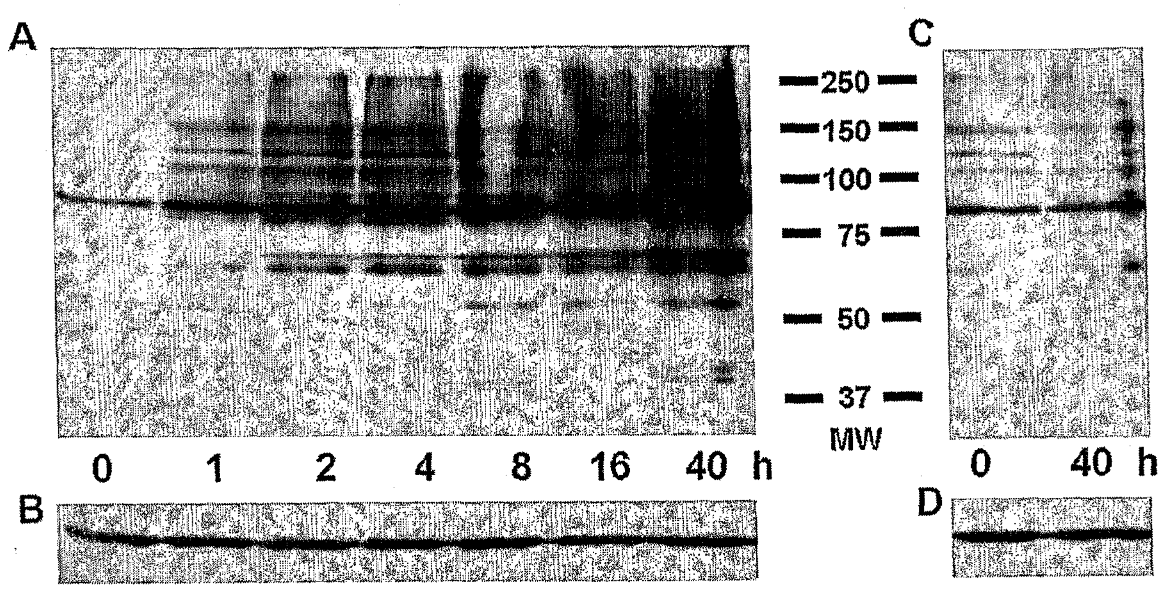

# Figure 7

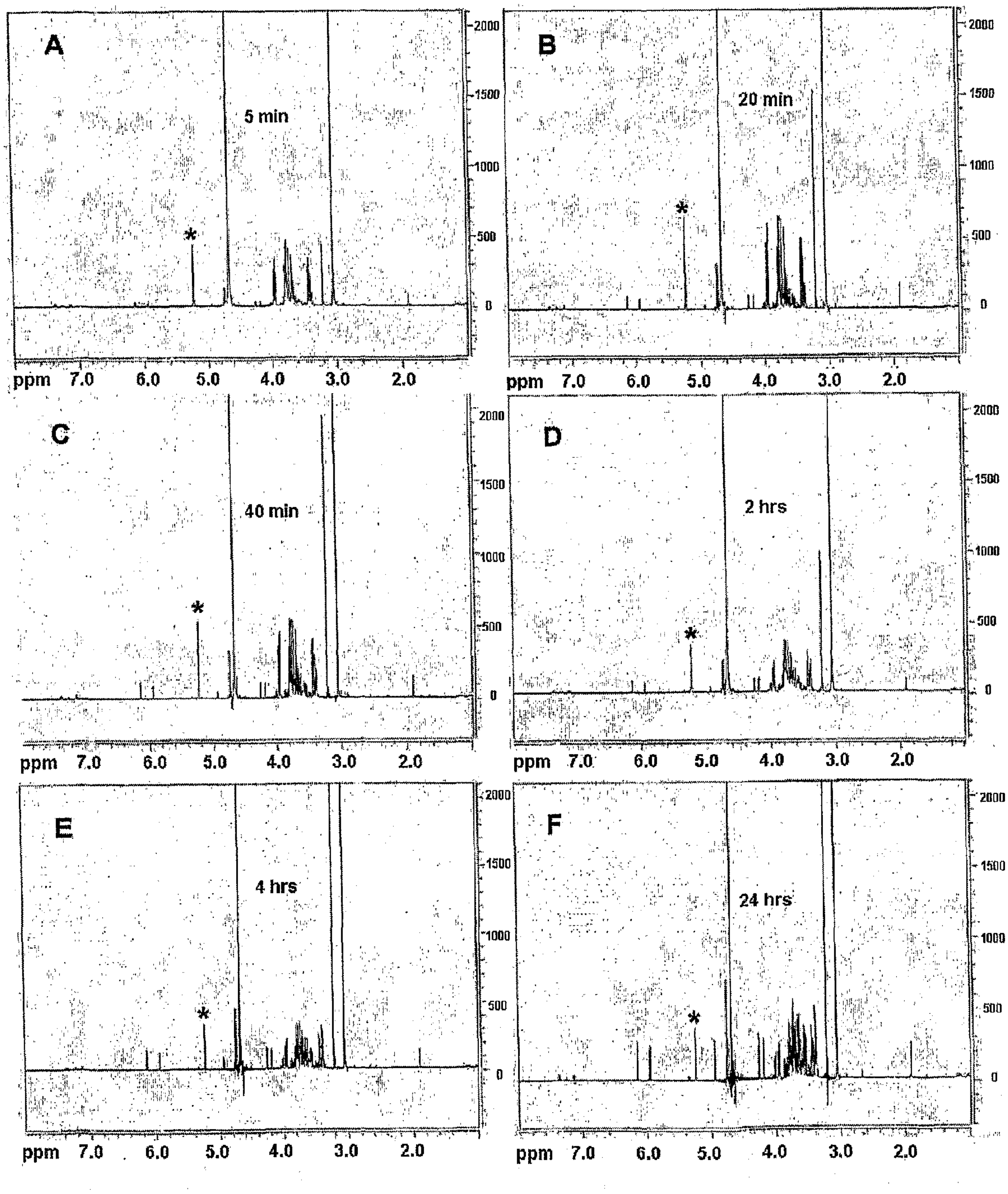
A
5 min
*
B
20 min
*
C
40 min
*
D
2 hrs
*
E
4 hrs
*
F
24 hrs
*
2000
1500
1000
500
0
ppm 7.0 6.0 5.0 4.0 3.0 2.0

Figure 8

A

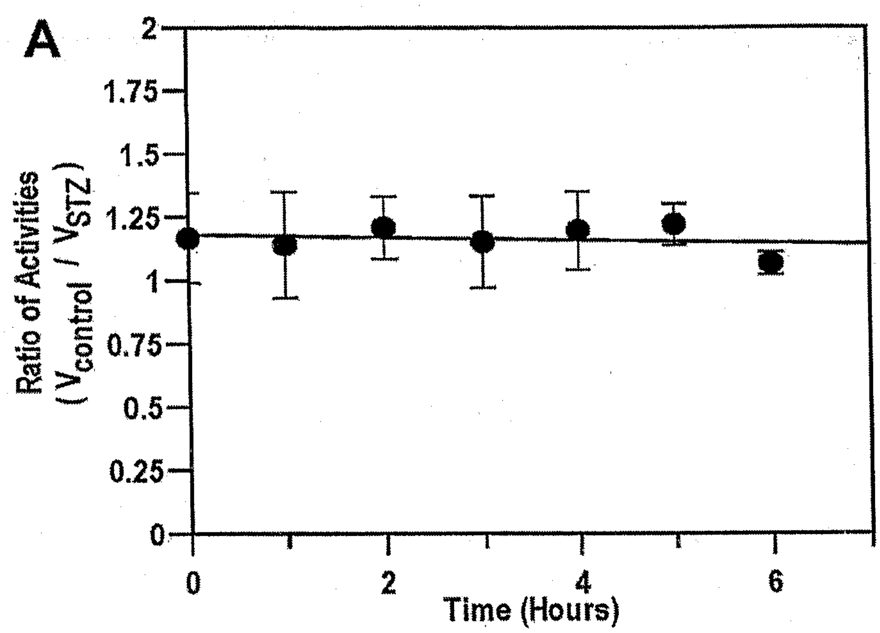
Ratio of Activities
( $V_{control}$ / $V_{STZ}$ )
2
1.75
1.5
1.25
1
0.75
0.5
0.25
0
0
2
4
6
Time (Hours)

B

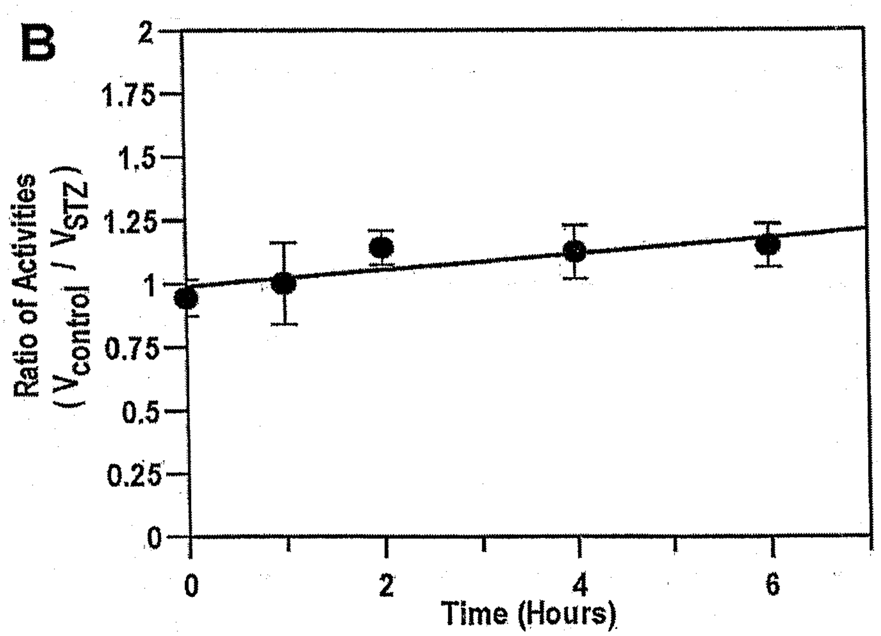
Ratio of Activities
( $V_{control}$ / $V_{STZ}$ )
2
1.75
1.5
1.25
1
0.75
0.5
0.25
0
0
2
4
6
Time (Hours)

Figure 9

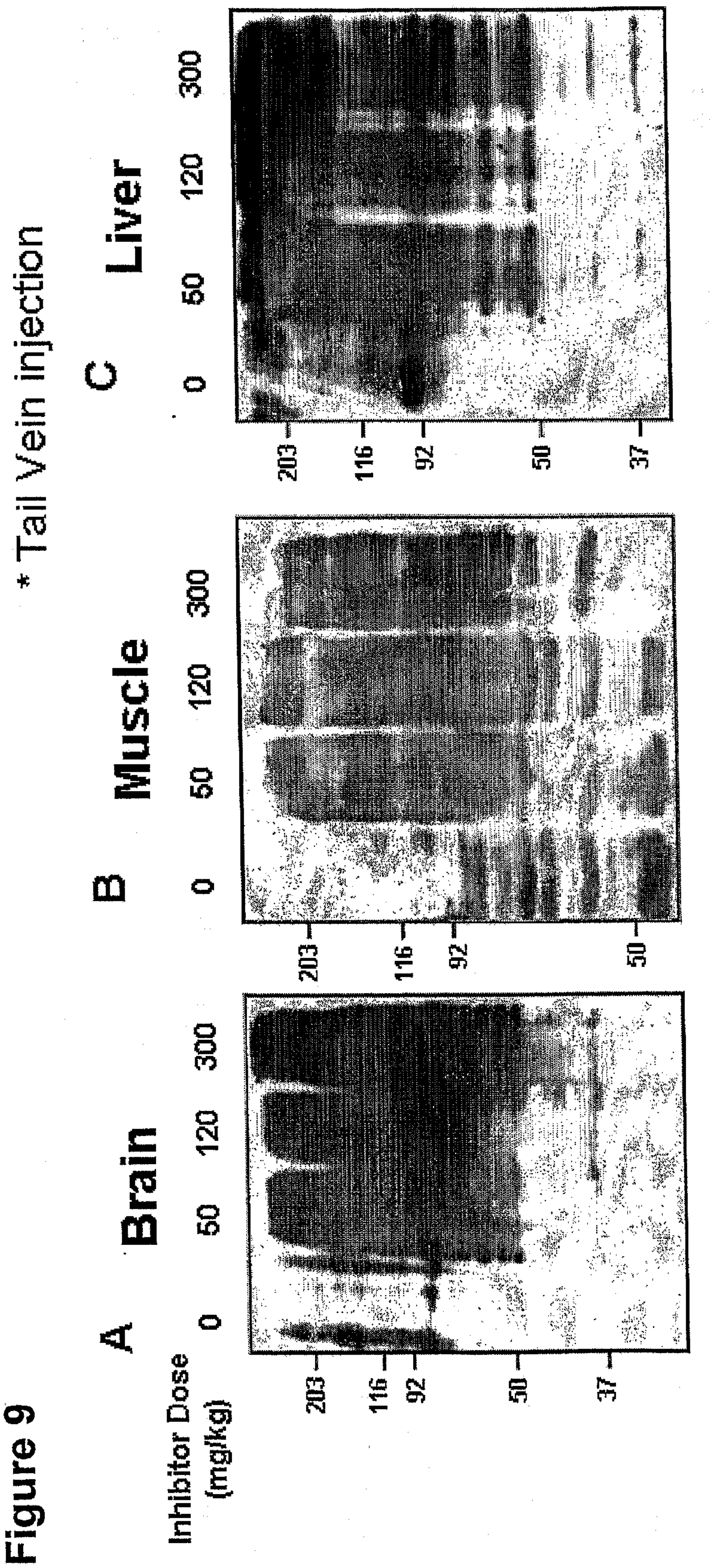
* Tail Vein injection
Inhibitor Dose
(mg/kg)
A
Brain
0
50
120
300
203
116
92
50
37
B
Muscle
0
50
120
300
203
116
92
50
C
Liver
0
50
120
300
203
116
92
50
37

Figure 10

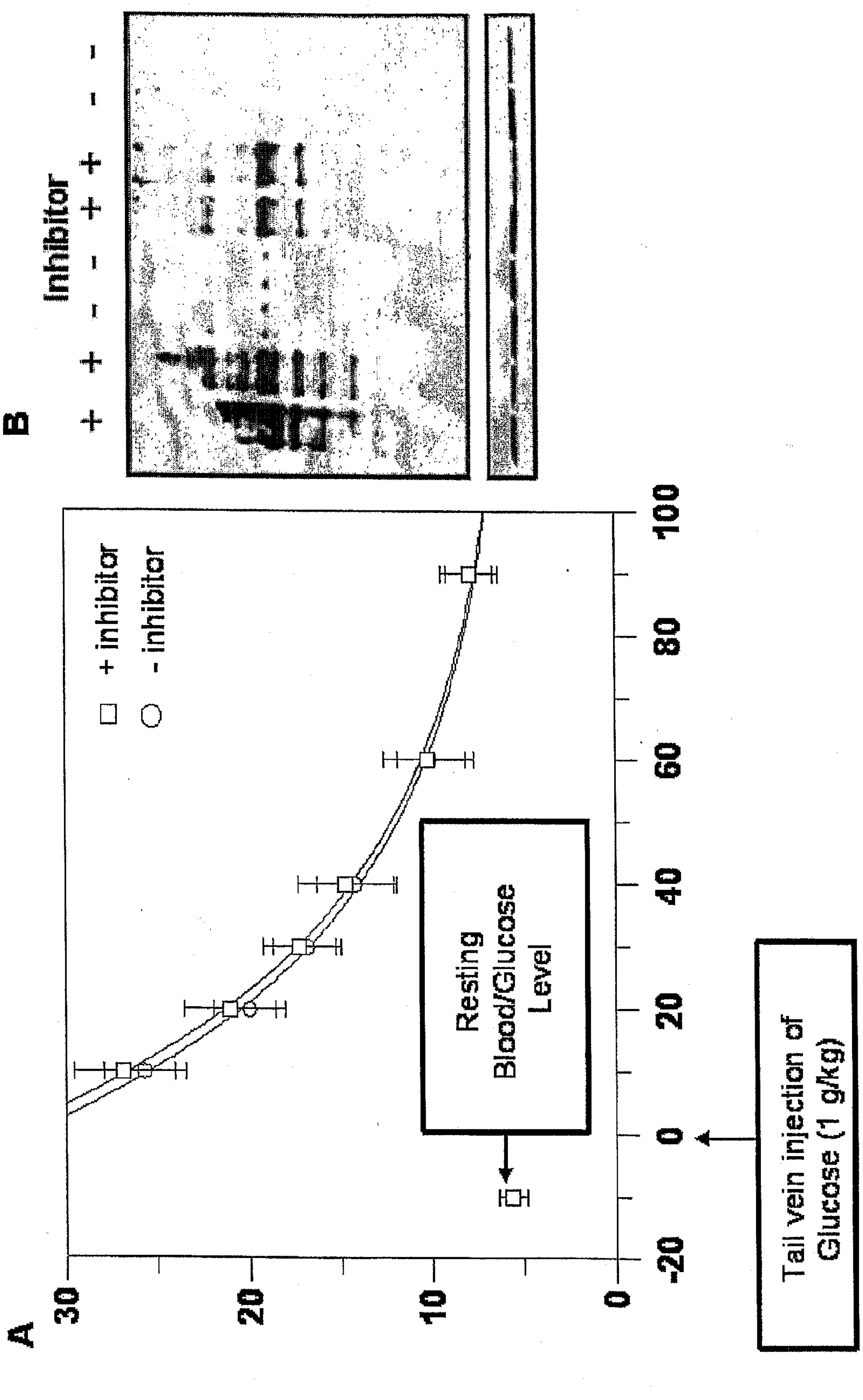
A
30
20
10
0
-20
0
20
40
60
80
100
+ inhibitor
- inhibitor
Resting Blood/Glucose Level
Tail vein injection of Glucose (1 g/kg)
B
Inhibitor
+ + - - + + - -

Figure 11

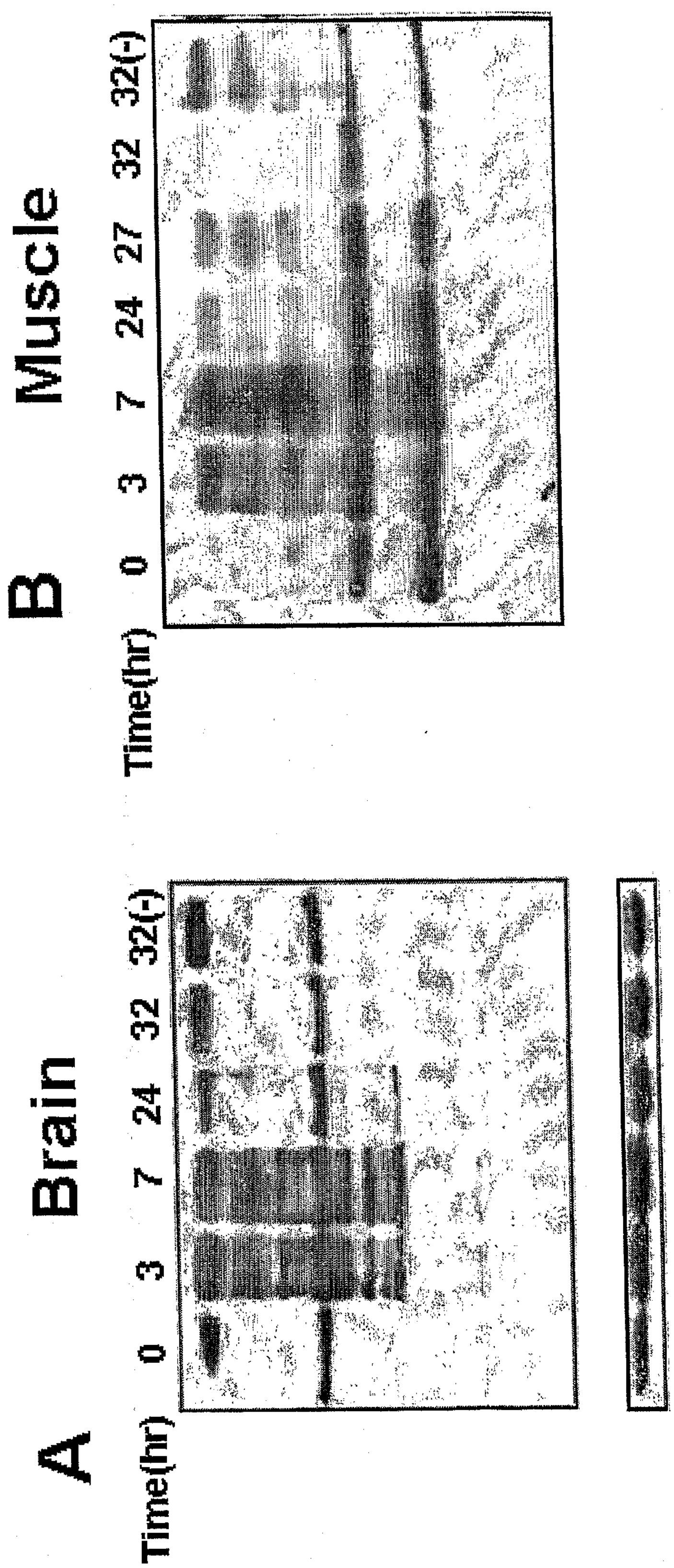

Figure 12

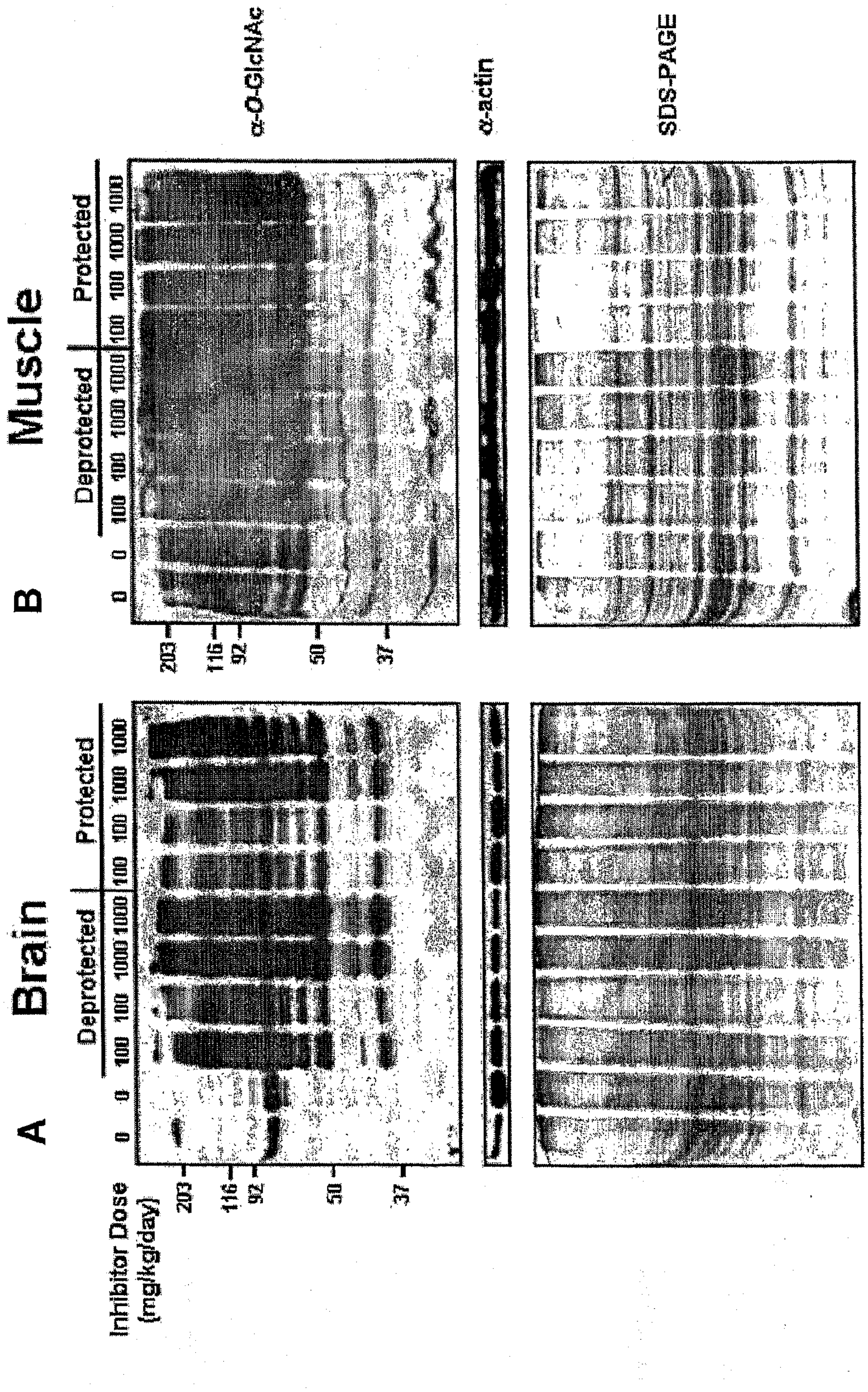
A
Brain
B
Muscle
Deprotected
Protected
Inhibitor Dose
(mg/kg/day)
0 0 100 100 1000 1000 100 100 1000 1000
203
116
92
50
37
α-O-GlcNAc
α-actin
SDS-PAGE

## Continuation of Figure 12

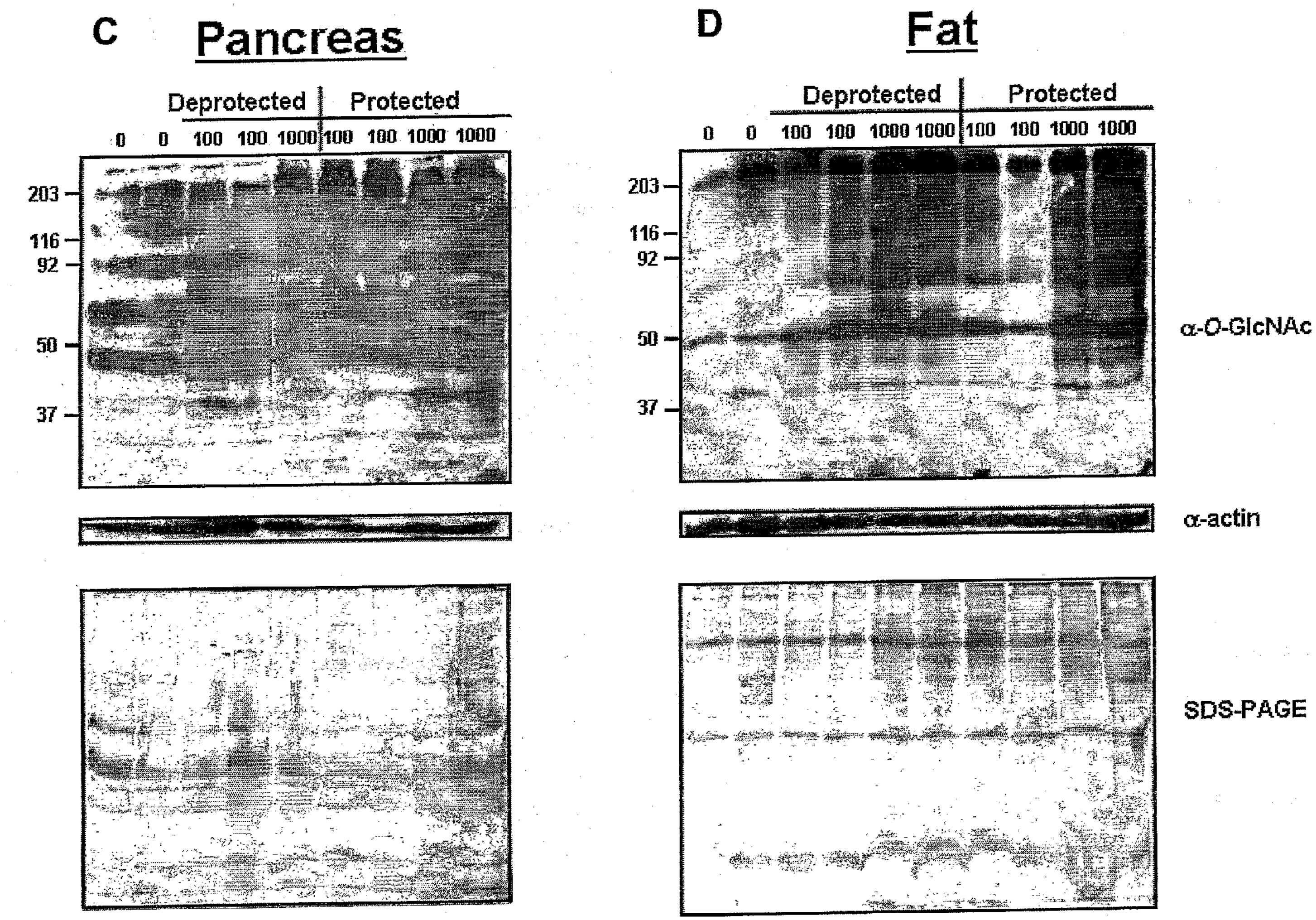

Continuation of Figure 12

E Spleen

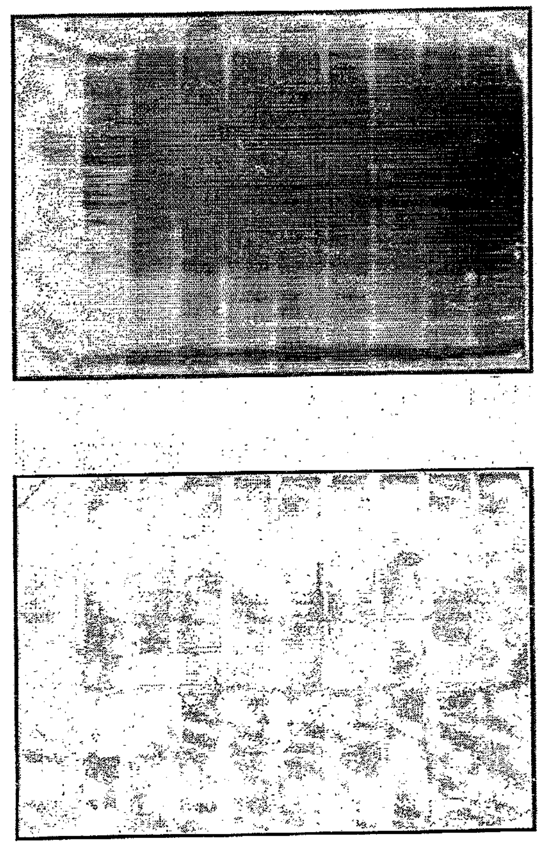

Europäisches Patentamt
European Patent Office
Office européen des brevets

# EUROPEAN SEARCH REPORT

Application Number
EP 11 18 4538

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EDWARD B. ARIAS, ET AL.: "Prolonged incubation in PUGNAc results in increased protein O-linked glycosylation and insulin resistance in rat skeletal muscle", DIABETES, vol. 53, 2004, pages 921-930, XP002529272, | 1 | INV. C07H17/00 C12Q1/00 C07D513/04 C07D471/04 A61K31/427 A61K31/35 C07D309/14 C07D277/62 C07D211/56 A61K31/445 |
| A | * the whole document * ----- | 2-8 | |
| X,P | KEITH A. STUBBS ET AL: "A divergent synthesis of 2-acyl derivatives of PUGNAc yields selective inhibitors of O-GlcNAcase", ORGANIC & BIOMOLECULAR CHEMISTRY, vol. 4, no. 5, 1 January 2006 (2006-01-01), page 839, XP55019600, ISSN: 1477-0520, DOI: 10.1039/b516273d * Scheme 3table 1 * ----- | 1-8 | |
| | | | TECHNICAL FIELDS SEARCHED (IPC) |
| | | | C07H |

The present search report has been drawn up for all claims

1

| Place of search | Date of completion of the search | Examiner |
|---|---|---|
| Munich | 27 February 2012 | Nikolai, Joachim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 60656878 B


### Non-patent literature cited in the description


- **COUTINHO, P.M. ; HENRISSAT, B.** *Carbohydrate-Active Enzymes,* 1999, URL: http://afmb.cnrs-mrs.fr/CAZY
- **C. R. TORRES ; G. W. HART.** *J Biol Chem,* 1984, vol. 259, 3308-3317
- **S. P. JACKSON ; R. TJIAN.** *Cell,* 1988, vol. 55, 125-133
- **W. G. KELLY ; M. E. DAHMUS ; G. W. HART.** *J Biol Chem,* 1993, vol. 268, 10416-10424
- **M. D. ROOS ; K. SU ; J. R. BAKER ; J. E. KUDLOW.** *Mol Cell Biol,* 1997, vol. 17, 6472-6480
- **N. LAMARRE-VINCENT ; L. C. HSIEH-WILSON.** *J Am Chem Soc,* 2003, vol. 125, 6612-6613
- **F. ZHANG ; K. SU ; X. YANG ; D. B. BOWE ; A. J. PATERSON ; J. E. KUDLOW.** *Cell,* 2003, vol. 115, 715-725
- **K. VOSSELLER ; L. WELLS ; M. D. LANE ; G. W. HART.** *Proc Natl Acad Sci U S A,* 2002, vol. 99, 5313-5318
- **W. A. LUBAS ; M. SMITH ; C. M. STARR ; J. A. HANOVER.** *Biochemistry,* 1995, vol. 34, 1686-1694
- **L. S. GRIFFITH ; B. SCHMITZ.** *Biochem Biophys Res Commun,* 1995, vol. 213, 424-431
- **R. N. COLE ; G. W. HART.** *J Neurochem,* 1999, vol. 73, 418-428
- **I. BRAIDMAN ; M. CARROLL ; N. DANCE ; D. ROBINSON.** *Biochem J,* 1974, vol. 143, 295-301
- **R. UENO ; C. S. YUAN.** *Biochim Biophys Acta,* 1991, vol. 1074, 79-84
- **D. L. DONG ; G. W. HART.** *J Biol Chem,* 1994, vol. 269, 19321-19330
- **C. TOLEMAN ; A. J. PATERSON ; T. R. WHISENHUNT ; J. E. KUDLOW.** *J Biol Chem,* 2004
- **Y. GAO ; L. WELLS ; F. I. COMER ; G. J. PARKER ; G. W. HART.** *J Biol Chem,* 2001, vol. 276, 9838-9845
- **L. WELLS ; Y. GAO ; J. A. MAHONEY ; K. VOSSELLER ; C. CHEN ; A. ROSEN ; G. W. HART.** 277. *J Biol Chem,* 2002, 1755-1761
- **F. LIU ; K. IQBAL ; I. GRUNDKE-IQBAL ; G. W. HART ; C. X. GONG.** *Proc Natl Acad Sci U S A,* 2004, vol. 101, 10804-10809
- **T. Y. CHOU ; G. W. HART.** *Adv Exp Med Biol,* 2001, vol. 491, 413-418
- **B. HENRISSAT ; A. BAIROCH.** *Biochem J,* 1996, vol. 316, 695-696
- **B. HENRISSAT ; A. BAIROCH.** *Biochem J,* 1993, vol. 293, 781-788
- **D. A. MCCLAIN ; W. A. LUBAS ; R. C. COOKSEY ; M. HAZEL ; G. J. PARKER ; D.C. LOVE ; J. A. HANOVER.** *Proc Natl Acad Sci U S A,* 2002, vol. 99, 10695-10699
- **L. WELLS ; K. VOSSELLER ; G. W. HART.** *Science,* 2001, vol. 291, 2376-2378
- **J. A. HANOVER.** *FASEB J,* 2001, vol. 15, 1865-1876
- **P. J. YAO ; P. D. COLEMAN.** *J Neurosci,* 1998, vol. 18, 2399-2411
- **B. TRIGGS-RAINE ; D. J. MAHURAN ; R. A. GRAVEL.** *Adv Genet,* 2001, vol. 44, 199-224
- **D. ZHOU ; J. MATTNER ; C. CANTU III ; N. SCHRANTZ ; N. YIN ; Y. GAO ; Y. SAGIV ; K. HUDSPETH ; Y. WU ; T. YAMASHITA.** *Science,* 2004
- **G. LEGLER ; E. LULLAU ; E. KAPPES ; F. KASTENHOLZ.** *Biochim Biophys Acta,* 1991, vol. 1080, 89-95
- **M. HORSCH ; L. HOESCH ; A. VASELLA ; D. M. RAST.** *Eur J Biochem,* 1991, vol. 197, 815-818
- **J. LIU ; A. R. SHIKHMAN ; M. K. LOTZ ; C. H. WONG.** *Chem Biol,* 2001, vol. 8, 701-711
- **S. KNAPP ; D. J. VOCADLO ; Z. N. GAO ; B. KIRK ; J. P. LOU ; S. G. WITHERS.** *J. Am. Chem. Soc.,* 1996, vol. 118, 6804-6805
- **V. H. LILLELUND ; H. H. JENSEN ; X. LIANG ; M. BOLS.** *Chem Rev,* 2002, vol. 102, 515-553
- **R. J. KONRAD ; I. MIKOLAENKO ; J. F. TOLAR ; K. LIU ; J. E. KUDLOW.** *Biochem J,* 2001, vol. 356, 31-41

- **K. LIU ; A. J. PATERSON ; F. ZHANG ; J. MCANDREW ; K. FUKUCHI ; J. M. WYSS ; L. PENG ; Y. HU ; J. E. KUDLOW.** *J Neurochem,* 2004, vol. 89, 1044-1055
- **G. PARKER ; R. TAYLOR ; D. JONES ; D. MCCLAIN.** *J Biol Chem,* 2004, vol. 279, 20636-20642
- **E. B. ARIAS ; J. KIM ; G. D. CARTEE.** *Diabetes,* 2004, vol. 53, 921-930
- **A. JUNOD ; A. E. LAMBERT ; L. ORCI ; R. PICTET ; A. E. GONET ; A. E. RENOLD.** *Proc Soc Exp Biol Med,* 1967, vol. 126, 201-205
- **R. A. BENNETT ; A. E. PEGG.** *Cancer Res,* 1981, vol. 41, 2786-2790
- **K. D. KRONCKE ; K. FEHSEL ; A. SOMMER ; M. L. RODRIGUEZ ; V. KOLB-BACHOFEN.** *Biol Chem Hoppe Seyler,* 1995, vol. 376, 179-185
- **H. YAMAMOTO ; Y. UCHIGATA ; H. OKAMOTO.** *Nature,* 1981, vol. 294, 284-286
- **K. YAMADA ; K. NONAKA ; T. HANAFUSA ; A. MIYAZAKI ; H. TOYOSHIMA ; S. TARUI.** *Diabetes,* 1982, vol. 31, 749-753
- **V. BURKART ; Z. Q. WANG ; J. RADONS ; B. HELLER ; Z. HERCEG ; L. STINGL ; E. F. WAGNER ; H. KOLB.** *Nat Med,* 1999, vol. 5, 314-319
- **M. D. ROOS ; W. XIE ; K. SU ; J. A. CLARK ; X. YANG ; E. CHIN ; A. J. PATERSON ; J. E. KUDLOW.** *Proc Assoc Am Physicians,* 1998, vol. 110, 422-432
- **Y. GAO ; G. J. PARKER ; G. W. HART.** *Arch Biochem Biophys,* 2000, vol. 383, 296-302
- **R. OKUYAMA ; M. YACHI.** *Biochem Biophys Res Commun,* 2001, vol. 287, 366-371
- **N. E. ZACHARA ; N. O'DONNELL ; W. D. CHEUNG ; J. J. MERCER ; J. D. MARTH ; G. W. HART.** *J Biol Chem,* 2004, vol. 279, 30133-30142
- **J. A. HANOVER ; Z. LAI ; G. LEE ; W. A. LUBAS ; S. M. SATO.** *Biochem Biophys,* 1999, vol. 362, 38-45
- **K. LIU ; A. J. PATERSON ; R. J. KONRAD ; A. F. PARLOW ; S. JIMI ; M. ROH ; E. CHIN, JR. ; J. E. KUDLOW.** *Mol Cell Endocrinol,* 2002, vol. 194, 135-146
- **B. L. MARK ; D. J. VOCADLO ; S. KNAPP ; B. L. TRIGGS-RAINE ; S. G. WITHERS ; M. N. JAMES.** *J Biol Chem,* 2001, vol. 276, 10330-10337
- **M. S. MACAULEY ; G. E. WHITWORTH ; A. W. DEBOWSKI ; D. CHIN ; D. J. VOCADLO.** *J Biol Chem,* 2005, vol. 280, 25313-25322
- **R. S. HALTIWANGER ; K. GROVE ; G. A. PHILIPSBERG.** *J Biol Chem,* 1998, vol. 273, 3611-3617
- **D. J. MILLER ; X. GONG ; B. D. SHUR.** *Development,* 1993, vol. 118, 1279-1289
- **S. MARSHALL ; W. T. GARVEY ; R. R. TRAXINGER.** *Faseb J,* 1991, vol. 5, 3031-3036
- **R. S. HALTIWANGER ; G. D. HOLT ; G. W. HART.** *J Biol Chem,* 1990, vol. 265, 2563-2568
- **L. K. KREPPEL ; M. A. BLOMBERG ; G. W. HART.** *J Biol Chem,* 1997, vol. 272, 9308-9315
- **W. A. LUBAS ; D. W. FRANK ; M. KRAUSE ; J. A. HANOVER.** *J Biol Chem,* 1997, vol. 272, 9316-9324
- **W. A. LUBAS ; J. A. HANOVER.** *J Biol Chem,* 2000, vol. 275, 10983-10988
- **S. P. IYER ; Y. AKIMOTO ; G. W. HART.** *J Biol Chem,* 2003, vol. 278, 5399-5409
- **K. BRICKLEY ; M. J. SMITH ; M. BECK ; F. A. STEPHENSON.** *J Biol Chem,* 2005, vol. 280, 14723-14732
- **X. YANG ; F. ZHANG ; J. E. KUDLOW.** *Cell,* 2002, vol. 110, 69-80
- **L. WELLS ; L. K. KREPPEL ; F. I. COMER ; B. E. WADZINSKI ; G. W. HART.** *J Biol Chem,* 2004, vol. 279, 38466-38470
- **S. P. IYER ; G. W. HART.** *J Biol Chem,* 2003, vol. 278, 24608-24616
- **M. JINEK ; J. REHWINKEL ; B. D. LAZARUS ; E. IZAURRALDE ; J. A. HANOVER ; E. CONTI.** *Nat Struct Mol Biol,* 2004, vol. 11, 1001-1007
- **E. P. ROQUEMORE ; M. R. CHEVRIER ; R. J. COTTER ; G. W. HART.** *Biochemistry,* 1996, vol. 35, 3578-3586
- **K. KAMEMURA ; B. K. HAYES ; F. I. CORNER ; G. W. HART.** *J Biol Chem,* 2002, vol. 277, 19229-19235
- **X. CHENG ; G. W. HART.** *J Biol Chem,* 2001, vol. 276, 10570-10575
- **X. CHENG ; R. N. COLE ; J. ZAIA ; G. W. HART.** *Biochemistry,* 2000, vol. 39, 11609-11620
- **L. K. KREPPEL ; G. W. HART.** *J Biol Chem,* 1999, vol. 274, 32015-32022
- **N. E. ZACHARA ; G. W. HART.** *Biochim Biophys Acta,* 2004, vol. 1673, 13-28
- **M. GOEDERT ; M. G. SPILLANTINI ; N. J. CAIRNS ; R. A. CROWTHER.** *Neuron,* 1992, vol. 8, 159-168
- **M. GOEDERT ; M. G. SPILLANTINI ; R. JAKES ; D. RUTHERFORD ; R. A. CROWTHER.** *Neuron,* 1989, vol. 3, 519-526
- **E. KOPKE ; Y. C. TUNG ; S. SHAIKH ; A. C. ALONSO ; K. IQBAL ; I. GRUNDKE-IQBAL.** *J Biol Chem,* 1993, vol. 268, 24374-24384
- **H. KSIEZAK-REDING ; W. K. LIU ; S. H. YEN.** *Brain Res,* 1992, vol. 597, 209-219
- **T. Y. CHOU ; G. W. HART ; C. V. DANG.** *J Biol Chem,* 1995, vol. 270, 18961-18965
- **L. S. GRIFFITH ; B. SCHMITZ.** *Eur JBiochem,* 1999, vol. 262, 824-831
- **K. KAMEMURA ; G. W. HART.** *Prog Nucleic Acid Res Mol Biol,* 2003, vol. 73, 107-136

- **L. BERTRAM ; D. BLACKER ; K. MULLIN ; D. KEENEY ; J. JONES ; S. BASU ; S. YHU ; M. G. MCINNIS ; R. C. GO ; K. VEKRELLIS.** *Science,* 2000, vol. 290, 2302-2303
- **U. J. G. CONFERENCE.** *US/Japan Glyco 2004 Conference,* 2004
- **B. L. MARK ; D. J. MAHURAN ; M. M. CHERNEY ; D. ZHAO ; S. KNAPP ; M. N. JAMES.** *J Mol Biol,* 2003, vol. 327, 1093-1109
- **R. KUROKI ; L. H. WEAVER ; B. L. MATTHEWS.** *Proc. Natl. Acad. Sci. USA,* 1999, vol. 96, 8949-8954
- **D. L. ZECHEL ; S. G. WITHERS.** *Acc Chem Res,* 2000, vol. 33, 11-18
- **D. J. VOCADLO ; C. MAYER ; S. HE ; S. G. WITHERS.** *Biochemistry,* 2000, vol. 39, 117-126
- **D. J. VOCADLO ; G. J. DAVIES ; R. LAINE ; S. G. WITHERS.** *Nature,* 2001, vol. 412, 835-838
- **I. TEWS ; A. PERRAKIS ; A. OPPENHEIM ; Z. DAUTER ; K. S. WILSON ; C. E. VORGIAS.** *Nat Struct Biol,* 1996, vol. 3, 638-648
- **K. R. ROESER ; G. LEGLER.** *Biochim Biophys Acta,* 1981, vol. 657, 321-333
- **M. S. MACAULEY ; K. A. STUBBS ; D. J. VOCADLO.** *J Am Chem Soc,* 2005, vol. 127, 17202-17203
- **C. HANSCH ; A. LEO.** Substituent constants for correlation analysis in chemistry and biology. Wiley, 1979
- **T. MAIER ; N. STRATER ; C. G. SCHUETTE ; R. KLINGENSTEIN ; K. SANDHOFF ; W. SAENGER.** *J Mol Biol,* 2003, vol. 328, 669-681
- **C. S. JONES ; D. J. KOSMAN.** *J Biol Chem,* 1980, vol. 255, 11861-11869
- **A. C. TERWISSCHA ; VAN SCHELTINGA ; S. ARMAND ; K. H. KALK ; A. ISOGAI ; B. HENRISSAT ; B. W. DIJKSTRA.** *Biochemistry,* 1995, vol. 34, 15619-15623
- **Z. MARKOVIC-HOUSLEY ; G. MIGLIERINI ; L. SOLDATOVA ; P. J. RIZKALLAH ; U. MULLER ; T. SCHIRMER.** *Structure Fold Des,* 2000, vol. 8, 1025-1035
- **B. L. MARK ; M. N. G. JAMES.** *Can. J Chem.,* 2002, vol. 80, 1064-1074
- **M. BERGMANN ; L. ZERVAS.** *Ber. Dtsch. Chem. Ges.,* 1999, vol. 64B, 975-980
- **A. C. CUNHA ; L. O. R. PEREIRA ; D. S. M. C. B. V. ; V. F. FERREIRA.** *J. Chem. Ed.,* 1999, vol. 76, 79-80
- **P. BOULLANGER ; M. JOUINEAU ; B. BOUAMMALI ; D. LAFONT ; G. DESCOTES.** *Carbohydr, Res.,* 1990, vol. 202, 151-164
- **H. MOHAN ; A. VASELLA.** *Nelv Chim Acta,* 2000, vol. 83, 114-118
- **T. D. HEIGHTMAN ; A. T. VASELLA.** *Angew Chem Int Edit,* 1999, vol. 38, 750-770
- **D. J. VOCADLO ; S. G. WITHERS.** *Biochemistry,* 2005, vol. 44, 12809-12818
- **M. JEYAKUMAR ; R. A. DWEK ; T. D. BUTTERS ; F. M. PLATT.** *Nat Rev Neurosci,* 2005, vol. 6, 713-725
- **R. J. VAN DEN BERG ; W. DONKER-KOOPMAN ; J. H. VAN BOOM ; H. M. AERTS ; D. NOORT.** *Bioorg Med Chem,* 2004, vol. 12, 891-902
- **E. SHITARA ; Y. NISHIMURA ; F. KOJIMA ; T. TAKEUCHI.** *Bioorg Med Chem,* 1999, vol. 7, 1241-1246
- **M. B. TROPAK ; S. P. REID ; M. GUIRAL ; S. G. WITHERS ; D. MAHURAN.** *J Biol Chem,* 2004, vol. 279, 13478-13487